# EUROPEAN PATENT APPLICATION

(11) **EP 4 534 554 A1**
(43) Date of publication of application: **09.04.2025**
(21) Application number: 23815288.8
(22) Date of filing: 01.06.2023
(51) Int. Cl.: C07K 14/725, C07K 16/46, C12N 15/12, C12N 15/85, C12N 5/0783, A61K 38/17, A61K 35/17, A61P 35/00

(54) **MAGE-A1 SPECIFIC TCR AND USE THEREOF**

(30) Priority: 01.06.2022 CN 202210622198
(71) Applicant: Corregene Biotechnology Co., Ltd., Beijing 102206 (CN)
(72) Inventor: JIANG, Dong, Beijing 102200 (CN); XIE, Xingwang, Beijing 102206 (CN); BI, Jinglei, Beijing 102200 (CN); WANG, Jianghua, Beijing 102206 (CN); WANG, Xueyan, Beijing 102200 (CN); SHI, Yunqiang, Beijing 102200 (CN)
(74) Representative: J A Kemp LLP
(86) International application number: PCT/CN2023/097804
(87) International publication number: WO 2023/232111

(57) **Abstract**

The present disclosure provides MAGE-A1-specific TCRs and their uses, specifically relating to TCRs modified to enhance binding affinity, nucleic acids encoding such TCRs, vectors comprising the nucleic acids of the invention, and cells comprising the TCRs, nucleic acids, or vectors of the invention, as well as methods for preparing such cells. The disclosure also covers conjugates or compositions comprising the TCRs of the invention, and methods for treating cancer using the TCRs or cells of the invention.

## Description

### PRIORITY CLAIM

This international patent application claims priority to Chinese patent application CN20221 0622198. 1, filed on June 1, 2022, the entirety of which is incorporated herein by reference.

### FIELD OF INVENTION

The present disclosure relates to T cell receptor (TCR) and uses thereof, particularly TCR specific to MAGE-A1 and their use in treating MAGE-A1-positive cancers.

### BACKGROUND

TCR-T (T-cell receptor engineered T cell therapy) involves cloning TCRs that can specifically recognize certain human leukocyte antigen (HLA)-tumor antigen peptide complexes through corresponding experimental techniques, and delivering the TCR gene encoding sequences to more T cells using gene delivery methods such as lentivirus, which endowing these T cells with new antigen recognition specificity. Patient-derived T cells are transduced with TCR genes in vitro and expanded in large quantities, thereby these cells can effectively recognize tumor cell-specific antigens, and when reinfused into the patient, can specifically kill tumor cells, exerting anti-tumor activity. Since both intracellular and extracellular antigens can be presented by HLA and recognized by TCRs, TCR-T can target most tumor-specific antigens, particularly those inside tumor cells (accounting for about 90% of all antigens). Therefore, TCR-T is promising for the treatment of almost all tumors, especially various solid tumors.

Numerous studies have found that tumor cells express specific tumor-associated antigens that are not expressed or are expressed at low levels in normal tissues. Therefore, developing TCR-T products targeting these tumor-associated antigens is a crucial approach in tumor immunotherapy. Among these tumor-associated antigens, some are specifically expressed on tumor cells and germ cells, known as "cancer-testis antigens". These antigen genes naturally become prime targets for TCR-T product research. The melanoma-associated antigen gene family, MAGE, is the most thoroughly studied cancer-testis antigen to date, with MAGE-A1 being the first identified cancer-testis antigen gene. Numerous studies have shown that MAGE-A1 is expressed on the surface of various solid tumor cells, comprising melanoma, lung cancer, colorectal cancer, cervical cancer, and breast cancer. Thus, developing TCR-T products targeting MAGE-A1 could benefit a broader patient population.

Previously, many HLA-A0201-restricted TCRs targeting MAGE-A1 have been developed, named 'ht27' and 'T1367' (Obenaus, M., Leitão, C., Leisegang, M., Chen, X., Gavvovidis, I., van der Bruggen, P., Uckert, W., et al., Identification of human T-cell receptors with optimal affinity to cancer antigens using antigen-negative humanized mice. Nat. Biotechnol. 2015. 33: 402-407; Ottaviani, S., Zhang, Y., Boon, T., and van der Bruggen, P., A MAGE-1 antigenic peptide recognized by human cytolytic T lymphocytes on HLA-A2 tumor cells. Cancer Immunol. Immunother. 2005. 54: 1214-1220.). Among them, ht27 is a natural TCR sequence found in humans, but its affinity is relatively low, making it challenging to exert effective anti-tumor effects in vivo. "T1367" is a murine TCR sequence targeting human MAGE-A1 antigen, discovered in transgenic mice. This TCR has high affinity and can effectively kill tumor cells in vitro, but it may cause strong autoimmune responses when applied in human experiments, rendering the treatment ineffective.

Therefore, there is a need to develop TCR products with high affinity specifically targeting MAGE-A1.

### SUMMARY OF THE INVENTION

This invention uses the human-derived TCR ht27 sequence as a template, employing in vitro mutagenesis to mutate the complementary determining region (CDR) of ht27. Through positive selection of the mutated TCR in vitro, high-affinity mutant clones are obtained, followed by in vitro negative selection to exclude mutant clones exhibiting nonspecific activation. As a result, a mutated TCR sequence with high affinity and no nonspecific reactions is obtained. Additionally, the specific and non-specific reactions of the mutated TCR sequences are verified in vitro experiments, ultimately yielding a mutated TCR sequence with strong specificity and high affinity.

Accordingly, in one aspect, the present disclosure provides a modified T-cell receptor (TCR), comprising an α chain variable region comprising CDR1α, CDR2α, and CDR3α, and a β chain variable region comprising CDR1β, CDR2β, and CDR3β, wherein the modified TCR comprises the following amino acid substitutions relative to the parental TCR:
(i) amino acid substitutions in the β chain variable region, comprising
   (a) one or more amino acid substitutions at the following positions in CDR2β: Asn57, Glu58, Gly59, Ser63, Lys64, and Ala65, and/or
   (b) one or more amino acid substitutions at the following positions in CDR3β: Ser105, Glu108, Gln111, and Tyr114;
      and/or
(ii) amino acid substitutions in the α chain variable region, comprising
   (c) one or more amino acid substitutions at the following positions in CDR1α: Ser29 and Ser36, and/or
   (d) an amino acid substitution at the following positions in CDR2α: Asn59;
      wherein the parental TCR comprises CDR1β having an amino acid sequence as set forth in SEQ ID NO: 24, CDR2β having an amino acid sequence as set forth in SEQ ID NO: 25, CDR3β having an amino acid sequence as set forth in SEQ ID NO: 26, CDR1α having an amino acid sequence as set forth in SEQ ID NO: 27, CDR2α having an amino acid sequence as set forth in SEQ ID NO: 28, and CDR3α having an amino acid sequence as set forth in SEQ ID NO: 29;
      wherein amino acid positions are numbered according to the International ImMunoGeneTics Information System (IMGT) nomenclature.

In some embodiments, the modified TCR comprises one or more of the following amino acid substitutions (1)-(13) relative to the parental TCR:
(1) substitution at position Asn57, selected from the group consisting of N57D, N57E, N57H, N57K, N57Q, N57R, N57S, and N57T;
(2) substitution at position Glu58, selected from the group consisting of E58A, E58F, E58I, E58L, E58M, E58P, E58V, E58Y, and E58W;
(3) substitution at position Gly59, selected from the group consisting of G59D, G59E, G59H, G59K, G59Q, G59R, G59S, and G59T;
(4) substitution at position Ser63, selected from the group consisting of S63D, S63E, S63H, S63K, S63Q, S63R, and S63T;
(5) substitution at position Lys64, selected from the group consisting of K64M, K64C, K64W, K64T, K64P, and K64A;
(6) substitution at position Ala65, selected from the group consisting of A65F, A65I, A65L, A65M, A65P, A65V, A65W, and A65Y;
(7) substitution at position Ser105, selected from the group consisting of S105L, S105N, S105H, S105Q, S105K, and S105R;
(8) substitution at position Glu108, selected from the group consisting of E108D, E108F, E108T, E108I, E108K, and E108P;
(9) substitution at position Gln111, selected from the group consisting of Q111N, Q111K, Q111S, Q111G, Q111A, Q111V, Q111E, Q111L, and Q111H;
(10) substitution at position Tyr114, selected from the group consisting of Y114I, Y114F, Y114K, Y114D, Y114L, Y114W, Y114N, Y114P, and Y114S;
(11) substitution at position Ser29, selected from the group consisting of S29A, S29F, S29I, S29L, S29M, S29P, S29V, S29W, and S29N;
(12) substitution at position Ser36, selected from the group consisting of S36M, S36L, S36Q, S36I, S36F, S36P, and S36V; and
(13) substitution at position Asn59, selected from the group consisting of N59F, N59D, N59E, N59H, N59K, N59Q, N59R, N59S, and N59T.

In some embodiments, the modified TCR does not comprise a single S105N amino acid substitution relative to the parental TCR.

In some embodiments, the modified TCR comprises one or more of the following amino acid substitutions in CDR2β relative to the parental TCR: N57D, E58Y, G59Q, S63T, K64C, and A65V; preferably, the modified TCR comprises amino acid substitutions N57D, E58Y, G59Q, S63T, K64C, and A65V in CDR2β relative to the parental TCR.

In some embodiments, the modified TCR comprises one or more of the following amino acid substitutions in CDR3β relative to the parental TCR:
(1) Q111G or Q111S;
(2) Y114F, Y114L, or Y114W; and
(3) S105L, S105Q, S105N, or S105H;
preferably, the modified TCR comprises amino acid substitutions Y114F, Y114W, Q111GY114F, Q111SY114F, S105L, or S105N in CDR3β relative to the parental TCR.

In some embodiments, the modified TCR comprises amino acid substitutions N57D, E58Y, G59Q, S63T, K64C, and A65V in CDR2β, and amino acid substitutions Y114F, Y114L, Y114W, Q111GY114F, or Q111SY114F in CDR3β relative to the parental TCR.

In some embodiments, the modified TCR comprises the following amino acid substitutions in CDR1α relative to the parental TCR:
(1) S29L; and/or
(2) S36M, S36L, or S36Q.

In some embodiments, the modified TCR comprises the amino acid substitution N59F in CDR2α relative to the parental TCR.

In some embodiments, the modified TCR comprises:
(i) an α chain variable region comprising CDR1α, CDR2α, and CDR3α with amino acid sequences as shown in SEQ ID NOs: 27-29, respectively; and
(ii) a β chain variable region comprising

CDR1β, CDR2β, and CDR3β with amino acid sequences as shown in SEQ ID NOs: 24, 30, and 26, respectively;
CDR1β, CDR2β, and CDR3β with amino acid sequences as shown in SEQ ID NOs: 24, 25, and 31, respectively;
CDR1β, CDR2β, and CDR3β with amino acid sequences as shown in SEQ ID NOs: 24, 25, and 32, respectively;
CDR1β, CDR2β, and CDR3β with amino acid sequences as shown in SEQ ID NOs: 24, 25, and 33, respectively;
CDR1β, CDR2β, and CDR3β with amino acid sequences as shown in SEQ ID NOs: 24, 25, and 34, respectively;
CDR1β, CDR2β, and CDR3β with amino acid sequences as shown in SEQ ID NOs: 24, 25, and 35, respectively;
CDR1β, CDR2β, and CDR3β with amino acid sequences as shown in SEQ ID NOs: 24, 30, and 33, respectively;
CDR1β, CDR2β, and CDR3β with amino acid sequences as shown in SEQ ID NOs: 24, 30, and 34, respectively;
CDR1β, CDR2β, and CDR3β with amino acid sequences as shown in SEQ ID NOs: 24, 30, and 35, respectively;
CDR1β, CDR2β, and CDR3β with amino acid sequences as shown in SEQ ID NOs: 24, 25, and 36, respectively;
CDR1β, CDR2β, and CDR3β with amino acid sequences as shown in SEQ ID NOs: 24, 25, and 37, respectively;
CDR1β, CDR2β, and CDR3β with amino acid sequences as shown in SEQ ID NOs: 24, 30, and 36, respectively;
CDR1β, CDR2β, and CDR3β with amino acid sequences as shown in SEQ ID NO:s 24, 30, and 37, respectively;
CDR1β, CDR2β, and CDR3β with amino acid sequences as shown in SEQ ID NOs: 24, 25, and 38, respectively;
CDR1β, CDR2β, and CDR3β with amino acid sequences as shown in SEQ ID NOs: 24, 25, and 39, respectively;
CDR1β, CDR2β, and CDR3β with amino acid sequences as shown in SEQ ID NOs: 24, 25, and 40, respectively;
CDR1β, CDR2β, and CDR3β with amino acid sequences as shown in SEQ ID NOs: 24, 25, and 41, respectively.

In some embodiments, the modified TCR comprises:
(i) a β chain variable region, which comprises CDR1β, CDR2β, and CDR3β with amino acid sequences as shown in SEQ ID NOs:24-26; and
(ii) an α chain variable region, which comprises:
   CDR1α, CDR2α, and CDR3α with amino acid sequences as shown in SEQ ID NOs:42, 28, and 29;
   CDR1α, CDR2α, and CDR3α with amino acid sequences as shown in SEQ ID NOs:43, 28, and 29;
   CDR1α, CDR2α, and CDR3α with amino acid sequences as shown in SEQ ID NOs:44, 28, and 29;
   CDR1α, CDR2α, and CDR3α with amino acid sequences as shown in SEQ ID NOs:45, 28, and 29; or
   CDR1α, CDR2α, and CDR3α with amino acid sequences as shown in SEQ ID NOs:27, 76, and 29.

In some embodiments, the modified TCR comprises:
(i) an α chain variable region, which comprises:
   CDR1α, CDR2α, and CDR3α with amino acid sequences as shown in SEQ ID NOs:42, 28, and 29;
   CDR1α, CDR2α, and CDR3α with amino acid sequences as shown in SEQ ID NOs:43, 28, and 29;
   CDR1α, CDR2α, and CDR3α with amino acid sequences as shown in SEQ ID NOs:44, 28, and 29;
   CDR1α, CDR2α, and CDR3α with amino acid sequences as shown in SEQ ID NOs:45, 28, and 29; or
   CDR1α, CDR2α, and CDR3α with amino acid sequences as shown in SEQ ID NOs:27, 76, and 29;
(ii) a β chain variable region, which comprises:
   CDR1β, CDR2β, and CDR3β with amino acid sequences as shown in SEQ ID NOs:24, 30, and 26;
   CDR1β, CDR2β, and CDR3β with amino acid sequences as shown in SEQ ID NOs:24, 25, and 31;
   CDR1β, CDR2β, and CDR3β with amino acid sequences as shown in SEQ ID NOs:24, 25, and 32;
   CDR1β, CDR2β, and CDR3β with amino acid sequences as shown in SEQ ID NOs:24, 25, and 33;
   CDR1β, CDR2β, and CDR3β with amino acid sequences as shown in SEQ ID NOs:24, 25, and 34;
   CDR1β, CDR2β, and CDR3β with amino acid sequences as shown in SEQ ID NOs:24, 25, and 35;
   CDR1β, CDR2β, and CDR3β with amino acid sequences as shown in SEQ ID NOs:24, 30, and 33;
   CDR1β, CDR2β, and CDR3β with amino acid sequences as shown in SEQ ID NOs:24, 30, and 34;
   CDR1β, CDR2β, and CDR3β with amino acid sequences as shown in SEQ ID NOs:24, 30, and 35;
   CDR1β, CDR2β, and CDR3β with amino acid sequences as shown in SEQ ID NOs:24, 25, and 36;
   CDR1β, CDR2β, and CDR3β with amino acid sequences as shown in SEQ ID NOs:24, 25, and 37;
   CDR1β, CDR2β, and CDR3β with amino acid sequences as shown in SEQ ID NOs:24, 30, and 36;
   CDR1β, CDR2β, and CDR3β with amino acid sequences as shown in SEQ ID NOs:24, 30, and 37;
   CDR1β, CDR2β, and CDR3β with amino acid sequences as shown in SEQ ID NOs:24, 25, and 38;
   CDR1β, CDR2β, and CDR3β with amino acid sequences as shown in SEQ ID NOs:24, 25, and 39;
   CDR1β, CDR2β, and CDR3β with amino acid sequences as shown in SEQ ID NOs:24, 25, and 40; or
   CDR1β, CDR2β, and CDR3β with amino acid sequences as shown in SEQ ID NOs:24, 25, and 41.

In some embodiments, the modified TCR comprises an α chain variable region having the amino acid sequence as shown in SEQ ID NO:46 or an amino acid sequence having at least 80%, at least 85%, at least 90%, at least 95%, or at least 99% sequence identity to SEQ ID NO:46; and a β chain variable region having an amino acid sequence selected from the group consisting of any one of SEQ ID NOs:48-64 or an amino acid sequence having at least 80%, at least 85%, at least 90%, at least 95%, or at least 99% sequence identity to any one of SEQ ID NOs:48-64.

In some embodiments, the modified TCR comprises an α chain variable region having an amino acid sequence selected from the group consisting of any one of SEQ ID NOs:65-68 and 77 or an amino acid sequence having at least 80%, at least 85%, at least 90%, at least 95%, or at least 99% sequence identity to any one of SEQ ID NOs:65-68 and 77; and a β chain variable region having the amino acid sequence as shown in SEQ ID NO:47 or an amino acid sequence having at least 80%, at least 85%, at least 90%, at least 95%, or at least 99% sequence identity to SEQ ID NO:47.

In some embodiments, the modified TCR comprises an α chain variable region having an amino acid sequence selected from the group consisting of any one of SEQ ID NOs:65-68 and 77 or an amino acid sequence having at least 80%, at least 85%, at least 90%, at least 95%, or at least 99% sequence identity to any one of SEQ ID NOs:65-68 and 77; and a β chain variable region having an amino acid sequence selected from the group consisting of any one of SEQ ID NOs:48-64 or an amino acid sequence having at least 80%, at least 85%, at least 90%, at least 95%, or at least 99% sequence identity to any one of SEQ ID NOs:48-64.

In some embodiments, the modified TCR further comprises a TCR constant region. In some embodiments, the TCR constant region is a murine constant region or a human constant region. In some embodiments, the TCR constant region comprises a TCR α chain constant region and/or a TCR β chain constant region. In preferred embodiments, the TCR α chain constant region and/or TCR β chain constant region comprises at least one cysteine substitution relative to the wild-type sequence to form a disulfide bond between the TCR α chain and TCR β chain. In preferred embodiments, the TCR α chain constant region comprises an LVL substitution or an LIV substitution such that the α chain constant region comprises the amino acid sequence LLVIVLRIL.

In some embodiments, the modified TCR comprises a TCR α chain having the amino acid sequence as shown in SEQ ID NO:69 or an amino acid sequence having at least 80%, at least 85%, at least 90%, at least 95%, or at least 99% sequence identity to SEQ ID NO:69; and a TCR β chain having an amino acid sequence selected from the group consisting of any one of SEQ ID NOs:3-19 or an amino acid sequence having at least 80%, at least 85%, at least 90%, at least 95%, or at least 99% sequence identity to any one of SEQ ID NOs:3-19.

In some embodiments, the modified TCR comprises a TCR α chain having an amino acid sequence selected from the group consisting of any one of SEQ ID NOs:20-23 and 78 or an amino acid sequence having at least 80%, at least 85%, at least 90%, at least 95%, or at least 99% sequence identity to any one of SEQ ID NOs:20-23 and 78; and a TCR β chain having the amino acid sequence as shown in SEQ ID NO:70 or an amino acid sequence having at least 80%, at least 85%, at least 90%, at least 95%, or at least 99% sequence identity to SEQ ID NO:70.

In some embodiments, the modified TCR comprises a TCR α chain having an amino acid sequence selected from the group consisting of any one of SEQ ID NOs:20-23 and 78 or an amino acid sequence having at least 80%, at least 85%, at least 90%, at least 95%, or at least 99% sequence identity to any one of SEQ ID NOs:20-23 and 78; and a TCR β chain having an amino acid sequence selected from the group consisting of any one of SEQ ID NOs:3-19 or an amino acid sequence having at least 80%, at least 85%, at least 90%, at least 95%, or at least 99% sequence identity to any one of SEQ ID NOs:3-19.

In some embodiments, the modified TCR binds to an epitope comprising the amino acid sequence KVLEYVIKV (SEQ ID NO:2) or a complex of the epitope with an MHC molecule.

In some embodiments, the MHC molecule is HLA-A*02 type, such as HLA-A*02:01 type or HLA-A*02:05 type.

In some embodiments, the modified TCR is soluble or membrane-bound.

In some embodiments, the modified TCR is isolated or purified.

On the other hand, the present disclosure provides a modified T-cell receptor (TCR), wherein the modified TCR comprises an α chain variable region comprising CDR1α, CDR2α, and CDR3α, and a β chain variable region comprising CDR1β, CDR2β, and CDR3β, wherein the modified TCR comprising the following amino acid substitutions relative to the parental TCR:
(i) amino acid substitutions in the β chain variable region, comprising:
   (a) one or more amino acid substitutions at the following positions in CDR1β: Gln29, Ala36, Thr37, and Thr38,
   (b) one or more amino acid substitutions at the following positions in CDR2β: Ser56, Asn57, Glu58, and Gly59, and/or
   (c) one or more amino acid substitutions at the following positions in CDR3β: Glu108, Pro109, Gly110, Gln111, Gly112, Pro113, Tyr114, and Glu115;
      and/or
(ii) amino acid substitutions in the α chain variable region, comprising:
   (d) one or more amino acid substitutions at the following positions in CDR1α: Ser28, Ser29, Ser36, Thr37, and Tyr38,
   (e) amino acid substitutions at the following positions in CDR2α: Ile56, Phe57, Ser58, Asn59, Met63, and Asp64, and/or
   (f) amino acid substitution at position Thr26 in FR1α;
      wherein the parental TCR comprises: CDR1β having the amino acid sequence as shown in SEQ ID NO: 24, CDR2β having the amino acid sequence as shown in SEQ ID NO: 25, CDR3β having the amino acid sequence as shown in SEQ ID NO: 26, CDR1α having the amino acid sequence as shown in SEQ ID NO: 27, CDR2α having the amino acid sequence as shown in SEQ ID NO: 28, and CDR3α having the amino acid sequence as shown in SEQ ID NO: 29;
      wherein the amino acid positions are numbered according to the International ImMunoGeneTics information system (IMGT) nomenclature.

In some embodiments, the modified TCR comprises one or more of the following amino acid substitutions (1)-(25) relative to the parental TCR:
(1) amino acid substitution Q29P at position Gln29 in CDR1β;
(2) amino acid substitution A36I at position Ala36 in CDR1β;
(3) amino acid substitution T37E at position Thr37 in CDR1β;
(4) amino acid substitution T38R at position Thr38 in CDR1β;
(5) amino acid substitution S56T at position Ser56 in CDR2β;
(6) amino acid substitution selected from the group consisting of N57Q and N57D at position Asn57 in CDR2β;
(7) amino acid substitution selected from the group consisting of E58L, E58P, and E58Y at position Glu58 in CDR2β;
(8) amino acid substitution selected from the group consisting of G59P and G59L at position Gly59 in CDR2β;
(9) amino acid substitution EPGQGP replaced with VNDGT at positions Glu108 to Pro113 in CDR3β;
(10) amino acid substitution G112E at position Gly112 in CDR3β;
(11) amino acid substitution P113H at position Pro113 in CDR3β;
(12) amino acid substitution Y114L at position Tyr114 in CDR3β;
(13) amino acid substitution E115I at position Glu115 in CDR3β;
(14) amino acid substitution selected from the group consisting of S28Y and S28P at position Ser28 in CDR1α;
(15) amino acid substitution selected from the group consisting of S29T, S29L, and S29Y at position Ser29 in CDR1α;
(16) amino acid substitution S36D at position Ser36 in CDR1α;
(17) amino acid substitution T37I at position Thr37 in CDR1α;
(18) amino acid substitution Y38L at position Tyr38 in CDR1α;
(19) amino acid substitution selected from the group consisting of I56T and 156G at position Ile56 in CDR2α;
(20) amino acid substitution selected from the group consisting of F57W and F57T at position Phe57 in CDR2α;
(21) amino acid substitution selected from the group consisting of S58G, S58I, S58N, S58A, S58P, and S58W at position Ser58 in CDR2α;
(22) amino acid substitution selected from the group consisting of N59F, N59T, N59H, and N59L at position Asn59 in CDR2α;
(23) amino acid substitution selected from the group consisting of M63D, M63Q, M63T, and M63E at position Met63 in CDR2α;
(24) amino acid substitution selected from the group consisting of D64T, D64Y, and D64S at position Asp64 in CDR2α;
(25) amino acid substitution selected from the group consisting of T26A and T26V at position Thr26 in FR1α.

In some embodiments, relative to the parental TCR, the modified TCR comprises one or more of the following amino acid substitutions in CDR1β: Q29P, A36I, T37E, and T38R; preferably, the modified TCR comprises amino acid substitutions Q29P, A36I, T37E, and T38R in CDR1β relative to the parental TCR.

In some embodiments, relative to the parental TCR, the modified TCR comprises one or more of the following amino acid substitutions (1)-(4) in CDR2β:
(1) S56T;
(2) N57Q or N57D;
(3) E58L, E58P, or E58Y;
(4) G59P or G59L;
preferably, the modified TCR comprises amino acid substitutions S56T E58L G59P, S56T N57Q E58P G59L, or S56T N57D E58Y in CDR2β relative to the parental TCR.

In some embodiments, relative to the parental TCR, the modified TCR comprises one or more of the following amino acid substitutions in CDR3β: G112E, P113H, Y114L, E1151, and the replacement of EPGQGP with VNDGT at positions Glu108 to Pro113; preferably, the modified TCR comprises amino acid substitutions EPGQGP replaced with VNDGT or G112EP113HY114LE115I in CDR3β relative to the parental TCR.

In some embodiments, relative to the parental TCR, the modified TCR comprises one or more of the following amino acid substitutions (1)-(6) in CDR1α and/or FR1α:
(1) S28Y or S28P;
(2) S29T, S29L, or S29Y;
(3) S36D;
(4) T37I;
(5) Y38L;
(6) T26A or T26V;
preferably, the modified TCR comprises amino acid substitutions S29TS36DT371Y38L, T26AS28YS29L, or T26VS28PS29Y in CDR1α and/or FR1α relative to the parental TCR.

In some embodiments, relative to the parental TCR, the modified TCR comprises one or more of the following amino acid substitutions (1)-(6) in CDR2α:
(1) I56T or I56G;
(2) F57W or F57T;
(3) S58G, S58I, S58N, S58A, S58P, or S58W;
(4) N59F, N59T, N59H, or N59L;
(5) M63D, M63Q, M63T, or M63E;
(6) D64T, D64Y, or D64S;
preferably, the modified TCR comprises amino acid substitutions S58GN59FM63DD64T, S58IN59FM63QD64Y, S58NN59TM63Q, N59FM63TD64Y, S58AN59FM63ED64S, I56TF57WS58PN59H, I56GF57TS58WN59L, or 156TS58WN59F in CDR2α relative to the parental TCR.

In some embodiments, the modified TCR comprises:
(i) an alpha chain variable region comprising CDR1α, CDR2α, and CDR3α with amino acid sequences as shown in SEQ ID NOs:27-29; and
(ii) a beta chain variable region comprising:
   CDR1β, CDR2β, and CDR3β with amino acid sequences as shown in SEQ ID NOs:24, 25, and 79, respectively;
   CDR1β, CDR2β, and CDR3β with amino acid sequences as shown in SEQ ID NOs:82, 25, and 26, respectively;
   CDR1β, CDR2β, and CDR3β with amino acid sequences as shown in SEQ ID NOs:24, 85, and 26, respectively;
   CDR1β, CDR2β, and CDR3β with amino acid sequences as shown in SEQ ID NOs:24, 88, and 26, respectively;
   CDR1β, CDR2β, and CDR3β with amino acid sequences as shown in SEQ ID NOs:24, 91, and 26, respectively; or
   CDR1β, CDR2β, and CDR3β with amino acid sequences as shown in SEQ ID NOs:24, 25, and 94, respectively.

In some embodiments, the modified TCR comprises:
(i) a beta chain variable region comprising CDR1β, CDR2β, and CDR3β with amino acid sequences as shown in SEQ ID NOs:24-26, respectively; and
(ii) an alpha chain variable region comprising:
   CDR1α, CDR2α, and CDR3α with amino acid sequences as shown in SEQ ID NOs:97, 28, and 29, respectively;
   CDR1α, CDR2α, and CDR3α with amino acid sequences as shown in SEQ ID NOs:100, 28, and 29, respectively;
   CDR1α, CDR2α, and CDR3α with amino acid sequences as shown in SEQ ID NOs:103, 28, and 29, respectively;
   CDR1α, CDR2α, and CDR3α with amino acid sequences as shown in SEQ ID NOs:27, 106, and 29, respectively;
   CDR1α, CDR2α, and CDR3α with amino acid sequences as shown in SEQ ID NOs:27, 109, and 29, respectively;
   CDR1α, CDR2α, and CDR3α with amino acid sequences as shown in SEQ ID NOs:27, 112, and 29, respectively;
   CDR1α, CDR2α, and CDR3α with amino acid sequences as shown in SEQ ID NOs:27, 115, and 29, respectively;
   CDR1α, CDR2α, and CDR3α with amino acid sequences as shown in SEQ ID NOs:27, 118, and 29, respectively;
   CDR1α, CDR2α, and CDR3α with amino acid sequences as shown in SEQ ID NOs:27, 121, and 29, respectively;
   CDR1α, CDR2α, and CDR3α with amino acid sequences as shown in SEQ ID NOs:27, 124, and 29, respectively; or
   CDR1α, CDR2α, and CDR3α with amino acid sequences as shown in SEQ ID NOs:27, 127, and 29, respectively.

In some embodiments, the modified TCR comprises:
(i) an alpha chain variable region comprising:
   CDR1α, CDR2α, and CDR3α with amino acid sequences as shown in SEQ ID NOs:97, 28, and 29, respectively;
   CDR1α, CDR2α, and CDR3α with amino acid sequences as shown in SEQ ID NOs:100, 28, and 29, respectively;
   CDR1α, CDR2α, and CDR3α with amino acid sequences as shown in SEQ ID NOs:103, 28, and 29, respectively;
   CDR1α, CDR2α, and CDR3α with amino acid sequences as shown in SEQ ID NOs:27, 106, and 29, respectively;
   CDR1α, CDR2α, and CDR3α with amino acid sequences as shown in SEQ ID NOs:27, 109, and 29, respectively;
   CDR1α, CDR2α, and CDR3α with amino acid sequences as shown in SEQ ID NOs:27, 112, and 29, respectively;
   CDR1α, CDR2α, and CDR3α with amino acid sequences as shown in SEQ ID NOs:27, 115, and 29, respectively;
   CDR1α, CDR2α, and CDR3α with amino acid sequences as shown in SEQ ID NOs:27, 118, and 29, respectively;
   CDR1α, CDR2α, and CDR3α with amino acid sequences as shown in SEQ ID NOs:27, 121, and 29, respectively;
   CDR1α, CDR2α, and CDR3α with amino acid sequences as shown in SEQ ID NOs:27, 124, and 29, respectively; or
   CDR1α, CDR2α, and CDR3α with amino acid sequences as shown in SEQ ID NOs:27, 127, and 29, respectively; and
(ii) a beta chain variable region comprising:
   CDR1β, CDR2β, and CDR3β with amino acid sequences as shown in SEQ ID NOs:24, 25, and 79, respectively;
   CDR1β, CDR2β, and CDR3β with amino acid sequences as shown in SEQ ID NOs:82, 25, and 26, respectively;
   CDR1β, CDR2β, and CDR3β with amino acid sequences as shown in SEQ ID NOs:24, 85, and 26, respectively;
   CDR1β, CDR2β, and CDR3β with amino acid sequences as shown in SEQ ID NOs:24, 88, and 26, respectively;
   CDR1β, CDR2β, and CDR3β with amino acid sequences as shown in SEQ ID NOs:24, 91, and 26, respectively; or
   CDR1β, CDR2β, and CDR3β with amino acid sequences as shown in SEQ ID NOs:24, 25, and 94, respectively.

In some embodiments, the modified TCR comprises an alpha chain variable region having the amino acid sequence as shown in SEQ ID NO:46, or an amino acid sequence having at least 80%, at least 85%, at least 90%, at least 95%, or at least 99% sequence identity to SEQ ID NO:46, and a beta chain variable region having an amino acid sequence selected from the group consisting of SEQ ID NOs:80, 83, 86, 89, 92, or 95, or an amino acid sequence having at least 80%, at least 85%, at least 90%, at least 95%, or at least 99% sequence identity to any of SEQ ID NOs:80, 83, 86, 89, 92, or 95.

In some embodiments, the modified TCR comprises an alpha chain variable region having an amino acid sequence selected from the group consisting of SEQ ID NOs:98, 101, 104, 107, 110, 113, 116, 119, 122, 125, or 128, or an amino acid sequence having at least 80%, at least 85%, at least 90%, at least 95%, or at least 99% sequence identity to any of SEQ ID NOs:98, 101, 104, 107, 110, 113, 116, 119, 122, 125, or 128, and a beta chain variable region having the amino acid sequence as shown in SEQ ID NO:47, or an amino acid sequence having at least 80%, at least 85%, at least 90%, at least 95%, or at least 99% sequence identity to SEQ ID NO:47.

In some embodiments, the modified TCR comprises an alpha chain variable region having an amino acid sequence selected from the group consisting of SEQ ID NOs:98, 101, 104, 107, 110, 113, 116, 119, 122, 125, or 128, or an amino acid sequence having at least 80%, at least 85%, at least 90%, at least 95%, or at least 99% sequence identity to any of SEQ ID NOs:98, 101, 104, 107, 110, 113, 116, 119, 122, 125, or 128, and a beta chain variable region having an amino acid sequence selected from the group consisting of SEQ ID NOs:80, 83, 86, 89, 92, or 95, or an amino acid sequence having at least 80%, at least 85%, at least 90%, at least 95%, or at least 99% sequence identity to any of SEQ ID NOs:80, 83, 86, 89, 92, or 95.

In some embodiments, the modified TCR further comprises a TCR constant region. In some embodiments, the TCR constant region is a murine constant region or a human constant region. In some embodiments, the TCR constant region comprises a TCR alpha chain constant region and/or a TCR beta chain constant region. In a preferred embodiment, the TCR alpha chain constant region and/or TCR beta chain constant region comprises at least one cysteine substitution relative to the wild-type sequence to form a disulfide bond between the TCR alpha chain and beta chain. In a preferred embodiment, the TCR alpha chain constant region comprises an LVL substitution or LIV substitution, such that the alpha chain constant region comprises the amino acid sequence LLVIVLRIL.

In some embodiments, the modified TCR comprises an alpha chain having the amino acid sequence as shown in SEQ ID NO:69, or an amino acid sequence having at least 80%, at least 85%, at least 90%, at least 95%, or at least 99% sequence identity to SEQ ID NO:69, and a beta chain having an amino acid sequence selected from the group consisting of SEQ ID NOs:81, 84, 87, 90, 93, or 96, or an amino acid sequence having at least 80%, at least 85%, at least 90%, at least 95%, or at least 99% sequence identity to any of SEQ ID NOs:81, 84, 87, 90, 93, or 96.

In some embodiments, the modified TCR comprises an alpha chain having an amino acid sequence selected from the group consisting of SEQ ID NOs:99, 102, 105, 108, 111, 114, 117, 120, 123, 126, or 129, or an amino acid sequence having at least 80%, at least 85%, at least 90%, at least 95%, or at least 99% sequence identity to any of SEQ ID NOs:99, 102, 105, 108, 111, 114, 117, 120, 123, 126, or 129, and a beta chain having the amino acid sequence as shown in SEQ ID NO:70, or an amino acid sequence having at least 80%, at least 85%, at least 90%, at least 95%, or at least 99% sequence identity to SEQ ID NO:70.

In some embodiments, the modified TCR comprises an alpha chain having an amino acid sequence selected from the group consisting of SEQ ID NOs:99, 102, 105, 108, 111, 114, 117, 120, 123, 126, or 129, or an amino acid sequence having at least 80%, at least 85%, at least 90%, at least 95%, or at least 99% sequence identity to any of SEQ ID NOs:99, 102, 105, 108, 111, 114, 117, 120, 123, 126, or 129, and a beta chain having an amino acid sequence selected from the group consisting of SEQ ID NOs:81, 84, 87, 90, 93, or 96, or an amino acid sequence having at least 80%, at least 85%, at least 90%, at least 95%, or at least 99% sequence identity to any of SEQ ID NOs:81, 84, 87, 90, 93, or 96.

In some embodiments, the modified TCR binds to an epitope comprising the amino acid sequence KVLEYVIKV (SEQ ID NO:2) or to a complex of this epitope with an MHC molecule.

In some embodiments, the MHC molecule is HLA-A*02 type, such as HLA-A*02:01 type or HLA-A*02:05 type.

In some embodiments, the modified TCR is soluble or membrane-bound.

In some embodiments, the modified TCR is isolated or purified.

In another aspect, the disclosure provides a modified T-cell receptor (TCR) comprising:
(i) an alpha chain variable region comprising CDR1α, CDR2α, and CDR3α, and
(ii) a beta chain variable region comprising CDR1β, CDR2β, and CDR3β,
   wherein the modified TCR comprises the following amino acid substitutions relative to the parental TCR:
   (i) amino acid substitutions in the beta chain variable region comprising:
      (a) one or more substitutions at the following positions in CDR1β: Gln29, Ala36, Thr37, and Thr38,
      (b) one or more substitutions at the following positions in CDR2β: Ser56, Asn57, Glu58, Ser63, and Lys64, and/or
      (c) one or more substitutions at the following positions in CDR3β: Gly110, Gln111, Gly112, Pro113, Tyr114, and Glu115;
         and/or
   (ii) amino acid substitutions in the alpha chain variable region comprising:
      (d) one or more substitutions at the following positions in CDR1α: Asp27, Ser28, Ser29, and Ser36,
      (e) substitutions at the following positions in CDR2α: Ser58, Asn59, Met63, and Asp64, and/or
      (f) a substitution at position Thr26 in FR1α;
      wherein the parental TCR comprises CDR1β having an amino acid sequences as set forth in SEQ ID NO:24, CDR2β having an amino acid sequence as set forth in SEQ ID NO:25, CDR3β having an amino acid sequence as set forth in SEQ ID NO: 26, CDR1α having an amino acid sequence as set forth in SEQ ID NO:27, CDR2α having an amino acid sequence as set forth in SEQ ID NO:28, and CDR3α having an amino acid sequence as set forth in SEQ ID NO:29;
      wherein amino acid positions are numbered according to the International Immunogenetics Information System (IMGT) nomenclature.

In some embodiments, the modified TCR relative to the parental TCR comprises one or more of the following amino acid substitutions (1)-(24):
(1) substitution at position Gln29, selected from the group consisting of Q29E and Q29P;
(2) substitution at position Ala36: A36I;
(3) substitution at position Thr37: T37E;
(4) substitution at position Thr38: T38R;
(5) substitution at position Ser56: S56T;
(6) substitution at position Asn57: N57D;
(7) substitution at position Glu58: E58Y;
(8) substitution at position Ser63: S63D;
(9) substitution at position Lys64: K64Q;
(10) substitution at position Gly110: G110W;
(11) substitution at position Gln111: Q111D;
(12) substitution at position Gly 112: selected from the group consisting of G112P and G112A;
(13) substitution at position Pro113: P113W;
(14) substitution at position Tyr114: selected from the group consisting of Y114W, Y114S, and Y114F;
(15) substitution at position Glu115: E115D;
(16) substitution at position Asp27: selected from the group consisting of D27C and D27Y;
(17) substitution at position Ser28: selected from the group consisting of S28P, S28M, S28A, and S28G;
(18) substitution at position Ser29: selected from the group consisting of S29Y, S29L, S29F, S29E, and S29M;
(19) substitution at position Ser36: selected from the group consisting of S36L, S36Q, S36M, and S36F;
(20) substitution at position Ser58: selected from the group consisting of S58I and S58A;
(21) substitution at position Asn59: N59F;
(22) substitution at position Met63: selected from the group consisting of M63Q and M63E;
(23) substitution at position Asp64: selected from the group consisting of D64A, D64S, and D64Q;
(24) substitution at position Thr26: selected from the group consisting of T26V, T26S, T26I, T26P, and T26Q.

In some embodiments, relative to the parental TCR, the modified TCR comprises one or more of the following amino acid substitutions (1)-(4) in CDR1β:
(1) Q29E or Q29P;
(2) A36I;
(3) T37E; and
(4) T38R;
preferably, the modified TCR comprises amino acid substitutions Q29E or Q29PA36IT37E T38R in CDR1β.

In some embodiments, relative to the parental TCR, the modified TCR comprises one or more of the following amino acid substitutions in CDR2β: S56T, N57D, E58Y, S63D, and K64Q; preferably, relative to the parental TCR, the modified TCR comprises S56TN57DE58Y, or S63DK64Q in CDR2β.

In some embodiments, relative to the parental TCR, the modified TCR comprises one or more of the following amino acid substitutions (1)-(6) in CDR3β:
(1) G110W;
(2) Q111D;
(3) G112P or G112A;
(4) P113W;
(5) Y114W, Y114S, or Y114F;
(6) E115D;
preferably, relative to the parental TCR, the modified TCR comprises G110WQ111DG112PP113W, Y114W, G112AP113WY114SE115D, or Y114F in CDR3β.

In some embodiments, relative to the parental TCR, the modified TCR comprises one or more of the following amino acid substitutions (1)-(3):
(1) Q29E or Q29PA36IT37ET38R in CDR1β;
(2) S56TN57DE58Y or S63DK64Q in CDR2β;
(3) G110WQ111DG112PP113W, Y114W, G112AP113WY114SE115D, or Y114F in CDR3β.

In some embodiments, relative to the parental TCR, the modified TCR comprises one or more of the following amino acid substitutions (1)-(5) in CDR1α and/or FR1α:
(1) D27C or D27Y;
(2) S28P, S28M, S28A, or S28G;
(3) S29Y, S29L, S29F, S29E, or S29M;
(4) S36L, S36Q, S36M, or S36F;
(5) T26V, T26S, T26I, T26P, or T26Q;
preferably, relative to the parental TCR, the modified TCR comprises amino acid substitutions T26VS28PS29Y, S36L, T26SS28MS29L, T26ID27CS28AS29F, S36Q, T26PS28MS29F, S36M, S29ES36F, or T26QD27YS28GS29M in CDR1α and/or FR1α.

In some embodiments, the modified TCR comprises one or more of the following amino acid substitutions (1)-(4) in CDR2α relative to the parental TCR:
(1) S58I or S58A;
(2) N59F;
(3) M63Q or M63E;
(4) D64A, D64S, or D64Q;
preferably, relative to the parental TCR, the modified TCR comprises amino acid substitutions N59F, S58IN59FM63QD64A, S58AN59FM63ED64S, S58AN59FD64Q, or S58IN59FM63QD64A in CDR2α.

In some embodiments, the modified TCR comprises one or more of the following amino acid substitutions (1)-(2) relative to the parental TCR:
(1) amino acid substitutions in CDR1α and/or FR1α: T26VS28PS29Y, S36L, T26SS28MS29L, T26ID27CS28AS29F, S36Q, T26PS28MS29F, S36M, S29ES36F, or T26QD27YS28GS29M;
(2) amino acid substitutions in CDR2α: N59F, S58IN59FM63QD64A, S58AN59FM63ED64S, S58AN59FD64Q, or S58IN59FM63QD64A.

In some embodiments, the modified TCR comprises:
(i) an alpha chain variable region, which comprises:
   CDR1α, CDR2α, and CDR3α with amino acid sequences as shown in SEQ ID NOs:103, 76, and 29 respectively;
   CDR1α, CDR2α, and CDR3α with amino acid sequences as shown in SEQ ID NOs:44, 76, and 29 respectively;
   CDR1α, CDR2α, and CDR3α with amino acid sequences as shown in SEQ ID NOs:140, 141, and 29 respectively;
   CDR1α, CDR2α, and CDR3α with amino acid sequences as shown in SEQ ID NOs:146, 118, and 29 respectively;
   CDR1α, CDR2α, and CDR3α with amino acid sequences as shown in SEQ ID NOs:45, 149, and 29 respectively;
   CDR1α, CDR2α, and CDR3α with amino acid sequences as shown in SEQ ID NOs:152, 149, and 29 respectively;
   CDR1α, CDR2α, and CDR3α with amino acid sequences as shown in SEQ ID NOs:43, 76, and 29 respectively;
   CDR1α, CDR2α, and CDR3α with amino acid sequences as shown in SEQ ID NOs:45, 28, and 29 respectively;
   CDR1α, CDR2α, and CDR3α with amino acid sequences as shown in SEQ ID NOs:165, 118, and 29 respectively;
   CDR1α, CDR2α, and CDR3α with amino acid sequences as shown in SEQ ID NOs:27, 118, and 29 respectively;
   CDR1α, CDR2α, and CDR3α with amino acid sequences as shown in SEQ ID NOs:45, 141, and 29 respectively;
   CDR1α, CDR2α, and CDR3α with amino acid sequences as shown in SEQ ID NOs:170, 76, and 29 respectively;
   CDR1α, CDR2α, and CDR3α with amino acid sequences as shown in SEQ ID NOs:43, 118, and 29 respectively; or
   CDR1α, CDR2α, and CDR3α with amino acid sequences as shown in SEQ ID NOs:152, 149, and 29 respectively; and
(ii) a beta chain variable region, which comprises:
   CDR1β, CDR2β, and CDR3β with amino acid sequences as shown in SEQ ID NOs:130, 25, and 131 respectively;
   CDR1β, CDR2β, and CDR3β with amino acid sequences as shown in SEQ ID NOs:24, 91, and 35 respectively;
   CDR1β, CDR2β, and CDR3β with amino acid sequences as shown in SEQ ID NOs:130, 91, and 35 respectively;
   CDR1β, CDR2β, and CDR3β with amino acid sequences as shown in SEQ ID NOs:130, 153, and 35 respectively;
   CDR1β, CDR2β, and CDR3β with amino acid sequences as shown in SEQ ID NOs:130, 91, and 26 respectively;
   CDR1β, CDR2β, and CDR3β with amino acid sequences as shown in SEQ ID NOs:130, 91, and 162 respectively; or
   CDR1β, CDR2β, and CDR3β with amino acid sequences as shown in SEQ ID NOs:82, 91, and 33 respectively.

In some embodiments, the modified TCR comprises an alpha chain variable region having an amino acid sequence selected from the group consisting of SEQ ID NOs: 132, 136, 142, 147, 150, 154, 158, 68, 166, 119, 168, 171, 173 or an amino acid sequence with at least 80%, at least 85%, at least 90%, at least 95%, or at least 99% sequence identity to any of SEQ ID NOs: 132, 136, 142, 147, 150, 154, 158, 68, 166, 119, 168, 171, 173, and a beta chain variable region having an amino acid sequence selected from the group consisting of SEQ ID NOs: 133, 137, 143, 155, 159, 163, 175 or an amino acid sequence with at least 80%, at least 85%, at least 90%, at least 95%, or at least 99% sequence identity to any of SEQ ID NOs: 133, 137, 143, 155, 159, 163, 175.

In some embodiments, the modified TCR further comprises a TCR constant region. In some embodiments, the TCR constant region is a murine constant region or a human constant region. In some embodiments, the TCR constant region comprises a TCR alpha chain constant region and/or a TCR beta chain constant region. In a preferred embodiment, the TCR alpha chain constant region and/or TCR beta chain constant region comprises at least one cysteine substitution relative to the wild-type sequence to form a disulfide bond between the TCR alpha chain and TCR beta chain. In a preferred embodiment, the TCR alpha chain constant region comprises LVL substitution or LIV substitution, such that the alpha chain constant region comprises the amino acid sequence LLVIVLRIL.

In some embodiments, the modified TCR comprises an alpha chain having an amino acid sequence selected from the group consisting of SEQ ID NOs: 134, 138, 144, 148, 151, 156, 160, 23, 167, 120, 169, 172, 174 or an amino acid sequence with at least 80%, at least 85%, at least 90%, at least 95%, or at least 99% sequence identity to any of SEQ ID NOs: 134, 138, 144, 148, 151, 156, 160, 23, 167, 120, 169, 172, 174, and a beta chain having an amino acid sequence selected from the group consisting of SEQ ID NOs: 135, 139, 145, 157, 161, 164, 176 or an amino acid sequence with at least 80%, at least 85%, at least 90%, at least 95%, or at least 99% sequence identity to any of SEQ ID NOs: 135, 139, 145, 157, 161, 164, 176.

In some embodiments, the modified TCR binds to an epitope comprising the amino acid sequence KVLEYVIKV (SEQ ID NO:2) or a complex of this epitope with an MHC molecule.

In some embodiments, the MHC molecule is HLA-A*02 type, such as HLA-A*02:01 type or HLA-A*02:05 type.

In some embodiments, the modified TCR is soluble or membrane-bound.

In some embodiments, the modified TCR is isolated or purified.

On the other hand, the present disclosure provides a modified T cell receptor (TCR) comprising an alpha chain variable region comprising CDR1α, CDR2α, and CDR3α, and a beta chain variable region comprising CDR1β, CDR2β, and CDR3β, wherein the modified TCR comprises the following amino acid substitutions relative to the parental TCR:
(i) amino acid substitutions in the beta chain variable region comprising:
   (a) amino acid substitutions at one or more of the following positions in CDR1β: Gln29, Ala36, Thr37, and Thr38;
   (b) amino acid substitutions at one or more of the following positions in CDR2β: Ser56, Asn57, Glu58, Gly59, Ser63, Lys64, and Ala65; and/or
   (c) amino acid substitutions at one or more of the following positions in CDR3β: Ser105, Glu108, Pro109, Gly110, Gln111, Gly112, Pro113, Tyr114, and Glu115;
      and/or
(ii) amino acid substitutions in the alpha chain variable region comprising:
   (d) amino acid substitutions at one or more of the following positions in CDR1α: Ser28, Ser29, Ser36, Thr37, and Tyr38;
   (e) amino acid substitutions at the following positions in CDR2α: Ile56, Phe57, Ser58, Asn59, Met63, and Asp64; and/or
   (f) amino acid substitutions at position Thr26 in FR1α;
      wherein the parental TCR comprises the amino acid sequences of CDR1β as shown in SEQ ID NO: 24, CDR2β as shown in SEQ ID NO: 25, CDR3β as shown in SEQ ID NO: 26, CDR1α as shown in SEQ ID NO: 27, CDR2α as shown in SEQ ID NO: 28, and CDR3α as shown in SEQ ID NO: 29;
      wherein amino acid positions are numbered according to the International Immunogenetics Information System (IMGT) nomenclature.

In some embodiments, the modified TCR relative to the parental TCR comprises one or more of the following amino acid substitutions (1)-(31):
(1) amino acid substitution Q29P at position Gln29 in CDR1β;
(2) amino acid substitution A36I at position Ala36 in CDR1β;
(3) amino acid substitution T37E at position Thr37 in CDR1β;
(4) amino acid substitution T38R at position Thr38 in CDR1β;
(5) amino acid substitution S56T at position Ser56 in CDR2β;
(6) amino acid substitution at position Asn57 in CDR2β, selected from the group consisting of N57D, N57E, N57H, N57K, N57Q, N57R, N57S, and N57T;
(7) amino acid substitution at position Glu58 in CDR2β, selected from the group consisting of E58A, E58F, E58I, E58L, E58M, E58P, E58V, E58Y, and E58W;
(8) amino acid substitution at position Gly59 in CDR2β, selected from the group consisting of G59P, G59L, G59D, G59E, G59H, G59K, G59Q, G59R, G59S, and G59T;
(9) amino acid substitution at position Ser63 in CDR2β, selected from the group consisting of S63D, S63E, S63H, S63K, S63Q, S63R, and S63T;
(10) amino acid substitution at position Lys64 in CDR2β, selected from the group consisting of K64M, K64C, K64W, K64T, K64P, and K64A;
(11) amino acid substitution at position Ala65 in CDR2β, selected from the group consisting of A65F, A65I, A65L, A65M, A65P, A65V, A65W, and A65Y;
(12) amino acid substitution at position Ser105 in CDR3β, selected from the group consisting of S105L, S105N, S105H, S105Q, S105K, and S105R;
(13) amino acid substitution at position Glu108 in CDR3β, selected from the group consisting of E108D, E108F, E108T, E108I, E108K, and E108P;
(14) amino acid substitution at position Gln111 in CDR3β, selected from the group consisting of Q111N, Q111K, Q111S, Q111G, Q111A, Q111V, Q111E, Q111L, and Q111H;
(15) amino acid substitution EPGQGP replaced with VNDGT at positions Glu108 to Pro113 in CDR3β;
(16) amino acid substitution G112E at position Gly112 in CDR3β;
(17) amino acid substitution P113H at position Pro113 in CDR3β;
(18) amino acid substitution at position Tyr114 in CDR3β, selected from the group consisting of Y114I, Y114F, Y114K, Y114D, Y114L, Y114W, Y114N, Y114P, and Y114S;
(19) amino acid substitution E115I at position Glu115 in CDR3β;
(20) amino acid substitution at position Ser28 in CDR1α, selected from the group consisting of S28Y and S28P;
(21) amino acid substitution at position Ser29 in CDR1α, selected from the group consisting of S29T, S29Y, S29A, S29F, S29I, S29L, S29M, S29P, S29V, S29W, and S29N;
(22) amino acid substitution at position Ser36 in CDR1α, selected from the group consisting of S36D, S36M, S36L, S36Q, S36I, S36F, S36P, and S36V;
(23) amino acid substitution T37I at position Thr37 in CDR1α;
(24) amino acid substitution Y38L at position Tyr38 in CDR1α;
(25) amino acid substitution at position Ile56 in CDR2α, selected from the group consisting of I56T and I56G;
(26) amino acid substitution at position Phe57 in CDR2α, selected from the group consisting of F57W and F57T;
(27) amino acid substitution at position Ser58 in CDR2α, selected from the group consisting of S58G, S58I, S58N, S58A, S58P, and S58W;
(28) amino acid substitution at position Asn59 in CDR2α, selected from the group consisting of N59L, N59F, N59D, N59E, N59H, N59K, N59Q, N59R, N59S, and N59T;
(29) amino acid substitution at position Met63 in CDR2α, selected from the group consisting of M63D, M63Q, M63T, and M63E;
(30) amino acid substitution at position Asp64 in CDR2α, selected from the group consisting of D64T, D64Y, and D64S;
(31) amino acid substitution at position Thr26 in FR1α, selected from the group consisting of T26A and T26V.

In some embodiments, the modified TCR does not contain a single S105N amino acid substitution relative to the parental TCR.

In some embodiments, the modified TCR comprises one or more of the following amino acid substitutions in CDR1β relative to the parental TCR: Q29P, A36I, T37E, and T38R; preferably, the modified TCR comprises amino acid substitutions Q29P, A36I, T37E, and T38R in CDR1β relative to the parental TCR.

In some embodiments, the modified TCR comprises one or more of the following amino acid substitutions (1)-(7) in CDR2β relative to the parental TCR:
(1) S56T;
(2) N57Q or N57D;
(3) E58L, E58P, or E58Y;
(4) G59Q, G59P, or G59L;
(5) S63T;
(6) K64C;
(7) A65V;
preferably, the modified TCR comprises amino acid substitutions N57DE58YG59QS63TK64CA65V, S56TE58LG59P, S56TN57QE58PG59L or S56TN57DE58Y in CDR2β relative to the parental TCR.

In some embodiments, the modified TCR comprises one or more of the following amino acid substitutions (1)-(7) in CDR3β relative to the parental TCR:
(1) Q111G or Q111S;
(2) G112E;
(3) P113H;
(4) Y114F, Y114L, or Y114W;
(5) E115I;
(6) S105L, S105Q, S105N, or S105H;
(7) EPGQGP at positions Glu108 to Pro113 replaced with VNDGT;
preferably, the modified TCR comprises amino acid substitutions Y114F , Y114W , Q111GY114F, Q111SY114F, S105L, S105N, EPGQGP at positions Glu108 to Pro113 replaced with VNDGT, or G112EP113HY114LE115I in CDR3β relative to the parental TCR.

In some embodiments, relative to the parental TCR, the modified TCR comprises amino acid substitutions N57D, E58Y, G59Q, S63T, K64C, and A65V in CDR2β; and amino acid substitutions Y114F, Y114L, Y114W, Q111GY114F, or Q111SY114F in CDR3β.

In some embodiments, the modified TCR comprises one or more of the following amino acid substitutions (1)-(6) in CDR1α and/or FR1α relative to the parental TCR:
(1) S28Y or S28P;
(2) S29T, S29L, or S29Y;
(3) S36D, S36M, S36L, or S36Q;
(4) T37I;
(5) Y38L;
(6) T26A or T26V;
preferably, the modified TCR comprises amino acid substitutions S29TS36DT37IY38L, T26AS28YS29L or T26VS28PS29Y in CDR1α and/or FR1α relative to the parental TCR.

In some embodiments, the modified TCR comprises one or more of the following amino acid substitutions (1)-(6) in CDR2α relative to the parental TCR:
(1) I56T or I56G;
(2) F57W or F57T;
(3) S58G, S58I, S58N, S58A, S58P, or S58W;
(4) N59F, N59T, N59H, or N59L;
(5) M63D, M63Q, M63T, or M63E;
(6) D64T, D64Y, or D64S;
preferably, the modified TCR comprises amino acid substitutions S58GN59FM63DD64T , S58IN59FM63QD64Y , S58NN59TM63Q , N59FM63TD64Y , S58AN59FM63ED64S . I56TF57WS58PN59H, 156GF57TS58WN59L or I56TS58WN59F in CDR2α relative to the parental TCR.

In some embodiments, the modified TCR comprises:
(i) an α chain variable region comprising CDR1α, CDR2α, and CDR3α with amino acid sequences as shown in SEQ ID NOs:27-29 respectively; and
(ii) a β chain variable region comprising
   CDR1β, CDR2β, and CDR3β with amino acid sequences as shown in SEQ ID NOs:24, 30, and 26 respectively;
   CDR1β, CDR2β, and CDR3β with amino acid sequences as shown in SEQ ID NOs:24, 25, and 31 respectively;
   CDR1β, CDR2β, and CDR3β with amino acid sequences as shown in SEQ ID NOs:24, 25, and 32 respectively;
   CDR1β, CDR2β, and CDR3β with amino acid sequences as shown in SEQ ID NOs:24, 25, and 33 respectively;
   CDR1β, CDR2β, and CDR3β with amino acid sequences as shown in SEQ ID NOs:24, 25, and 34 respectively;
   CDR1β, CDR2β, and CDR3β with amino acid sequences as shown in SEQ ID NOs:24, 25, and 35 respectively;
   CDR1β, CDR2β, and CDR3β with amino acid sequences as shown in SEQ ID NOs:24, 30, and 33 respectively;
   CDR1β, CDR2β, and CDR3β with amino acid sequences as shown in SEQ ID NOs:24, 30, and 34 respectively;
   CDR1β, CDR2β, and CDR3β with amino acid sequences as shown in SEQ ID NOs:24, 30, and 35 respectively;
   CDR1β, CDR2β, and CDR3β with amino acid sequences as shown in SEQ ID NOs:24, 25, and 36 respectively;
   CDR1β, CDR2β, and CDR3β with amino acid sequences as shown in SEQ ID NOs:24, 25, and 37 respectively;
   CDR1β, CDR2β, and CDR3β with amino acid sequences as shown in SEQ ID NOs:24, 30, and 36 respectively;
   CDR1β, CDR2β, and CDR3β with amino acid sequences as shown in SEQ ID NOs:24, 30, and 37 respectively;
   CDR1β, CDR2β, and CDR3β with amino acid sequences as shown in SEQ ID NOs:24, 25, and 38 respectively;
   CDR1β, CDR2β, and CDR3β with amino acid sequences as shown in SEQ ID NOs:24, 25, and 39 respectively;
   CDR1β, CDR2β, and CDR3β with amino acid sequences as shown in SEQ ID NOs:24, 25, and 40 respectively;
   CDR1β, CDR2β, and CDR3β with amino acid sequences as shown in SEQ ID NOs:24, 25, and 41 respectively;
   CDR1β, CDR2β, and CDR3β with amino acid sequences as shown in SEQ ID NOs:24, 25, and 79 respectively;
   CDR1β, CDR2β, and CDR3β with amino acid sequences as shown in SEQ ID NOs:82, 25, and 26 respectively;
   CDR1β, CDR2β, and CDR3β with amino acid sequences as shown in SEQ ID NOs:24, 85, and 26 respectively;
   CDR1β, CDR2β, and CDR3β with amino acid sequences as shown in SEQ ID NOs:24, 88, and 26 respectively;
   CDR1β, CDR2β, and CDR3β with amino acid sequences as shown in SEQ ID NOs:24, 91, and 26 respectively; or
   CDR1β, CDR2β, and CDR3β with amino acid sequences as shown in SEQ ID NOs:24, 25, and 94 respectively.

In some embodiments, the modified TCR comprises:
(i) a β chain variable region comprising CDR1β, CDR2β, and CDR3β with amino acid sequences as shown in SEQ ID NOs:24-26 respectively; and
(ii) an α chain variable region comprising
   CDR1α, CDR2α, and CDR3α with amino acid sequences as shown in SEQ ID NOs:42, 28, and 29, respectively;
   CDR1α, CDR2α, and CDR3α with amino acid sequences as shown in SEQ ID NOs:43, 28, and 29, respectively;
   CDR1α, CDR2α, and CDR3α with amino acid sequences as shown in SEQ ID NOs:44, 28, and 29, respectively;
   CDR1α, CDR2α, and CDR3α with amino acid sequences as shown in SEQ ID NOs:45, 28, and 29, respectively;
   CDR1α, CDR2α, and CDR3α with amino acid sequences as shown in SEQ ID NOs:27, 76, and 29, respectively;
   CDR1α, CDR2α, and CDR3α with amino acid sequences as shown in SEQ ID NOs:97, 28, and 29, respectively;
   CDR1α, CDR2α, and CDR3α with amino acid sequences as shown in SEQ ID NOs:100, 28, and 29, respectively;
   CDR1α, CDR2α, and CDR3α with amino acid sequences as shown in SEQ ID NOs:103, 28, and 29, respectively;
   CDR1α, CDR2α, and CDR3α with amino acid sequences as shown in SEQ ID NOs:27, 106, and 29, respectively;
   CDR1α, CDR2α, and CDR3α with amino acid sequences as shown in SEQ ID NOs:27, 109, and 29, respectively;
   CDR1α, CDR2α, and CDR3α with amino acid sequences as shown in SEQ ID NOs:27, 112, and 29, respectively;
   CDR1α, CDR2α, and CDR3α with amino acid sequences as shown in SEQ ID NOs:27, 115, and 29, respectively;
   CDR1α, CDR2α, and CDR3α with amino acid sequences as shown in SEQ ID NOs:27, 118, and 29, respectively;
   CDR1α, CDR2α, and CDR3α with amino acid sequences as shown in SEQ ID NOs:27, 121, and 29, respectively;
   CDR1α, CDR2α, and CDR3α with amino acid sequences as shown in SEQ ID NOs:27, 124, and 29, respectively; or
   CDR1α, CDR2α, and CDR3α with amino acid sequences as shown in SEQ ID NOs:27, 127, and 29, respectively.

In some embodiments, the modified TCR comprises:
(i) an α chain variable region, comprising
   CDR1α, CDR2α, and CDR3α with amino acid sequences as shown in SEQ ID NOs:42, 28, and 29, respectively;
   CDR1α, CDR2α, and CDR3α with amino acid sequences as shown in SEQ ID NOs:43, 28, and 29, respectively;
   CDR1α, CDR2α, and CDR3α with amino acid sequences as shown in SEQ ID NOs:44, 28, and 29, respectively;
   CDR1α, CDR2α, and CDR3α with amino acid sequences as shown in SEQ ID NOs:45, 28, and 29, respectively;
   CDR1α, CDR2α, and CDR3α with amino acid sequences as shown in SEQ ID NOs:27, 76, and 29, respectively;
   CDR1α, CDR2α, and CDR3α with amino acid sequences as shown in SEQ ID NOs:97, 28, and 29, respectively;
   CDR1α, CDR2α, and CDR3α with amino acid sequences as shown in SEQ ID NOs:100, 28, and 29, respectively;
   CDR1α, CDR2α, and CDR3α with amino acid sequences as shown in SEQ ID NOs:103, 28, and 29, respectively;
   CDR1α, CDR2α, and CDR3α with amino acid sequences as shown in SEQ ID NOs:27, 106, and 29, respectively;
   CDR1α, CDR2α, and CDR3α with amino acid sequences as shown in SEQ ID NOs:27, 109, and 29, respectively;
   CDR1α, CDR2α, and CDR3α with amino acid sequences as shown in SEQ ID NOs:27, 112, and 29, respectively;
   CDR1α, CDR2α, and CDR3α with amino acid sequences as shown in SEQ ID NOs:27, 115, and 29, respectively;
   CDR1α, CDR2α, and CDR3α with amino acid sequences as shown in SEQ ID NOs:27, 118, and 29, respectively;
   CDR1α, CDR2α, and CDR3α with amino acid sequences as shown in SEQ ID NOs:27, 121, and 29, respectively;
   CDR1α, CDR2α, and CDR3α with amino acid sequences as shown in SEQ ID NOs:27, 124, and 29, respectively, or
   CDR1α, CDR2α, and CDR3α with amino acid sequences as shown in SEQ ID NOs:27, 127, and 29, respectively; and
(ii) a β chain variable region, comprising
   CDR1β, CDR2β, and CDR3β with amino acid sequences as shown in SEQ ID NOs:24, 30, and 26, respectively;
   CDR1β, CDR2β, and CDR3β with amino acid sequences as shown in SEQ ID NOs:24, 25, and 31, respectively;
   CDR1β, CDR2β, and CDR3β with amino acid sequences as shown in SEQ ID NOs:24, 25, and 32, respectively;
   CDR1β, CDR2β, and CDR3β with amino acid sequences as shown in SEQ ID NOs:24, 25, and 33, respectively;
   CDR1β, CDR2β, and CDR3β with amino acid sequences as shown in SEQ ID NOs:24, 25, and 34, respectively;
   CDR1β, CDR2β, and CDR3β with amino acid sequences as shown in SEQ ID NOs:24, 25, and 35, respectively;
   CDR1β, CDR2β, and CDR3β with amino acid sequences as shown in SEQ ID NOs:24, 30, and 33, respectively;
   CDR1β, CDR2β, and CDR3β with amino acid sequences as shown in SEQ ID NOs:24, 30, and 34, respectively;
   CDR1β, CDR2β, and CDR3β with amino acid sequences as shown in SEQ ID NOs:24, 30, and 35, respectively;
   CDR1β, CDR2β, and CDR3β with amino acid sequences as shown in SEQ ID NOs:24, 25, and 36, respectively;
   CDR1β, CDR2β, and CDR3β with amino acid sequences as shown in SEQ ID NOs:24, 25, and 37, respectively;
   CDR1β, CDR2β, and CDR3β with amino acid sequences as shown in SEQ ID NOs:24, 30, and 36, respectively;
   CDR1β, CDR2β, and CDR3β with amino acid sequences as shown in SEQ ID NOs:24, 30, and 37, respectively;
   CDR1β, CDR2β, and CDR3β with amino acid sequences as shown in SEQ ID NOs:24, 25, and 38, respectively;
   CDR1β, CDR2β, and CDR3β with amino acid sequences as shown in SEQ ID NOs:24, 25, and 39, respectively;
   CDR1β, CDR2β, and CDR3β with amino acid sequences as shown in SEQ ID NOs:24, 25, and 40, respectively;
   CDR1β, CDR2β, and CDR3β with amino acid sequences as shown in SEQ ID NOs:24, 25, and 41, respectively;
   CDR1β, CDR2β, and CDR3β with amino acid sequences as shown in SEQ ID NOs:24, 25, and 79, respectively;
   CDR1β, CDR2β, and CDR3β with amino acid sequences as shown in SEQ ID NOs:82, 25, and 26, respectively;
   CDR1β, CDR2β, and CDR3β with amino acid sequences as shown in SEQ ID NOs:24, 85, and 26, respectively;
   CDR1β, CDR2β, and CDR3β with amino acid sequences as shown in SEQ ID NOs:24, 88, and 26, respectively;
   CDR1β, CDR2β, and CDR3β with amino acid sequences as shown in SEQ ID NOs:24, 91, and 26, respectively, or
   CDR1β, CDR2β, and CDR3β with amino acid sequences as shown in SEQ ID NOs:24, 25, and 94, respectively.

In some embodiments, the modified TCR comprises an α chain variable region having an amino acid sequence as shown in SEQ ID NO:46 or an amino acid sequence with at least 80%, at least 85%, at least 90%, at least 95%, or at least 99% sequence identity to SEQ ID NO:46, and a β chain variable region having an amino acid sequence selected from any of SEQ ID NOs: 48-64, 80, 83, 86, 89, 92, 95 or an amino acid sequence with at least 80%, at least 85%, at least 90%, at least 95%, or at least 99% sequence identity to any of SEQ ID NOs: 48-64, 80, 83, 86, 89, 92, 95.

In some embodiments, the modified TCR comprises an α chain variable region having an amino acid sequence selected from any of SEQ ID NOs:65-68, 77, 98, 101, 104, 107, 110, 113, 116, 119, 122, 125, 128 or an amino acid sequence with at least 80%, at least 85%, at least 90%, at least 95%, or at least 99% sequence identity to any of SEQ ID NOs: 65-68, 77, 98, 101, 104, 107, 110, 113, 116, 119, 122, 125, 128, and a β chain variable region having an amino acid sequence as shown in SEQ ID NO:47 or an amino acid sequence with at least 80%, at least 85%, at least 90%, at least 95%, or at least 99% sequence identity to SEQ ID NO:47.

In some embodiments, the modified TCR comprises an α chain variable region having an amino acid sequence selected from any of SEQ ID NOs: 65-68, 77, 98, 101, 104, 107, 110, 113, 116, 119, 122, 125, 128 or an amino acid sequence with at least 80%, at least 85%, at least 90%, at least 95%, or at least 99% sequence identity to any of SEQ ID NOs: 65-68, 77, 98, 101, 104, 107, 110, 113, 116, 119, 122, 125, 128, and a β chain variable region having an amino acid sequence selected from any of SEQ ID NOs: 48-64, 80, 83, 86, 89, 92, 95 or an amino acid sequence with at least 80%, at least 85%, at least 90%, at least 95%, or at least 99% sequence identity to any of SEQ ID NOs: 48-64, 80, 83, 86, 89, 92, 95.

In some embodiments, the modified TCR further comprises a TCR constant region. In some embodiments, the TCR constant region is a murine constant region or a human constant region. In some embodiments, the TCR constant region comprises a TCR α chain constant region and/or a TCR β chain constant region. In a preferred embodiment, the TCR α chain constant region and/or TCR β chain constant region comprises at least one cysteine substitution relative to the wild-type sequence to form a disulfide bond between the TCR α chain and the TCR β chain. In a preferred embodiment, the TCR α chain constant region comprises an LVL substitution or an LIV substitution, such that the α chain constant region comprises the amino acid sequence LLVIVLRIL.

In some embodiments, the modified TCR comprises a TCR α chain having an amino acid sequence as shown in SEQ ID NO:69 or an amino acid sequence with at least 80%, at least 85%, at least 90%, at least 95%, or at least 99% sequence identity to SEQ ID NO:69, and a TCR β chain having an amino acid sequence selected from any of SEQ ID NOs: 3-19, 81, 84, 87, 90, 93, 96 or an amino acid sequence with at least 80%, at least 85%, at least 90%, at least 95%, or at least 99% sequence identity to any of SEQ ID NOs: 3-19, 81, 84, 87, 90, 93, 96.

In some embodiments, the modified TCR comprises a TCR α chain having an amino acid sequence selected from any of SEQ ID NOs:20-23, 78, 99, 102, 105, 108, 111, 114, 117, 120, 123, 126, 129 or an amino acid sequence with at least 80%, at least 85%, at least 90%, at least 95%, or at least 99% sequence identity to any of SEQ ID NOs: 20-23, 78, 99, 102, 105, 108, 111, 114, 117, 120, 123, 126, 129, and a TCR β chain having an amino acid sequence as shown in SEQ ID NO:70 or an amino acid sequence with at least 80%, at least 85%, at least 90%, at least 95%, or at least 99% sequence identity to SEQ ID NO:70.

In some embodiments, the modified TCR comprises a TCR α chain having an amino acid sequence selected from any of SEQ ID NOs: 20-23, 78, 99, 102, 105, 108, 111, 114, 117, 120, 123, 126, 129 or an amino acid sequence with at least 80%, at least 85%, at least 90%, at least 95%, or at least 99% sequence identity to any of SEQ ID NOs: 20-23, 78, 99, 102, 105, 108, 111, 114, 117, 120, 123, 126, 129, and a TCR β chain having an amino acid sequence selected from any of SEQ ID NOs: 3-19, 81, 84, 87, 90, 93, 96 or an amino acid sequence with at least 80%, at least 85%, at least 90%, at least 95%, or at least 99% sequence identity to any of SEQ ID NOs: 3-19, 81, 84, 87, 90, 93, 96.

In some embodiments, the modified TCR binds to an epitope comprising the amino acid sequence KVLEYVIKV (SEQ ID NO:2) or a complex of the epitope with an MHC molecule.

In some embodiments, the MHC molecule is of HLA-A*02 type, such as HLA-A*02:01 type or HLA-A*02:05 type.

In some embodiments, the modified TCR is soluble or membrane-bound.

In some embodiments, the modified TCR is isolated or purified.

In another aspect, the present disclosure provides a modified T cell receptor (TCR) comprising an α chain variable region comprising CDR1α, CDR2α, and CDR3α and a β chain variable region comprising CDR1β, CDR2β, and CDR3β, wherein the modified TCR comprises the following amino acid substitutions relative to the parental TCR:
(i) amino acid substitutions in the β chain variable region, comprising:
   (a) amino acid substitutions at one or more of the following positions in CDR1β: Gln29, Ala36, Thr37, and Thr38,
   (b) amino acid substitutions at one or more of the following positions in CDR2β: Ser56, Asn57, Glu58, Gly59, Ser63, Lys64, and Ala65, and/or
   (c) amino acid substitutions at one or more of the following positions in CDR3β: Ser105, Glu108, Pro109, Gly110, Gln111, Gly112, Pro113, Tyr114, and Glu115;
      and/or
(ii) amino acid substitutions in the α chain variable region, comprising:
   (d) amino acid substitutions at one or more of the following positions in CDR1α: Asp27, Ser28, Ser29, Ser36, Thr37, and Tyr38,
   (e) amino acid substitutions at the following positions in CDR2α: Ile56, Phe57, Ser58, Asn59, Met63, and Asp64, and/or
   (f) amino acid substitution at Thr26 in FR1α;
      wherein the parental TCR comprises CDR1βhaving an amino acid sequence as shown in SEQ ID NO: 24, CDR2βhaving an amino acid sequence as shown in SEQ ID NO: 25, CDR3βhaving an amino acid sequence as shown in SEQ ID NO: 26, CDR1αhaving an amino acid sequence as shown in SEQ ID NO: 27, CDR2αhaving an amino acid sequence as shown in SEQ ID NO: 28, and CDR3αhaving an amino acid sequence as shown in SEQ ID NO: 29;
      wherein amino acid positions are numbered according to the International ImMunoGeneTics Information System (IMGT) nomenclature.

In some embodiments, the modified TCR comprises one or more of the following amino acid substitutions (1)-(33) relative to the parental TCR:
(1) amino acid substitution at Gln29 in CDR1β, selected from the group consisting of Q29E and Q29P;
(2) amino acid substitution at Ala36 in CDR1β, A36I;
(3) amino acid substitution at Thr37 in CDR1β, T37E;
(4) amino acid substitution at Thr38 in CDR1β, T38R;
(5) amino acid substitution at Ser56 in CDR2β, S56T;
(6) amino acid substitution at Asn57 in CDR2β, selected from the group consisting ofN57D, N57E, N57H, N57K, N57Q, N57R, N57S, and N57T;
(7) amino acid substitution at Glu58 in CDR2β, selected from the group consisting of E58A, E58F, E58I, E58L, E58M, E58P, E58V, E58Y, and E58W;
(8) amino acid substitution at Gly59 in CDR2β, selected from the group consisting of G59P, G59L, G59D, G59E, G59H, G59K, G59Q, G59R, G59S, and G59T;
(9) amino acid substitution at Ser63 in CDR2β, selected from the group consisting of S63D, S63E, S63H, S63K, S63Q, S63R, and S63T;
(10) amino acid substitution at Lys64 in CDR2β, selected from the group consisting of K64Q, K64M, K64C, K64W, K64T, K64P, and K64A;
(11) amino acid substitution at Ala65 in CDR2β, selected from the group consisting of A65F, A65I, A65L, A65M, A65P, A65V, A65W, and A65Y;
(12) amino acid substitution at Ser105 in CDR3β, selected from the group consisting of S105L, S105N, S105H, S105Q, S105K, and S105R;
(13) amino acid substitution at Glu108 in CDR3β, selected from the group consisting of E108D, E108F, E108T, E108I, E108K, and E108P;
(14) amino acid substitution at Gly110 in CDR3β, G110W;
(15) amino acid substitution at Gln111 in CDR3β, selected from the group consisting of Q111D, Q111N, Q111K, Q111S, Q111G, Q111A, Q111V, Q111E, Q111L, and Q111H;
(16) amino acid substitutions at positions Glu108 to Pro113 in CDR3β, EPGQGP replaced with VNDGT;
(17) amino acid substitution at Gly112 in CDR3β, selected from the group consisting of G112P, G112A, and G112E;
(18) amino acid substitution at Pro113 in CDR3β, selected from the group consisting of P113W and P113H;
(19) amino acid substitution at Tyr114 in CDR3β, selected from the group consisting of Y114I, Y114F, Y114K, Y114D, Y114L, Y114W, Y114N, Y114P, and Y114S;
(20) amino acid substitution at Glu115 in CDR3β, selected from the group consisting of E115D and E115I;
(21) amino acid substitution at Asp27 in CDR1α, selected from the group consisting of D27C and D27Y;
(22) amino acid substitution at Ser28 in CDR1α, selected from the group consisting of S28M, S28A, S28G, S28Y, and S28P;
(23) amino acid substitution at Ser29 in CDR1α, selected from the group consisting of S29E, S29T, S29Y, S29A, S29F, S29I, S29L, S29M, S29P, S29V, S29W, and S29N;
(24) amino acid substitution at Ser36 in CDR1α, selected from the group consisting of S36D, S36M, S36L, S36Q, S36I, S36F, S36P, and S36V;
(25) amino acid substitution at Thr37 in CDR1α, T37I;
(26) amino acid substitution at Tyr38 in CDR1α, Y38L;
(27) amino acid substitution at Ile56 in CDR2α, selected from the group consisting of I56T and I56G;
(28) amino acid substitution at Phe57 in CDR2α, selected from the group consisting of F57W and F57T;
(29) amino acid substitution at Ser58 in CDR2α, selected from the group consisting of S58G, S58I, S58N, S58A, S58P, and S58W;
(30) amino acid substitution at Asn59 in CDR2α, selected from the group consisting of N59L, N59F, N59D, N59E, N59H, N59K, N59Q, N59R, N59S, and N59T;
(31) amino acid substitution at Met63 in CDR2α, selected from the group consisting of M63D, M63Q, M63T, and M63E;
(32) amino acid substitution at Asp64 in CDR2α, selected from the group consisting of D64A, D64Q, D64T, D64Y, and D64S;
(33) amino acid substitution at Thr26 in FR1α, selected from the group consisting of T26S, T26I, T26P, T26Q, T26A, and T26V.

In some embodiments, the modified TCR does not contain a single S105N amino acid substitution relative to the parental TCR.

In some embodiments, the modified TCR comprises one or more of the following amino acid substitutions (1)-(4) in CDR1β relative to the parental TCR:
(1) Q29E or Q29P;
(2) A36I;
(3) T37E;
(4) T38R;
preferably, the modified TCR comprises the amino acid substitutions Q29E or Q29PA36IT37ET38R in CDR1β relative to the parental TCR.

In some embodiments, the modified TCR comprises one or more of the following amino acid substitutions (1)-(7) in CDR2β relative to the parental TCR:
(1) S56T;
(2) N57Q or N57D;
(3) E58L, E58P, or E58Y;
(4) G59Q, G59P, or G59L;
(5) S63T or S63D;
(6) K64C or K64Q;
(7) A65V;
preferably, the modified TCR comprises the amino acid substitutions N57DE58YG59QS63TK64CA65V, S56TE58LG59P, S56TN57QE58PG59L, S56TN57DE58Y, or S63DK64Q in CDR2β relative to the parental TCR.

In some embodiments, the modified TCR comprises one or more of the following amino acid substitutions (1)-(8) in CDR3β relative to the parental TCR:
(1) G110W;
(2) Q111D, Q111G, or Q111S;
(3) G112E, G112P, or G112A;
(4) P113H or P113W;
(5) Y114S, Y114F, Y114L, or Y114W;
(6) E115I or E115D;
(7) S105L, S105Q, S105N, or S105H;
(8) EPGQGP replaced with VNDGT at position Glu108 to Pro113;
preferably, the modified TCR comprises the amino acid substitutions: G110WQ111DG112PP113W, G112AP113WY114SE115D, Y114F, Y114W, Q111GY114F, Q111SY114F, S105L, S105N, EPGQGP replaced with VNDGT at position Glu108 to Pro113, or G112EP113HY114LE1151 in CDR3β relative to the parental TCR.

In some embodiments, the modified TCR comprises one or more of the following amino acid substitutions (1)-(3) relative to the parental TCR:
(1) amino acid substitutions in CDR1β: Q29E or Q29PA36IT37ET38R;
(2) amino acid substitutions in CDR2β: N57DE58YG59QS63TK64CA65V, S56TE58LG59P, S56TN57QE58PG59L, S56TN57DE58Y, or S63DK64Q;
(3) amino acid substitutions in CDR3β: G110WQ111DG112PP113W, G112AP113WY114SE115D, Y114F, Y114W, Q111GY114F, Q111SY114F, S105L, S105N, EPGQGP replaced with VNDGT at position Glu108 to Pro113, or G112EP113HY114LE115I.

In some embodiments, relative to the parental TCR, the modified TCR comprises the amino acid substitutions N57D, E58Y, G59Q, S63T, K64C, and A65V in CDR2β; and Y114F, Y114L, Y114W, Q111GY114F, or Q111SY114F in CDR3β.

In some embodiments, the modified TCR comprises one or more of the following amino acid substitutions (1)-(7) in CDR1α and/or FR1α relative to the parental TCR:
(1) D27C or D27Y;
(2) S28Y, S28P, S28M, S28A, or S28G;
(3) S29T, S29L, S29Y, S29F, S29E, or S29M;
(4) S36F, S36D, S36M, S36L, or S36Q;
(5) T37I;
(6) Y38L;
(7) T26A, T26V, T26S, T26I, T26P, or T26Q;
preferably, the modified TCR comprises the amino acid substitutions: S29TS36DT371Y38L, T26AS28YS29L, T26VS28PS29Y, S36L, T26SS28MS29L, T26ID27CS28AS29F, S36Q, T26PS28MS29F, S36M, S29ES36F, or T26QD27YS28GS29M in CDR1α and/or FR1α relative to the parental TCR.

In some embodiments, the modified TCR comprises one or more of the following amino acid substitutions (1)-(6) in CDR2α relative to the parental TCR:
(1) I56T or I56G;
(2) F57W or F57T;
(3) S58G, S58I, S58N, S58A, S58P, or S58W;
(4) N59F, N59T, N59H, or N59L;
(5) M63D, M63Q, M63T, or M63E;
(6) D64A, D64Q, D64T, D64Y, or D64S;
preferably, the modified TCR comprises the amino acid substitutions N59F, S58IN59FM63QD64A, S58AN59FD64Q, S58IN59FM63QD64A, S58GN59FM63DD64T, S58IN59FM63QD64Y, S58NN59TM63Q, N59FM63TD64Y, S58AN59FM63ED64S, I56TF57WS58PN59H, 156GF57TS58WN59L, or I56TS58WN59F in CDR2α relative to the parental TCR.

In some embodiments, the modified TCR comprises one or more of the following amino acid substitutions (1)-(2) relative to the parental TCR:
(1) amino acid substitutions in CDR1α and/or FR1α: S29TS36DT37IY38L, T26AS28YS29L, T26VS28PS29Y, S36L, T26SS28MS29L, T26ID27CS28AS29F, S36Q, T26PS28MS29F, S36M, S29ES36F, or T26QD27YS28GS29M;
(2) amino acid substitutions in CDR2α: N59F, S58IN59FM63QD64A, S58AN59FD64Q, S58IN59FM63QD64A, S58GN59FM63DD64T, S58IN59FM63QD64Y, S58NN59TM63Q, N59FM63TD64Y, S58AN59FM63ED64S, I56TF57WS58PN59H, 156GF57TS58WN59L, or 156TS58WN59F.

In some embodiments, the modified TCR comprises:
(i) an α chain variable region , comprising CDR1α, CDR2α, and CDR3α with amino acid sequences as shown in SEQ ID NOs: 27-29, respectively; and
(ii) a β chain variable region, comprising:
   CDR1β, CDR2β, and CDR3β with amino acid sequences as shown in SEQ ID NOs:24; 30; and 26, respectively;
   CDR1β, CDR2β, and CDR3β with amino acid sequences as shown in SEQ ID NOs:24; 25; and 31, respectively;
   CDR1β, CDR2β, and CDR3β with amino acid sequences as shown in SEQ ID NOs:24; 25; and 32, respectively;
   CDR1β, CDR2β, and CDR3β with amino acid sequences as shown in SEQ ID NOs:24; 25; and 33, respectively;
   CDR1β, CDR2β, and CDR3β with amino acid sequences as shown in SEQ ID NOs:24; 25; and 34, respectively;
   CDR1β, CDR2β, and CDR3β with amino acid sequences as shown in SEQ ID NOs:24; 25; and 35, respectively;
   CDR1β, CDR2β, and CDR3β with amino acid sequences as shown in SEQ ID NOs:24; 30; and 33, respectively;
   CDR1β, CDR2β, and CDR3β with amino acid sequences as shown in SEQ ID NOs:24; 30; and 34, respectively;
   CDR1β, CDR2β, and CDR3β with amino acid sequences as shown in SEQ ID NOs:24; 30; and 35, respectively;
   CDR1β, CDR2β, and CDR3β with amino acid sequences as shown in SEQ ID NOs:24; 25; and 36, respectively;
   CDR1β, CDR2β, and CDR3β with amino acid sequences as shown in SEQ ID NOs:24; 25; and 37, respectively;
   CDR1β, CDR2β, and CDR3β with amino acid sequences as shown in SEQ ID NOs:24; 30; and 36, respectively;
   CDR1β, CDR2β, and CDR3β with amino acid sequences as shown in SEQ ID NOs:24; 30; and 37, respectively;
   CDR1β, CDR2β, and CDR3β with amino acid sequences as shown in SEQ ID NOs:24; 25; and 38, respectively;
   CDR1β, CDR2β, and CDR3β with amino acid sequences as shown in SEQ ID NOs:24; 25; and 39, respectively;
   CDR1β, CDR2β, and CDR3β with amino acid sequences as shown in SEQ ID NOs:24; 25; and 40, respectively;
   CDR1β, CDR2β, and CDR3β with amino acid sequences as shown in SEQ ID NOs:24; 25; and 41, respectively;
   CDR1β, CDR2β, and CDR3β with amino acid sequences as shown in SEQ ID NOs:24; 25; and 79, respectively;
   CDR1β, CDR2β, and CDR3β with amino acid sequences as shown in SEQ ID NOs:82; 25; and 26, respectively;
   CDR1β, CDR2β, and CDR3β with amino acid sequences as shown in SEQ ID NOs:24; 85; and 26, respectively;
   CDR1β, CDR2β, and CDR3β with amino acid sequences as shown in SEQ ID NOs:24; 88; and 26, respectively;
   CDR1β, CDR2β, and CDR3β with amino acid sequences as shown in SEQ ID NOs:24; 91; and 26, respectively;
   CDR1β, CDR2β, and CDR3β with amino acid sequences as shown in SEQ ID NOs:24; 25; and 94, respectively;
   CDR1β, CDR2β, and CDR3β with amino acid sequences as shown in SEQ ID NOs:130; 25; and 131, respectively;
   CDR1β, CDR2β, and CDR3β with amino acid sequences as shown in SEQ ID NOs:24; 91; and 35, respectively;
   CDR1β, CDR2β, and CDR3β with amino acid sequences as shown in SEQ ID NOs:130; 91; and 35, respectively;
   CDR1β, CDR2β, and CDR3β with amino acid sequences as shown in SEQ ID NOs:130; 153; and 35, respectively;
   CDR1β, CDR2β, and CDR3β with amino acid sequences as shown in SEQ ID NOs:130; 91; and 26, respectively;
   CDR1β, CDR2β, and CDR3β with amino acid sequences as shown in SEQ ID NOs:130; 91; and 162, respectively;
   CDR1β, CDR2β, and CDR3β with amino acid sequences as shown in SEQ ID NOs:82, 91, and 33, respectively.

In some embodiments, the modified TCR comprises:
(i) a β-chain variable region comprising CDR1β, CDR2β, and CDR3β with amino acid sequences as shown in SEQ ID NOs:24-26, respectively; and
(ii) an α-chain variable region comprising:
   CDR1α, CDR2α, and CDR3α with amino acid sequences as shown in SEQ ID NOs:42, 28, and 29, respectively;
   CDR1α, CDR2α, and CDR3α with amino acid sequences as shown in SEQ ID NOs:43, 28, and 29, respectively;
   CDR1α, CDR2α, and CDR3α with amino acid sequences as shown in SEQ ID NOs:44, 28, and 29, respectively;
   CDR1α, CDR2α, and CDR3α with amino acid sequences as shown in SEQ ID NOs:45, 28, and 29, respectively;
   CDR1α, CDR2α, and CDR3α with amino acid sequences as shown in SEQ ID NOs:27, 76, and 29, respectively;
   CDR1α, CDR2α, and CDR3α with amino acid sequences as shown in SEQ ID NOs:97, 28, and 29, respectively;
   CDR1α, CDR2α, and CDR3α with amino acid sequences as shown in SEQ ID NOs:100, 28, and 29, respectively;
   CDR1α, CDR2α, and CDR3α with amino acid sequences as shown in SEQ ID NOs:103, 28, and 29, respectively;
   CDR1α, CDR2α, and CDR3α with amino acid sequences as shown in SEQ ID NOs:27, 106, and 29, respectively;
   CDR1α, CDR2α, and CDR3α with amino acid sequences as shown in SEQ ID NOs:27, 109, and 29, respectively;
   CDR1α, CDR2α, and CDR3α with amino acid sequences as shown in SEQ ID NOs:27, 112, and 29, respectively;
   CDR1α, CDR2α, and CDR3α with amino acid sequences as shown in SEQ ID NOs:27, 115, and 29, respectively;
   CDR1α, CDR2α, and CDR3α with amino acid sequences as shown in SEQ ID NOs:27, 118, and 29, respectively;
   CDR1α, CDR2α, and CDR3α with amino acid sequences as shown in SEQ ID NOs:27, 121, and 29, respectively;
   CDR1α, CDR2α, and CDR3α with amino acid sequences as shown in SEQ ID NOs:27, 124, and 29, respectively;
   CDR1α, CDR2α, and CDR3α with amino acid sequences as shown in SEQ ID NOs:27, 127, and 29, respectively;
   CDR1α, CDR2α, and CDR3α with amino acid sequences as shown in SEQ ID NOs:103, 76, and 29, respectively;
   CDR1α, CDR2α, and CDR3α with amino acid sequences as shown in SEQ ID NOs:44, 76, and 29, respectively;
   CDR1α, CDR2α, and CDR3α with amino acid sequences as shown in SEQ ID NOs:140, 141, and 29, respectively;
   CDR1α, CDR2α, and CDR3α with amino acid sequences as shown in SEQ ID NOs:146, 118, and 29, respectively;
   CDR1α, CDR2α, and CDR3α with amino acid sequences as shown in SEQ ID NOs:45, 149, and 29, respectively;
   CDR1α, CDR2α, and CDR3α with amino acid sequences as shown in SEQ ID NOs:152, 149, and 29, respectively;
   CDR1α, CDR2α, and CDR3α with amino acid sequences as shown in SEQ ID NOs:43, 76, and 29, respectively;
   CDR1α, CDR2α, and CDR3α with amino acid sequences as shown in SEQ ID NOs:45, 28, and 29, respectively;
   CDR1α, CDR2α, and CDR3α with amino acid sequences as shown in SEQ ID NOs:165, 118, and 29, respectively;
   CDR1α, CDR2α, and CDR3α with amino acid sequences as shown in SEQ ID NOs:27, 118, and 29, respectively;
   CDR1α, CDR2α, and CDR3α with amino acid sequences as shown in SEQ ID NOs:45, 141, and 29, respectively;
   CDR1α, CDR2α, and CDR3α with amino acid sequences as shown in SEQ ID NOs:170, 76, and 29, respectively;
   CDR1α, CDR2α, and CDR3α with amino acid sequences as shown in SEQ ID NOs:43, 118, and 29, respectively; or
   CDR1α, CDR2α, and CDR3α with amino acid sequences as shown in SEQ ID NOs:152, 149, and 29, respectively.

In some embodiments, the modified TCR comprises:
(i) an α-chain variable region, comprising:
   CDR1α, CDR2α, and CDR3α with amino acid sequences as shown in SEQ ID NOs:42, 28, and 29, respectively;
   CDR1α, CDR2α, and CDR3α with amino acid sequences as shown in SEQ ID NOs:43, 28, and 29, respectively;
   CDR1α, CDR2α, and CDR3α with amino acid sequences as shown in SEQ ID NOs:44, 28, and 29, respectively;
   CDR1α, CDR2α, and CDR3α with amino acid sequences as shown in SEQ ID NOs:45, 28, and 29, respectively;
   CDR1α, CDR2α, and CDR3α with amino acid sequences as shown in SEQ ID NOs:27, 76, and 29, respectively;
   CDR1α, CDR2α, and CDR3α with amino acid sequences as shown in SEQ ID NOs:97, 28, and 29, respectively;
   CDR1α, CDR2α, and CDR3α with amino acid sequences as shown in SEQ ID NOs:100, 28, and 29, respectively;
   CDR1α, CDR2α, and CDR3α with amino acid sequences as shown in SEQ ID NOs:103, 28, and 29, respectively;
   CDR1α, CDR2α, and CDR3α with amino acid sequences as shown in SEQ ID NOs:27, 106, and 29, respectively;
   CDR1α, CDR2α, and CDR3α with amino acid sequences as shown in SEQ ID NOs:27, 109, and 29, respectively;
   CDR1α, CDR2α, and CDR3α with amino acid sequences as shown in SEQ ID NOs:27, 112, and 29, respectively;
   CDR1α, CDR2α, and CDR3α with amino acid sequences as shown in SEQ ID NOs:27, 115, and 29, respectively;
   CDR1α, CDR2α, and CDR3α with amino acid sequences as shown in SEQ ID NOs:27, 118, and 29, respectively;
   CDR1α, CDR2α, and CDR3α with amino acid sequences as shown in SEQ ID NOs:27, 121, and 29, respectively;
   CDR1α, CDR2α, and CDR3α with amino acid sequences as shown in SEQ ID NOs:27, 124, and 29, respectively;
   CDR1α, CDR2α, and CDR3α with amino acid sequences as shown in SEQ ID NOs:27, 127, and 29, respectively;
   CDR1α, CDR2α, and CDR3α with amino acid sequences as shown in SEQ ID NOs:103, 76, and 29, respectively;
   CDR1α, CDR2α, and CDR3α with amino acid sequences as shown in SEQ ID NOs:44, 76, and 29, respectively;
   CDR1α, CDR2α, and CDR3α with amino acid sequences as shown in SEQ ID NOs:140, 141, and 29, respectively;
   CDR1α, CDR2α, and CDR3α with amino acid sequences as shown in SEQ ID NOs:146, 118, and 29, respectively;
   CDR1α, CDR2α, and CDR3α with amino acid sequences as shown in SEQ ID NOs:45, 149, and 29, respectively;
   CDR1α, CDR2α, and CDR3α with amino acid sequences as shown in SEQ ID NOs:152, 149, and 29, respectively;
   CDR1α, CDR2α, and CDR3α with amino acid sequences as shown in SEQ ID NOs:43, 76, and 29, respectively;
   CDR1α, CDR2α, and CDR3α with amino acid sequences as shown in SEQ ID NOs:45, 28, and 29, respectively;
   CDR1α, CDR2α, and CDR3α with amino acid sequences as shown in SEQ ID NOs:165, 118, and 29, respectively;
   CDR1α, CDR2α, and CDR3α with amino acid sequences as shown in SEQ ID NOs:27, 118, and 29, respectively;
   CDR1α, CDR2α, and CDR3α with amino acid sequences as shown in SEQ ID NOs:45, 141, and 29, respectively;
   CDR1α, CDR2α, and CDR3α with amino acid sequences as shown in SEQ ID NOs:170, 76, and 29, respectively;
   CDR1α, CDR2α, and CDR3α with amino acid sequences as shown in SEQ ID NOs:43, 118, and 29, respectively; or
   CDR1α, CDR2α, and CDR3α with amino acid sequences as shown in SEQ ID NOs:152, 149, and 29, respectively; and
(ii) a β chain variable region, comprising:
   CDR1β, CDR2β, and CDR3β with amino acid sequences as shown in SEQ ID NOs:24, 30, and 26, respectively;
   CDR1β, CDR2β, and CDR3β with amino acid sequences as shown in SEQ ID NOs:24, 25, and 31, respectively;
   CDR1β, CDR2β, and CDR3β with amino acid sequences as shown in SEQ ID NOs:24, 25, and 32, respectively;
   CDR1β, CDR2β, and CDR3β with amino acid sequences as shown in SEQ ID NOs:24, 25, and 33, respectively;
   CDR1β, CDR2β, and CDR3β with amino acid sequences as shown in SEQ ID NOs:24, 25, and 34, respectively;
   CDR1β, CDR2β, and CDR3β with amino acid sequences as shown in SEQ ID NOs:24, 25, and 35, respectively;
   CDR1β, CDR2β, and CDR3β with amino acid sequences as shown in SEQ ID NOs:24, 30, and 33, respectively;
   CDR1β, CDR2β, and CDR3β with amino acid sequences as shown in SEQ ID NOs:24, 30, and 34, respectively;
   CDR1β, CDR2β, and CDR3β with amino acid sequences as shown in SEQ ID NOs:24, 30, and 35, respectively;
   CDR1β, CDR2β, and CDR3β with amino acid sequences as shown in SEQ ID NOs:24, 25, and 36, respectively;
   CDR1β, CDR2β, and CDR3β with amino acid sequences as shown in SEQ ID NOs:24, 25, and 37, respectively;
   CDR1β, CDR2β, and CDR3β with amino acid sequences as shown in SEQ ID NOs:24, 30, and 36, respectively;
   CDR1β, CDR2β, and CDR3β with amino acid sequences as shown in SEQ ID NOs:24, 30, and 37, respectively;
   CDR1β, CDR2β, and CDR3β with amino acid sequences as shown in SEQ ID NOs:24, 25, and 38, respectively;
   CDR1β, CDR2β, and CDR3β with amino acid sequences as shown in SEQ ID NOs:24, 25, and 39, respectively;
   CDR1β, CDR2β, and CDR3β with amino acid sequences as shown in SEQ ID NOs:24, 25, and 40, respectively;
   CDR1β, CDR2β, and CDR3β with amino acid sequences as shown in SEQ ID NOs:24, 25, and 41, respectively;
   CDR1β, CDR2β, and CDR3β with amino acid sequences as shown in SEQ ID NOs:24, 25, and 79, respectively;
   CDR1β, CDR2β, and CDR3β with amino acid sequences as shown in SEQ ID NOs:82, 25, and 26, respectively;
   CDR1β, CDR2β, and CDR3β with amino acid sequences as shown in SEQ ID NOs:24, 85, and 26, respectively;
   CDR1β, CDR2β, and CDR3β with amino acid sequences as shown in SEQ ID NOs:24, 88, and 26, respectively;
   CDR1β, CDR2β, and CDR3β with amino acid sequences as shown in SEQ ID NOs:24, 91, and 26, respectively;
   CDR1β, CDR2β, and CDR3β with amino acid sequences as shown in SEQ ID NOs:24, 25, and 94, respectively;
   CDR1β, CDR2β, and CDR3β with amino acid sequences as shown in SEQ ID NOs:130, 25, and 131, respectively;
   CDR1β, CDR2β, and CDR3β with amino acid sequences as shown in SEQ ID NOs:24, 91, and 35, respectively;
   CDR1β, CDR2β, and CDR3β with amino acid sequences as shown in SEQ ID NOs:130, 91, and 35, respectively;
   CDR1β, CDR2β, and CDR3β with amino acid sequences as shown in SEQ ID NOs:130, 153, and 35, respectively;
   CDR1β, CDR2β, and CDR3β with amino acid sequences as shown in SEQ ID NOs:130, 91, and 26, respectively;
   CDR1β, CDR2β, and CDR3β with amino acid sequences as shown in SEQ ID NOs:130, 91, and 162, respectively; or
   CDR1β, CDR2β, and CDR3β with amino acid sequences as shown in SEQ ID NOs:82, 91, and 33, respectively.

In some embodiments, the modified TCR comprises an α chain variable region having the amino acid sequence as shown in SEQ ID NO: 46 or an amino acid sequence with at least 80%, at least 85%, at least 90%, at least 95%, or at least 99% sequence identity to SEQ ID NO: 46, and a β chain variable region having an amino acid sequence selected from any of SEQ ID NOs: 48-64, 80, 83, 86, 89, 92, 95, 133, 137, 143, 155, 159, 163, and 175 or an amino acid sequence with at least 80%, at least 85%, at least 90%, at least 95%, or at least 99% sequence identity to any of SEQ ID NOs: 48-64, 80, 83, 86, 89, 92, 95, 133, 137, 143, 155, 159, 163, and 175.

In some embodiments, the modified TCR comprises an α chain variable region having an amino acid sequence selected from any of SEQ ID NOs: 65-68, 77, 98, 101, 104, 107, 110, 113, 116, 119, 122, 125, 128, 132, 136, 142, 147, 150, 154, 158, 166, 168, 171, and 173 or an amino acid sequence with at least 80%, at least 85%, at least 90%, at least 95%, or at least 99% sequence identity to any of SEQ ID NOs: 65-68, 77, 98, 101, 104, 107, 110, 113, 116, 119, 122, 125, 128, 132, 136, 142, 147, 150, 154, 158, 166, 168, 171, and 173, and a β chain variable region having an amino acid sequence as shown in SEQ ID NO: 47 or an amino acid sequence with at least 80%, at least 85%, at least 90%, at least 95%, or at least 99% sequence identity to SEQ ID NO: 47.

In some embodiments, the modified TCR comprises an α chain variable region having an amino acid sequence selected from any of SEQ ID NOs: 65-68, 77, 98, 101, 104, 107, 110, 113, 116, 119, 122, 125, 128, 132, 136, 142, 147, 150, 154, 158, 166, 168, 171, and 173 or an amino acid sequence with at least 80%, at least 85%, at least 90%, at least 95%, or at least 99% sequence identity to any of SEQ ID NOs: 65-68, 77,98, 101, 104, 107, 110, 113, 116, 119, 122, 125, 128, 132, 136, 142, 147, 150, 154, 158, 166, 168, 171, and 173, and a β chain variable region having an amino acid sequence selected from any of SEQ ID NOs: 48-64, 80, 83, 86, 89, 92, 95, 133, 137, 143, 155, 159, 163, and 175 or an amino acid sequence with at least 80%, at least 85%, at least 90%, at least 95%, or at least 99% sequence identity to any of SEQ ID NOs: 48-64, 80, 83, 86, 89, 92, 95, 133, 137, 143, 155, 159, 163, and 175.

In some embodiments, the modified TCR further comprises a TCR constant region. In some embodiments, the TCR constant region is a murine constant region or a human constant region. In some embodiments, the TCR constant region comprises TCR α chain constant region and/or a TCR β chain constant region. In a preferred embodiment, the TCR α chain constant region and/or TCR β chain constant region comprises at least one cysteine substitution relative to the wild-type sequence to form a disulfide bond between the TCR α chain and TCR β chain. In a preferred embodiment, the TCR α chain constant region comprises an LVL substitution or LIV substitution, such that the α chain constant region comprises the amino acid sequence LLVIVLRIL.

In some embodiments, the modified TCR comprises a TCR α chain having an amino acid sequence as shown in SEQ ID NO: 69 or an amino acid sequence with at least 80%, at least 85%, at least 90%, at least 95%, or at least 99% sequence identity to SEQ ID NO: 69, and a TCR β chain having an amino acid sequence selected from any of SEQ ID NOs: 3-19, 81, 84, 87, 90, 93, 96, 135, 139, 145, 157, 161, 164, and 176 or an amino acid sequence with at least 80%, at least 85%, at least 90%, at least 95%, or at least 99% sequence identity to any of SEQ ID NOs: 3-19, 81, 84, 87, 90, 93, 96, 135, 139, 145, 157, 161, 164, and 176.

In some embodiments, the modified TCR comprises a TCR α chain having an amino acid sequence selected from any of SEQ ID NOs: 20-23, 78, 99, 102, 105, 108, 111, 114, 117, 120, 123, 126, 129, 134, 138, 144, 148, 151, 156, 160, 167, 169, 172, and 174 or an amino acid sequence with at least 80%, at least 85%, at least 90%, at least 95%, or at least 99% sequence identity to any of SEQ ID NOs: 20-23, 78, 99, 102, 105, 108, 111, 114, 117, 120, 123, 126, 129, 134, 138, 144, 148, 151, 156, 160, 167, 169, 172, and 174, and a TCR β chain having an amino acid sequence as shown in SEQ ID NO: 70 or an amino acid sequence with at least 80%, at least 85%, at least 90%, at least 95%, or at least 99% sequence identity to SEQ ID NO: 70.

In some embodiments, the modified TCR comprises a TCR α chain having an amino acid sequence selected from any of SEQ ID NOs: 20-23, 78, 99, 102, 105, 108, 111, 114, 117, 120, 123, 126, 129, 134, 138, 144, 148, 151, 156, 160, 167, 169, 172, and 174 or an amino acid sequence with at least 80%, at least 85%, at least 90%, at least 95%, or at least 99% sequence identity to any of SEQ ID NOs: 20-23, 78, 99, 102, 105, 108, 111, 114, 117, 120, 123, 126, 129, 134, 138, 144, 148, 151, 156, 160, 167, 169, 172, and 174, and a TCR β chain having an amino acid sequence selected from any of SEQ ID NOs: 3-19, 81, 84, 87, 90, 93, 96, 135, 139, 145, 157, 161, 164, and 176 or an amino acid sequence with at least 80%, at least 85%, at least 90%, at least 95%, or at least 99% sequence identity to any of SEQ ID NOs: 3-19, 81, 84, 87, 90, 93, 96, 135, 139, 145, 157, 161, 164, and 176.

In some embodiments, the modified TCR binds to an epitope comprising the amino acid sequence KVLEYVIKV (SEQ ID NO:2) or a complex of this epitope with an MHC molecule.

In some embodiments, the MHC molecule is HLA-A*02 type, such as HLA-A*02:01 type or HLA-A*02:05 type.

In some embodiments, the modified TCR is soluble or membrane-bound.

In some embodiments, the modified TCR is isolated or purified.

In another aspect, the present disclosure provides a nucleic acid encoding the modified TCR of the invention.

In a further aspect, the present disclosure provides a vector comprising the nucleic acid of the invention.

In some embodiments, the vector is selected from the group consisting of lentiviral vectors, retroviral vectors, plasmids, DNA vectors, mRNA vectors, transposon-based vectors, and artificial chromosomes.

In another aspect, the present disclosure provides a cell containing the modified TCR, the nucleic acid, or the vector of the invention.

In some embodiments, the cells are selected from the group consisting of lymphocytes (e.g., T cells, NK cells), monocytes (e.g., PBMCs), and stem cells. In some embodiments, the stem cells are lymphoid progenitor cells or induced pluripotent stem cells (iPSCs). In some embodiments, the cells are T cells. In some embodiments, the T cells do not express endogenous TCRs.

In a further aspect, the present disclosure provides a method for preparing the cell of the invention comprising the step of transducing or transfecting cells with the vector of the invention.

In some embodiments, the method further comprises steps of expanding and/or activating the cells before or after transduction or transfection.

In another aspect, the present disclosure provides a conjugate comprising the modified TCR of the invention and an active agent conjugated or coupled to the TCR.

In some embodiments, the active agent is selected from the group consisting of a detectable marker, an immune stimulatory molecule, and a therapeutic agent; preferably, the detectable marker is selected from the group consisting of biotins, streptavidin, enzymes or catalytically active fragments thereof, radionuclides, nanoparticles, paramagnetic metal ions, nucleic acid probes, contrast agents, and fluorescent, phosphorescent or chemiluminescent molecules; preferably, the immune stimulatory molecule is selected from the group consisting of cytokines, chemokines, platelet factors and complement initiators; preferably, the therapeutic agent is selected from the group consisting of immunomodulators, radioactive compounds, enzymes, chemotherapeutic agents and toxins.

In another aspect, the present disclosure provides a bispecific or multispecific antigen-binding molecule comprising the modified TCR of the invention or an antigen-binding fragment thereof, and one or more additional antigen-binding regions that bind to one or more additional antigens; optionally, wherein the one or more additional antigen-binding regions are independently selected from the group consisting of antigen-binding regions derived from antibodies and antigen-binding regions derived from TCRs.

In some embodiments, the one or more additional antigens are independently selected from the group consisting of tumor-associated antigens (TAAs), tumor-specific antigens (TSAs), viral antigens, autoantigens, and immune cell surface molecules. In some embodiments, the one or more additional antigens are CD3, and preferably, the one or more additional antigen-binding regions are derived from antigen-binding regions of anti-CD3 antibodies.

In another aspect, the present disclosure provides a composition comprising the modified TCR, nucleic acid, vector, cell, conjugate, or bispecific or multispecific antigen-binding molecule of the invention; preferably, the composition further comprises a pharmaceutically acceptable carrier or excipient.

In some embodiments, the composition further comprises a second therapeutic agent, preferably, the second therapeutic agent is selected from the group consisting of antibodies, chemotherapeutic agents, and small molecule drugs.

In another aspect, the present disclosure provides a method for treating MAGE-A1-positive cancer in a subject, comprising administering an effective amount of the modified TCR of the invention to the subject.

In another aspect, the present disclosure provides a method for treating MAGE-A1-positive cancer in a subject, comprising administering an effective amount of the cells of the invention to the subject.

In some embodiments of the treatment method of the present disclosure, the cells are autologous or allogeneic to the subject.

In some embodiments, the method comprises the following steps:
(i) isolating a sample containing cells from the subject;
(ii) transducing or transfecting the cells with the vector of the invention; and
(iii) administering the cells obtained in step (ii) to the subject.

In some embodiments, the method further comprises a step of knocking out endogenous TCRs in the cells after step (i) and before step (ii).

In some embodiments, the MAGE-A1-positive cancer is selected from the group consisting of melanoma, lung cancer, colorectal cancer, cervical cancer, breast cancer, liver cancer (such as hepatocellular carcinoma), gastric cancer, esophageal cancer, bladder cancer (such as urothelial carcinoma of the bladder), ovarian cancer (such as serous cystadenocarcinoma of the ovary), head and neck cancer (such as squamous cell carcinoma of the head and neck), uterine cancer, endometrial cancer, cholangiocarcinoma, prostate cancer, adrenocortical carcinoma, mesothelioma, sarcoma, pheochromocytoma, paraganglioma, and thymic carcinoma.

In some embodiments, the method further comprises administering a second therapeutic agent, preferably, the second therapeutic agent is selected from the group consisting of antibodies, chemotherapeutic agents, and small molecule drugs.

In some embodiments of the treatment method of the present disclosure, the subject has an HLA-A*02 allele, such as HLA-A*02:01 allele or HLA-A*02:05 allele.

In another aspect, the present disclosure provides an usage of the modified TCR, nucleic acid, vector, cell, conjugate, bispecific or multispecific antigen-binding molecule, or composition of the invention in the preparation of drugs for treating MAGE-A1-positive cancer in a subject.

In a further aspect, the present disclosure provides the modified TCR, nucleic acid, vector, cell, conjugate, bispecific or multispecific antigen-binding molecule, or composition of the invention for treating MAGE-A1-positive cancer in a subject.

In some embodiments of the use, the MAGE-A1-positive cancer is selected from the group consisting of melanoma, lung cancer, colorectal cancer, cervical cancer, breast cancer, liver cancer (such as hepatocellular carcinoma), gastric cancer, esophageal cancer, bladder cancer (such as urothelial carcinoma of the bladder), ovarian cancer (such as serous cystadenocarcinoma of the ovary), head and neck cancer (such as squamous cell carcinoma of the head and neck), uterine cancer, endometrial cancer, cholangiocarcinoma, prostate cancer, adrenocortical carcinoma, mesothelioma, sarcoma, pheochromocytoma, paraganglioma, and thymic carcinoma. In some embodiments, the subject has the HLA-A*02:01 allele, such as HLA-A*02:01 allele or HLA-A*02:05 allele.

### DESCRIPTION OF THE DRAWINGS

Figure 1 shows the screening of the affinity-optimized TCR of the present invention by flow cytometry.
Figure 2 shows the activation of target cells loaded with the target antigen peptide (KVLEYVIKV) by the affinity-optimized TCR of the present invention, as determined by flow cytometry.
Figure 3 shows the activation of target cells loaded with the gp100 target antigen by the gp100 mutation TCR known in the prior art, as determined by flow cytometry.
Figure 4 shows the staining of cells expressing the gp100 mutation TCR known in the prior art with different concentrations of gp100 Dextramer, as determined by flow cytometry.
Figure 5 shows the gradient staining results of gp100 Dextramer on cells expressing the gp100 mutation TCR known in the prior art.
Figure 6 shows the linear relationship between Dextramer staining intensity and the affinity (KD-SPR) of the gp100 mutation TCR known in the prior art.
Figure 7 shows the staining of cells expressing the mutated TCR of the present invention with different concentrations of MAGE-A1 Dextramer, as determined by flow cytometry.
Figure 8 shows the gradient staining results of MAGE-A1 Dextramer on cells expressing the mutated TCR of the present invention.
Figure 9 shows the phenotype of TCR-T cells with the affinity-optimized TCR of the present invention, as determined by flow cytometry.
Figure 10 shows the specific killing of antigen-positive tumor cells by TCR-T cells with the affinity-optimized TCR of the present invention.
Figure 11 shows the specific IFN-γ secretion by TCR-T cells with the affinity-optimized TCR of the present invention against antigen-positive tumor cells.
Figure 12 shows the non-specific responses of TCR-T cells with the affinity-optimized TCR of the present invention against different HLA types, as determined by IFN-γ secretion assays.

### DETAILED DESCRIPTION OF THE INVENTION

Unless otherwise specified, all technical and scientific terms used in this specification have the same meaning as commonly understood by those skilled in the art. For example, terms used herein are as defined in "Immunobiology" by Janeway CA Jr, Travers P, Walport M, 5th Edition, New York: Garland Science (2001) and "A multilingual glossary of biotechnological terms: (IUPAC Recommendations)" edited by Leuenberger, H.G.W, Nagel, B., and Kölbl, H. (1995), Helvetica Chimica Acta, CH-4010 Basel, Switzerland.

It should be noted that, as used herein and in the appended claims, the singular forms "a", "an" and "the/the" include plural forms, unless the context clearly defined otherwise. Thus, the terms "a", "an", "one or more" and "at least one" can be used interchangeably. Similarly, the terms "comprise", "include" and "have" can be used interchangeably.

When the term "comprise" is used herein and in the appended claims, it does not exclude other elements. For the purposes of the present invention, the term "consist of" is considered to be a preferred embodiment of the term "comprise". If a group is defined below as including or comprising at least a certain number of embodiments, it is also to be understood as disclosing a group that preferably consists of only those embodiments.

In one aspect, the present disclosure provides a modified T cell receptor (TCR), wherein the modified TCR comprises an α variable region comprising CDR1α, CDR2α, and CDR3α, and a β chain variable region comprising CDR1β, CDR2β, and CDR3β, the modified TCR comprises the following amino acid substitutions relative to its parental TCR:
(i) amino acid substitutions in the β chain variable region, comprising:
   (a) amino acid substitutions at one or more of the following positions in CDR1β: Gln29, Ala36, Thr37, and Thr38;
   (b) amino acid substitutions at one or more of the following positions in CDR2β: Ser56, Asn57, Glu58, Gly59, Ser63, Lys64, and Ala65; and/or
   (c) amino acid substitutions at one or more of the following positions in CDR3β: Ser105, Glu108, Pro109, Gly110, Gln111, Gly112, Pro113, Tyr114, and Glu115;
      and/or
(ii) amino acid substitutions in the α chain variable region, comprising:
   (d) amino acid substitutions at one or more of the following positions in CDR1α: Asp27, Ser28, Ser29, Ser36, Thr37, and Tyr38;
   (e) amino acid substitutions at the following positions in CDR2α: Ile56, Phe57, Ser58, Asn59, Met63, and Asp64; and/or
   (f) amino acid substitution at position Thr26 in FR1α;
      wherein the parental TCR comprises amino acid sequences as shown in SEQ ID NO: 24 for CDR1β, SEQ ID NO: 25 for CDR2β, SEQ ID NO: 26 for CDR3β, SEQ ID NO: 27 for CDR1α, SEQ ID NO: 28 for CDR2α, and SEQ ID NO: 29 for CDR3α;
      wherein the amino acid positions are numbered according to the International ImMunoGeneTics Information System (IMGT) nomenclature.

In some embodiments, the modified TCR comprises amino acid substitutions as follows relative to its parental TCR:
(i) amino acid substitutions in the β chain variable region, comprising:
   (a) amino acid substitutions at one or more positions in CDR2β: Asn57, Glu58, Gly59, Ser63, Lys64, and Ala65, and/or
   (b) amino acid substitutions at one or more positions in CDR3β: Ser105, Glu108, Gln111, and Tyr114; and/or
(ii) amino acid substitutions in the α chain variable region, comprising:
   (c) amino acid substitutions at one or more positions in CDR1α: Ser29 and Ser36, and/or
   (d) amino acid substitution at one position in CDR2α: Asn59;

In a natural form, the TCR exists as a complex of several proteins on the surface of T cells. The T cell receptor consists of two (separate) protein chains that are produced by separate T cell receptor alpha and beta (TCRα and TCRβ) genes and are referred to as alpha chain and beta chain. Each chain of the TCR has an N-terminal immunoglobulin-like (Ig)-variable (V) region/domain, an Ig-constant (C) region/domain, a transmembrane/cytomembrane spanning region that anchors the chain into the plasma membrane, and a C-terminal short cytoplasmic tail.

Typically, the TCR is a heterodimer of an α chain and a β chain, which can bind MHC molecules presenting peptide antigens.

Antigen specificity is conferred by the variable regions of α chain and β chain of TCR. Both variable regions of TCR α and β chains (α chain variable region (Vα) and β chain variable region (Vβ)) contain three highly variable or complementary determining regions (CDR1α/β, CDR2α/β, and CDR3α/β) surrounded by four framework (FR) regions (FR1α/β, FR2α/β, FR3α/β, and FR4α/β). CDR3 is the major determinant for antigen recognition and specificity (i.e., the ability to recognize and interact with the specific antigen), whereas CDR1 and CDR2 interact primarily with MHC molecules that present antigenic peptides.

The TCR recognizes an antigenic peptide that binds to a major histocompatibility complex (MHC) molecule at the surface of the antigen-presenting cell ("presenting/exhibiting on the MHC molecule"). The antigenic peptide presented on the MHC molecule is also referred to herein as a "complex of epitope with MHC molecule", "epitope-MHC complex" or "target antigenic peptide-MHC complex". There are two different classes of MHC molecules: MHC I and MHC II, which present peptides from different cellular compartments. MHC class I molecules are expressed on the surface of all nucleated cells in the human and present peptides or protein fragments from intracellular compartments to cytotoxic T cells. In human, MHC is also known as human leukocyte antigen (HLA). There are three main types of MHC class I molecules: HLA-A, HLA-B, and HLA-C. Once the TCR binds to its specific epitope-MHC complex, the T cells are activated and exert a biological effector function.

The positions of amino acid sequences of the TCR in the present invention are numbered according to the International ImMunoGeneTics information system (IMGT) nomenclature. For example, the positions of amino acid sequences in the TCR α chain variable region (Vα) and β chain variable region (Vβ) are numbered as the positions designated in IMGT. For instance, an amino acid in Vα designated as position 29 according to IMGT is described as the 29th amino acid herein; an amino acid in Vβ listed as position 57 according to IMGT is described as the 57th amino acid herein; and so forth. When the sequence position numbering of other amino acids is specifically described in the present invention, they are numbered as specifically described.

The term "parental TCR" as used herein refers to the previously developed human TCR ht27 targeting MAGE-A1 restricted to HLA-A0201, which comprises amino acid sequences as shown in SEQ ID NO: 24 (DFQATT) for CDR1β, SEQ ID NO: 25 (SNEGSKA) for CDR2β, SEQ ID NO: 26 (SAREPGQGPYEQY) for CDR3β, SEQ ID NO: 27 (DSSSTY) for CDR1α, SEQ ID NO: 28 (IFSNMDM) for CDR2α, and SEQ ID NO: 29 (AGSGGGTDKLI) for CDR3α, and whose amino acid sequences of Vα and Vβ variable regions are as shown in SEQ ID NO: 46 and SEQ ID NO: 47, respectively.
α chain variable region 
RGEDVEQSLFLSVREGDSSVINCTYTDSSSTYLYWYKQEPGAGLQLLTYIFSNMDMKQD
QRLTVLLNKKDKHLSLRIADTQTGDSAIYFCAGSGGGTDKLIFGTGTRLQVFPN (SEQ ID
NO:46)
β chain variable region:

GAVVSQHPSWVICKSGTSVKIECRSLDFQATTMFWYRQFPKQSLMLMATSNEGSKATY
EQGVEKDKFLINHASLTLSTLTVTSAHPEDSSFYICSAREPGQGPYEQYFGPGTRLTVTE (SEQ
ID NO:47).

The inventors have discovered that the modified TCR of the present invention, comprising amino acid substitutions at one or more of the following positions in the variable region relative to the parental TCR, exhibits improved binding affinity for MAGE-A1: amino acid substitutions at the following positions in CDR2β: Asn57, Glu58, Gly59, Ser63, Lys64 and Ala65; amino acid substitutions at the following positions in CDR3β: Ser105, Glu108, Gln111 and Tyr114; amino acid substitutions at the following positions in CDR1α: Ser29 and Ser36; amino acid substitution at the following position in CDR2α: Asn59. For example, amino acid substitution at Ser105 can increase the binding affinity of TCR approximately 10-400 times; substitution at Glu108 can increase the binding affinity approximately 1-2 times; substitution at Gln111 can increase the binding affinity approximately 12-14 times; substitution at Tyr114 position can increase the binding affinity approximately 11-944 times. Specific amino acid substitutions at these positions comprise but are not limited to: N57D, N57E, N57H, N57K, N57Q, N57R, N57S, N57T, E58A, E58F, E58I, E58L, E58M, E58P, E58V, E58Y, E58W, G59D, G59E, G59H, G59K, G59Q, G59R, G59S, G59T, S63D, S63E, S63H, S63K, S63Q, S63R, S63T, K64M, K64C, K64W, K64T, K64P, K64A, A65F, A65I, A65L, A65M, A65P, A65V, A65W, A65Y, S105L, S105N, S105H, S105Q, S105K, S105R, E108D, E108F, E108T, E108I, E108K, E108P, Q111N, Q111K, Q111S, Q111G, Q111A, Q111V, Q111E, Q111L, Q111H, Y114I, Y114F, Y114K, Y114D, Y114L, Y114W, Y114N, Y114P, Y114S, S29A, S29F, S29I, S29L, S29M, S29P, S29V, S29W, S29N, S36M, S36L, S36Q, S36I, S36F, S36P, S36V, N59F, N59D, N59E, N59H, N59K, N59Q, N59R, N59S and N59T.

Therefore, in some embodiments, the modified TCR relative to the parental TCR comprises one or more of the amino acid substitutions (1)-(33) as follows:
(1) amino acid substitution at Gln29 in CDR1β, selected from the group consisting of Q29E and Q29P;
(2) amino acid substitution at Ala36 in CDR1β, A36I;
(3) amino acid substitution at Thr37 in CDR1β, T37E;
(4) amino acid substitution at Thr38 in CDR1β, T38R;
(5) amino acid substitution at Ser56 in CDR2β, S56T;
(6) amino acid substitution at Asn57 in CDR2β, selected from the group consisting of N57D, N57E, N57H, N57K, N57Q, N57R, N57S, and N57T;
(7) amino acid substitution at Glu58 in CDR2β, selected from the group consisting of E58A, E58F, E58I, E58L, E58M, E58P, E58V, E58Y, and E58W;
(8) amino acid substitution at Gly59 in CDR2β, selected from the group consisting of G59P, G59L, G59D, G59E, G59H, G59K, G59Q, G59R, G59S, and G59T;
(9) amino acid substitution at Ser63 in CDR2β, selected from the group consisting of S63D, S63E, S63H, S63K, S63Q, S63R, and S63T;
(10) amino acid substitution at Lys64 in CDR2β, selected from the group consisting of K64Q, K64M, K64C, K64W, K64T, K64P, and K64A;
(11) amino acid substitution at Ala65 in CDR2β, selected from the group consisting of A65F, A65I, A65L, A65M, A65P, A65V, A65W, and A65Y;
(12) amino acid substitution at Ser105 position in CDR3β, selected from the group consisting of S105L, S105N, S105H, S105Q, S105K, and S105R;
(13) amino acid substitution at Glu108 position in CDR3β, selected from the group consisting of E108D, E108F, E108T, E108I, E108K, and E108P;
(14) amino acid substitution at Gly110 position in CDR3β, G110W;
(15) amino acid substitution at Gln111 position in CDR3β, selected from the group consisting of Q111D, Q111N, Q111K, Q111S, Q111G, Q111A, Q111V, Q111E, Q111L, and Q111H;
(16) amino acid substitution of EPGQGP at Glu108 to Pro113 in CDR3β replaced with VNDGT;
(17) amino acid substitution at Gly112 in CDR3β, selected from the group consisting of G112P, G112A, and G112E;
(18) amino acid substitution at Pro113 in CDR3β, selected from the group consisting of P113W and P113H;
(19) amino acid substitution at Tyr114 in CDR3β, selected from the group consisting of Y114I, Y114F, Y114K, Y114D, Y114L, Y114W, Y114N, Y114P, and Y114S;
(20) amino acid substitution at Glu115 in CDR3β, selected from the group consisting of E115D and E115I;
(21) amino acid substitution at Asp27 in CDR1α, selected from the group consisting of D27C and D27Y;
(22) amino acid substitution at Ser28 in CDR1α, selected from the group consisting of S28M, S28A, S28G, S28Y, and S28P;
(23) amino acid substitution at Ser29 in CDR1α, selected from the group consisting of S29E, S29T, S29Y, S29A, S29F, S29I, S29L, S29M, S29P, S29V, S29W, and S29N;
(24) amino acid substitution at Ser36 in CDR1α, selected from the group consisting of S36D, S36M, S36L, S36Q, S36I, S36F, S36P, and S36V;
(25) amino acid substitution at Thr37 in CDR1α, T37I;
(26) amino acid substitution at Tyr38 in CDR1α, Y38L;
(27) amino acid substitution at Ile56 in CDR2α, selected from the group consisting of I56T and I56G;
(28) amino acid substitution at Phe57 in CDR2α, selected from the group consisting of F57W and F57T;
(29) amino acid substitution at Ser58 in CDR2α, selected from the group consisting of S58G, S58I, S58N, S58A, S58P, and S58W;
(30) amino acid substitution at Asn59 in CDR2α, selected from the group consisting of N59L, N59F, N59D, N59E, N59H, N59K, N59Q, N59R, N59S, and N59T;
(31) amino acid substitution at Met63 in CDR2α, selected from the group consisting of M63D, M63Q, M63T, and M63E;
(32) amino acid substitution at Asp64 in CDR2α, selected from the group consisting of D64A, D64Q, D64T, D64Y, and D64S;
(33) amino acid substitution at Thr26 in FR1α, selected from the group consisting of T26S, T26I, T26P, T26Q, T26A, and T26V.

In some embodiments, the modified TCR relative to the parental TCR comprises one or more of the amino acid substitutions (1)-(13) as follows:
(1) amino acid substitution at Asn57, selected from the group consisting of N57D, N57E, N57H, N57K, N57Q, N57R, N57S, and N57T;
(2) amino acid substitution at Glu58, selected from the group consisting of E58A, E58F, E58I, E58L, E58M, E58P, E58V, E58Y, and E58W;
(3) amino acid substitution at Gly59, selected from the group consisting of G59D, G59E, G59H, G59K, G59Q, G59R, G59S, and G59T;
(4) amino acid substitution at Ser63, selected from the group consisting of S63D, S63E, S63H, S63K, S63Q, S63R, and S63T;
(5) amino acid substitution at Lys64, selected from the group consisting of K64M, K64C, K64W, K64T, K64P, and K64A;
(6) amino acid substitution at Ala65, selected from the group consisting of A65F, A65I, A65L, A65M, A65P, A65V, A65W, and A65Y;
(7) amino acid substitution at Ser105, selected from the group consisting of S105L, S105N, S105H, S105Q, S105K, and S105R;
(8) amino acid substitution at Glu108, selected from the group consisting of E108D, E108F, E108T, E108I, E108K, and E108P;
(9) amino acid substitution at Gln111, selected from the group consisting of Q111N, Q111K, Q111S, Q111G, Q111A, Q111V, Q111E, Q111L, and Q111H;
(10) amino acid substitution at Tyr114, selected from the group consisting of Y114I, Y114F, Y114K, Y114D, Y114L, Y114W, Y114N, Y114P, and Y114S;
(11) amino acid substitution at Ser29, selected from the group consisting of S29A, S29F, S29I, S29L, S29M, S29P, S29V, S29W, and S29N;
(12) amino acid substitution at Ser36, selected from the group consisting of S36M, S36L, S36Q, S36I, S36F, S36P, and S36V;
(13) amino acid substitution at Asn59, selected from the group consisting of N59F, N59D, N59E, N59H, N59K, N59Q, N59R, N59S, and N59T.

In some embodiments, the modified TCR relative to the parental TCR does not comprise a single S105N amino acid substitution. In other embodiments, the modified TCR relative to the parental TCR comprises a combination of amino acid substitutions comprising S105N and the other amino acid substitutions listed above.

In some embodiments, the modified TCR relative to the parental TCR in CDR2β comprises one or more of the following amino acid substitutions: N57D, E58Y, G59Q, S63T, K64C, and A65V. In preferred embodiments, the modified TCR relative to the parental TCR in CDR2β comprises the amino acid substitutions N57D, E58Y, G59Q, S63T, K64C, and A65V.

In some embodiments, the modified TCR comprises one or more of the following amino acid substitutions (1)-(3) in the CDR3β relative to the parental TCR: (1) Q111G or Q111S; (2) Y114F, Y114L, or Y114W; (3) S105L, S105Q, S105N, or S105H. In some embodiments, the modified TCR comprises amino acid substitutions in the CDR3β relative to the parental TCR: Q111GY114F, Q111GY114L, Q111GY114W, Q111SY114F, Q111SY114L, or Q114SY114W. In preferred embodiments, the modified TCR comprises amino acid substitutions in the CDR3β relative to the parental TCR: Y114F, Y114W, Q111GY114F, Q111SY114F, S105L, or S105N.

In some embodiments, the modified TCR comprises one or more of the following amino acid substitutions in the CDR2β relative to the parental TCR: N57D, E58Y, G59Q, S63T, K64C, and A65V; and comprises one or more of the following amino acid substitutions (1)-(3) in the CDR3β: (1) Q111G or Q111S; (2) Y114F, Y114L, or Y114W; (3) S105L, S105Q, S105N, or S105H.

In some embodiments, the modified TCR comprises amino acid substitutions in the CDR2β relative to the parental TCR: N57D, E58Y, G59Q, S63T, K64C, and A65V; and comprises one or more of the following amino acid substitutions (1)-(3) in the CDR3β: (1) Q111G or Q111S; (2) Y114F, Y114L, or Y114W; (3) S105L, S105Q, S105N, or S105H.

In some embodiments, the modified TCR comprises amino acid substitutions in the CDR2β relative to the parental TCR: N57D, E58Y, G59Q, S63T, K64C, and A65V; and comprises amino acid substitutions in the CDR3β: Q111GY114F, Q111GY114L, Q111GY114W, Q111SY114F, Q111SY114L, or Q114SY114W.

In some embodiments, the modified TCR comprises amino acid substitutions in the CDR2β relative to the parental TCR: N57D, E58Y, G59Q, S63T, K64C, and A65V; and comprises amino acid substitutions in the CDR3β: Y114F, Y114W, Q111GY114F, Q111SY114F, S105L, or S105N.

In some embodiments, the modified TCR comprises amino acid substitutions in the CDR2β relative to the parental TCR: N57D, E58Y, G59Q, S63T, K64C, and A65V; and comprises amino acid substitutions in the CDR3β: Y114F, Y114L, Y114W, Q111GY114F, or Q111SY114F.

In some embodiments, the modified TCR comprises the following amino acid substitutions (1) and/or (2) in the CDR1α relative to the parental TCR: (1) S29L; (2) S36M, S36L, or S36Q.

In some embodiments, the modified TCR comprises an amino acid substitution N59F in the CDR2α relative to the parental TCR.

In some embodiments, the modified TCR comprises the following amino acid substitutions (1) and/or (2) in the CDR1α relative to the parental TCR: (1) S29L; (2) S36M, S36L, or S36Q; and comprises an amino acid substitution N59F in the CDR2α.

The respective three CDR regions of both the TCR α and β chains bind to different sites of the MHC-peptide complex and interact with the MHC-peptide complex in a relatively independent manner. For the disclosed mutations in the CDR regions of this invention, whether as single or combined mutations, they effectively enhance the affinity of the TCR relative to the wild-type TCR. Some combinations of mutations can even produce synergistic effects, achieving higher affinity.

Therefore, in some embodiments, relative to the parental TCR, the modified TCR comprises one or more of the following amino acid substitutions (1) and/or (2) in the CDR1α: (1) S29L; (2) S36M, S36L, or S36Q; an amino acid substitution N59F in the CDR2α; one or more of the following amino acid substitutions in the CDR2β: N57D, E58Y, G59Q, S63T, K64C, and A65V; and one or more of the following amino acid substitutions (1)-(3) in the CDR3β: (1) Q111G or Q111S; (2) Y114F, Y114L, or Y114W; (3) S105L, S105Q, S105N, or S105H.

In some embodiments, relative to the parental TCR, the modified TCR comprises one or more of the following amino acid substitutions (1) and/or (2) in the CDR1α: (1) S29L; (2) S36M, S36L, or S36Q; an amino acid substitution N59F in the CDR2α; amino acid substitutions N57D, E58Y, G59Q, S63T, K64C, and A65V in the CDR2β; and one or more of the following amino acid substitutions (1)-(3) in the CDR3β: (1) Q111Gor Q111S; (2) Y114F, Y114L, or Y114W; (3) S105L, S105Q, S105N, or S105H.

In some embodiments, relative to the parental TCR, the modified TCR comprises one or more of the following amino acid substitutions (1) and/or (2) in the CDR1α: (1) S29L; (2) S36M, S36L, or S36Q; an amino acid substitution N59F in the CDR2α; amino acid substitutions N57D, E58Y, G59Q, S63T, K64C, and A65V in the CDR2β; and amino acid substitutions Q111GY114F, Q111GY114L, Q111GY114W, Q111SY114F, Q111SY114L, or Q114SY114W in the CDR3β.

In some embodiments, relative to the parental TCR, the modified TCR comprises one or more of the following amino acid substitutions (1) and/or (2) in the CDR1α: (1) S29L; (2) S36M, S36L, or S36Q; an amino acid substitution N59F in the CDR2α; amino acid substitutions N57D, E58Y, G59Q, S63T, K64C, and A65V in the CDR2β; and amino acid substitutions Y114F, Y114W, Q111GY114F, Q111SY114F, S105L, or S105N in the CDR3β.

In some embodiments, relative to the parental TCR, the modified TCR comprises one or more of the following amino acid substitutions (1) and/or (2) in the CDR1α: (1) S29L; (2) S36M, S36L, or S36Q; an amino acid substitution N59F in the CDR2α; amino acid substitutions N57D, E58Y, G59Q, S63T, K64C, and A65V in the CDR2β; and amino acid substitutions Y114F, Y114L, Y114W, Q111GY114F, or Q111SY114F in the CDR3β.

In some embodiments, the modified TCR comprises:
(i) an α chain variable region, comprising CDR1α, CDR2α, and CDR3α with amino acid sequences as shown in SEQ ID NOs: 27-29, respectively; and
(ii) a β chain variable region comprising:
   CDR1β, CDR2β, and CDR3β with amino acid sequences as shown in SEQ ID NOs: 24, 30, and 26, respectively;
   CDR1β, CDR2β, and CDR3β with amino acid sequences as shown in SEQ ID NOs: 24, 25, and 31, respectively;
   CDR1β, CDR2β, and CDR3β with amino acid sequences as shown in SEQ ID NOs: 24, 25, and 32, respectively;
   CDR1β, CDR2β, and CDR3β with amino acid sequences as shown in SEQ ID NOs: 24, 25, and 33, respectively;
   CDR1β, CDR2β, and CDR3β with amino acid sequences as shown in SEQ ID NOs: 24, 25, and 34, respectively;
   CDR1β, CDR2β, and CDR3β with amino acid sequences as shown in SEQ ID NOs: 24, 25, and 35, respectively;
   CDR1β, CDR2β, and CDR3β with amino acid sequences as shown in SEQ ID NOs: 24, 30, and 33, respectively;
   CDR1β, CDR2β, and CDR3β with amino acid sequences as shown in SEQ ID NOs: 24, 30, and 34, respectively;
   CDR1β, CDR2β, and CDR3β with amino acid sequences as shown in SEQ ID NOs: 24, 30, and 35, respectively;
   CDR1β, CDR2β, and CDR3β with amino acid sequences as shown in SEQ ID NOs: 24, 25, and 36, respectively;
   CDR1β, CDR2β, and CDR3β with amino acid sequences as shown in SEQ ID NOs: 24, 25, and 37, respectively;
   CDR1β, CDR2β, and CDR3β with amino acid sequences as shown in SEQ ID NOs: 24, 30, and 36, respectively;
   CDR1β, CDR2β, and CDR3β with amino acid sequences as shown in SEQ ID NOs: 24, 30, and 37, respectively;
   CDR1β, CDR2β, and CDR3β with amino acid sequences as shown in SEQ ID NOs: 24, 25, and 38, respectively;
   CDR1β, CDR2β, and CDR3β with amino acid sequences as shown in SEQ ID NOs: 24, 25, and 39, respectively;
   CDR1β, CDR2β, and CDR3β with amino acid sequences as shown in SEQ ID NOs: 24, 25, and 40, respectively;
   CDR1β, CDR2β, and CDR3β with amino acid sequences as shown in SEQ ID NOs: 24, 25, and 41, respectively;
   CDR1β, CDR2β, and CDR3β with amino acid sequences as shown in SEQ ID NOs: 24, 25, and 79, respectively;
   CDR1β, CDR2β, and CDR3β with amino acid sequences as shown in SEQ ID NOs: 82, 25, and 26, respectively;
   CDR1β, CDR2β, and CDR3β with amino acid sequences as shown in SEQ ID NOs: 24, 85, and 26, respectively;
   CDR1β, CDR2β, and CDR3β with amino acid sequences as shown in SEQ ID NOs: 24, 88, and 26, respectively;
   CDR1β, CDR2β, and CDR3β with amino acid sequences as shown in SEQ ID NOs: 24, 91, and 26, respectively;
   CDR1β, CDR2β, and CDR3β with amino acid sequences as shown in SEQ ID NOs: 24, 25, and 94, respectively;
   CDR1β, CDR2β, and CDR3β with amino acid sequences as shown in SEQ ID NOs: 130, 25, and 131, respectively;
   CDR1β, CDR2β, and CDR3β with amino acid sequences as shown in SEQ ID NOs: 24, 91, and 35, respectively;
   CDR1β, CDR2β, and CDR3β with amino acid sequences as shown in SEQ ID NOs: 130, 91, and 35, respectively;
   CDR1β, CDR2β, and CDR3β with amino acid sequences as shown in SEQ ID NOs: 130, 153, and 35, respectively;
   CDR1β, CDR2β, and CDR3β with amino acid sequences as shown in SEQ ID NOs: 130, 91, and 26, respectively;
   CDR1β, CDR2β, and CDR3β with amino acid sequences as shown in SEQ ID NOs: 130, 91, and 162, respectively; or
   CDR1β, CDR2β, and CDR3β with amino acid sequences as shown in SEQ ID NOs: 82, 91, and 33, respectively.

In some embodiments, the modified TCR comprises:
(i) an α chain variable region comprising CDR1α, CDR2α, and CDR3α with amino acid sequences as shown in SEQ ID NOs: 27-29, respectively; and
(ii) an β chain variable region comprising:
   CDR1β, CDR2β, and CDR3β with amino acid sequences as shown in SEQ ID NOs: 24, 30, and 26, respectively;
   CDR1β, CDR2β, and CDR3β with amino acid sequences as shown in SEQ ID NOs: 24, 25, and 31, respectively;
   CDR1β, CDR2β, and CDR3β with amino acid sequences as shown in SEQ ID NOs: 24, 25, and 32, respectively;
   CDR1β, CDR2β, and CDR3β with amino acid sequences as shown in SEQ ID NOs: 24, 25, and 33, respectively;
   CDR1β, CDR2β, and CDR3β with amino acid sequences as shown in SEQ ID NOs: 24, 25, and 34, respectively;
   CDR1β, CDR2β, and CDR3β with amino acid sequences as shown in SEQ ID NOs: 24, 25, and 35, respectively;
   CDR1β, CDR2β, and CDR3β with amino acid sequences as shown in SEQ ID NOs: 24, 30, and 33, respectively;
   CDR1β, CDR2β, and CDR3β with amino acid sequences as shown in SEQ ID NOs: 24, 30, and 34, respectively;
   CDR1β, CDR2β, and CDR3β with amino acid sequences as shown in SEQ ID NOs: 24, 30, and 35, respectively;
   CDR1β, CDR2β, and CDR3β with amino acid sequences as shown in SEQ ID NOs: 24, 25, and 36, respectively;
   CDR1β, CDR2β, and CDR3β with amino acid sequences as shown in SEQ ID NOs: 24, 25, and 37, respectively;
   CDR1β, CDR2β, and CDR3β with amino acid sequences as shown in SEQ ID NOs: 24, 30, and 36, respectively;
   CDR1β, CDR2β, and CDR3β with amino acid sequences as shown in SEQ ID NOs: 24, 30, and 37, respectively;
   CDR1β, CDR2β, and CDR3β with amino acid sequences as shown in SEQ ID NOs: 24, 25, and 38, respectively;
   CDR1β, CDR2β, and CDR3β with amino acid sequences as shown in SEQ ID NOs: 24, 25, and 39, respectively;
   CDR1β, CDR2β, and CDR3β with amino acid sequences as shown in SEQ ID NOs: 24, 25, and 40, respectively, or
   CDR1β, CDR2β, and CDR3β with amino acid sequences as shown in SEQ ID NOs: 24, 25, and 41, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 30, and 26, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 25, and 31, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 25, and 32, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 25, and 33, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 25, and 34, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 25, and 35, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 30, and 33, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 30, and 34, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 30, and 35, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 25, and 36, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 25, and 37, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 30, and 36, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 30, and 37, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 25, and 38, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 25, and 39, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 25, and 40, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 25, and 41, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 25, and 79, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:82, 25, and 26, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 85, and 26, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 88, and 26, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 91, and 26, respectively.

In a preferred embodiment, the modified TCR comprises: (i) an α chain variable region, which comprises CDR1α, CDR2α, and CDR3α having the amino acid sequences as shown in SEQ ID NOs:27-29, respectively; and (ii) a β chain variable region, which comprises CDR1β, CDR2β, and CDR3β having the amino acid sequences as shown in SEQ ID NOs:24, 25, and 94, respectively.

In some embodiments, the modified TCR comprises:
(i) a β chain variable region, which comprises CDR1β, CDR2β, and CDR3β having the amino acid sequences as shown in SEQ ID NOs:24-26, respectively; and
(ii) an α chain variable region, which comprises
   CDR1α, CDR2α, and CDR3α having the amino acid sequences as shown in SEQ ID NOs:42, 28, and 29, respectively,
   CDR1α, CDR2α, and CDR3α having the amino acid sequences as shown in SEQ ID NOs:43, 28, and 29, respectively,
   CDR1α, CDR2α, and CDR3α having the amino acid sequences as shown in SEQ ID NOs:44, 28, and 29, respectively,
   CDR1α, CDR2α, and CDR3α having the amino acid sequences as shown in SEQ ID NOs:45, 28, and 29, respectively,
   CDR1α, CDR2α, and CDR3α having the amino acid sequences as shown in SEQ ID NOs:27, 76, and 29, respectively,
   CDR1α, CDR2α, and CDR3α having the amino acid sequences as shown in SEQ ID NOs:97, 28, and 29, respectively,
   CDR1α, CDR2α, and CDR3α having the amino acid sequences as shown in SEQ ID NOs:100, 28, and 29, respectively,
   CDR1α, CDR2α, and CDR3α having the amino acid sequences as shown in SEQ ID NOs:103, 28, and 29, respectively,
   CDR1α, CDR2α, and CDR3α having the amino acid sequences as shown in SEQ ID NOs:27, 106, and 29, respectively,
   CDR1α, CDR2α, and CDR3α having the amino acid sequences as shown in SEQ ID NOs:27, 109, and 29, respectively,
   CDR1α, CDR2α, and CDR3α having the amino acid sequences as shown in SEQ ID NOs:27, 112, and 29, respectively,
   CDR1α, CDR2α, and CDR3α having the amino acid sequences as shown in SEQ ID NOs:27, 115, and 29, respectively,
   CDR1α, CDR2α, and CDR3α having the amino acid sequences as shown in SEQ ID NOs:27, 118, and 29, respectively,
   CDR1α, CDR2α, and CDR3α having the amino acid sequences as shown in SEQ ID NOs:27, 121, and 29, respectively,
   CDR1α, CDR2α, and CDR3α having the amino acid sequences as shown in SEQ ID NOs:27, 124, and 29, respectively,
   CDR1α, CDR2α, and CDR3α having the amino acid sequences as shown in SEQ ID NOs:27, 127, and 29, respectively,
   CDR1α, CDR2α, and CDR3α having the amino acid sequences as shown in SEQ ID NOs:103, 76, and 29, respectively,
   CDR1α, CDR2α, and CDR3α having the amino acid sequences as shown in SEQ ID NOs:44, 76, and 29, respectively,
   CDR1α, CDR2α, and CDR3α having the amino acid sequences as shown in SEQ ID NOs: 140, 141, and 29, respectively,
   CDR1α, CDR2α, and CDR3α having the amino acid sequences as shown in SEQ ID NOs: 146, 118, and 29, respectively,
   CDR1α, CDR2α, and CDR3α having the amino acid sequences as shown in SEQ ID NOs:45, 149, and 29, respectively,
   CDR1α, CDR2α, and CDR3α having the amino acid sequences as shown in SEQ ID NOs: 152, 149, and 29, respectively,
   CDR1α, CDR2α, and CDR3α having the amino acid sequences as shown in SEQ ID NOs:43, 76, and 29, respectively,
   CDR1α, CDR2α, and CDR3α having the amino acid sequences as shown in SEQ ID NOs:45, 28, and 29, respectively,
   CDR1α, CDR2α, and CDR3α having the amino acid sequences as shown in SEQ ID NOs: 165, 118, and 29, respectively,
   CDR1α, CDR2α, and CDR3α having the amino acid sequences as shown in SEQ ID NOs:27, 118, and 29, respectively,
   CDR1α, CDR2α, and CDR3α having the amino acid sequences as shown in SEQ ID NOs:45, 141, and 29, respectively,
   CDR1α, CDR2α, and CDR3α having the amino acid sequences as shown in SEQ ID NOs:170, 76, and 29, respectively,
   CDR1α, CDR2α, and CDR3α having the amino acid sequences as shown in SEQ ID NOs:43, 118, and 29, respectively, or
   CDR1α, CDR2α, and CDR3α having the amino acid sequences as shown in SEQ ID NOs: 152, 149, and 29, respectively.

In some embodiments, the modified TCR comprises:
(i) a β chain variable region, which comprises CDR1β, CDR2β, and CDR3β having the amino acid sequences as shown in SEQ ID NOs:24-26, respectively; and
(ii) an α chain variable region, which comprises
   CDR1α, CDR2α, and CDR3α having the amino acid sequences as shown in SEQ ID NOs:42, 28, and 29, respectively,
   CDR1α, CDR2α, and CDR3α having the amino acid sequences as shown in SEQ ID NOs:43, 28, and 29, respectively,
   CDR1α, CDR2α, and CDR3α having the amino acid sequences as shown in SEQ ID NOs:44, 28, and 29, respectively,
   CDR1α, CDR2α, and CDR3α having the amino acid sequences as shown in SEQ ID NOs:45, 28, and 29, respectively, or
   CDR1α, CDR2α, and CDR3α having the amino acid sequences as shown in SEQ ID NOs:27, 76, and 29, respectively.

In a preferred embodiment, the modified TCR comprises: (i) an α chain variable region, which comprises CDR1α, CDR2α, and CDR3α having the amino acid sequences as shown in SEQ ID NOs:42, 28, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:43, 28, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:44, 28, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:45, 28, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:27, 76, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:97, 28, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:100, 28, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:103, 28, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:27, 106, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:27, 109, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:27, 112, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:27, 115, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:27, 118, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:27, 121, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NsO:27, 124, and 29, respectively; and (ii) a β chain variable region, which comprises CDR1β, CDR2β, and CDR3β having the amino acid sequences as shown in SEQ ID NOs:24-26, respectively.

In a preferred embodiment, the modified TCR comprises: (i) an α chain variable region, which comprises CDR1α, CDR2α, and CDR3α having the amino acid sequences as shown in SEQ ID NOs:27, 127, and 29, respectively; and (ii) a β chain variable region, which comprises CDR1β, CDR2β, and CDR3β having the amino acid sequences as shown in SEQ ID NOs:24-26, respectively.

In some embodiments, the modified TCR comprises:
(i) an α chain variable region which comprises:
   CDR1α, CDR2α, and CDR3α with amino acid sequences as shown in SEQ ID NOs:42, 28, and 29, respectively,
   CDR1α, CDR2α, and CDR3α with amino acid sequences as shown in SEQ ID NOs:43, 28, and 29, respectively,
   CDR1α, CDR2α, and CDR3α with amino acid sequences as shown in SEQ ID NOs:44, 28, and 29, respectively,
   CDR1α, CDR2α, and CDR3α with amino acid sequences as shown in SEQ ID NOs:45, 28, and 29, respectively,
   CDR1α, CDR2α, and CDR3α with amino acid sequences as shown in SEQ ID NOs:27, 76, and 29, respectively,
   CDR1α, CDR2α, and CDR3α with amino acid sequences as shown in SEQ ID NOs:97, 28, and 29, respectively,
   CDR1α, CDR2α, and CDR3α with amino acid sequences as shown in SEQ ID NOs:100, 28, and 29, respectively,
   CDR1α, CDR2α, and CDR3α with amino acid sequences as shown in SEQ ID NOs:103, 28, and 29, respectively,
   CDR1α, CDR2α, and CDR3α with amino acid sequences as shown in SEQ ID NOs:27, 106, and 29, respectively,
   CDR1α, CDR2α, and CDR3α with amino acid sequences as shown in SEQ ID NOs:27, 109, and 29, respectively,
   CDR1α, CDR2α, and CDR3α with amino acid sequences as shown in SEQ ID NOs:27, 112, and 29, respectively,
   CDR1α, CDR2α, and CDR3α with amino acid sequences as shown in SEQ ID NOs:27, 115, and 29, respectively,
   CDR1α, CDR2α, and CDR3α with amino acid sequences as shown in SEQ ID NOs:27, 118, and 29, respectively,
   CDR1α, CDR2α, and CDR3α with amino acid sequences as shown in SEQ ID NOs:27, 121, and 29, respectively,
   CDR1α, CDR2α, and CDR3α with amino acid sequences as shown in SEQ ID NOs:27, 124, and 29, respectively,
   CDR1α, CDR2α, and CDR3α with amino acid sequences as shown in SEQ ID NOs:27, 127, and 29, respectively,
   CDR1α, CDR2α, and CDR3α with amino acid sequences as shown in SEQ ID NOs:103, 76, and 29, respectively,
   CDR1α, CDR2α, and CDR3α with amino acid sequences as shown in SEQ ID NOs:44, 76, and 29, respectively,
   CDR1α, CDR2α, and CDR3α with amino acid sequences as shown in SEQ ID NOs:140, 141, and 29, respectively,
   CDR1α, CDR2α, and CDR3α with amino acid sequences as shown in SEQ ID NOs:146, 118, and 29, respectively,
   CDR1α, CDR2α, and CDR3α with amino acid sequences as shown in SEQ ID NOs:45, 149, and 29, respectively,
   CDR1α, CDR2α, and CDR3α with amino acid sequences as shown in SEQ ID NOs:152, 149, and 29, respectively,
   CDR1α, CDR2α, and CDR3α with amino acid sequences as shown in SEQ ID NOs:43, 76, and 29, respectively,
   CDR1α, CDR2α, and CDR3α with amino acid sequences as shown in SEQ ID NOs:45, 28, and 29, respectively,
   CDR1α, CDR2α, and CDR3α with amino acid sequences as shown in SEQ ID NOs:165, 118, and 29, respectively,
   CDR1α, CDR2α, and CDR3α with amino acid sequences as shown in SEQ ID NOs:27, 118, and 29, respectively,
   CDR1α, CDR2α, and CDR3α with amino acid sequences as shown in SEQ ID NOs:45, 141, and 29, respectively,
   CDR1α, CDR2α, and CDR3α with amino acid sequences as shown in SEQ ID NOs:170, 76, and 29, respectively,
   CDR1α, CDR2α, and CDR3α with amino acid sequences as shown in SEQ ID NOs:43, 118, and 29, respectively, or
   CDR1α, CDR2α, and CDR3α with amino acid sequences as shown in SEQ ID NOs:152, 149, and 29, respectively; and
(ii) a β chain variable region which comprises:
   CDR1β, CDR2β, and CDR3β with amino acid sequences as shown in SEQ ID NOs:24, 30, and 26, respectively,
   CDR1β, CDR2β, and CDR3β with amino acid sequences as shown in SEQ ID NOs:24, 25, and 31, respectively,
   CDR1β, CDR2β, and CDR3β with amino acid sequences as shown in SEQ ID NOs:24, 25, and 32, respectively,
   CDR1β, CDR2β, and CDR3β with amino acid sequences as shown in SEQ ID NOs:24, 25, and 33, respectively,
   CDR1β, CDR2β, and CDR3β with amino acid sequences as shown in SEQ ID NOs:24, 25, and 34, respectively,
   CDR1β, CDR2β, and CDR3β with amino acid sequences as shown in SEQ ID NOs:24, 25, and 35, respectively,
   CDR1β, CDR2β, and CDR3β with amino acid sequences as shown in SEQ ID NOs:24, 30, and 33, respectively,
   CDR1β, CDR2β, and CDR3β with amino acid sequences as shown in SEQ ID NOs:24, 30, and 34, respectively,
   CDR1β, CDR2β, and CDR3β with amino acid sequences as shown in SEQ ID NOs:24, 30, and 35, respectively,
   CDR1β, CDR2β, and CDR3β with amino acid sequences as shown in SEQ ID NOs:24, 25, and 36, respectively,
   CDR1β, CDR2β, and CDR3β with amino acid sequences as shown in SEQ ID NOs:24, 25, and 37, respectively,
   CDR1β, CDR2β, and CDR3β with amino acid sequences as shown in SEQ ID NOs:24, 30, and 36, respectively,
   CDR1β, CDR2β, and CDR3β with amino acid sequences as shown in SEQ ID NOs:24, 30, and 37, respectively,
   CDR1β, CDR2β, and CDR3β with amino acid sequences as shown in SEQ ID NOs:24, 25, and 38, respectively,
   CDR1β, CDR2β, and CDR3β with amino acid sequences as shown in SEQ ID NOs:24, 25, and 39, respectively,
   CDR1β, CDR2β, and CDR3β with amino acid sequences as shown in SEQ ID NOs:24, 25, and 40, respectively,
   CDR1β, CDR2β, and CDR3β with amino acid sequences as shown in SEQ ID NOs:24, 25, and 41, respectively,
   CDR1β, CDR2β, and CDR3β with amino acid sequences as shown in SEQ ID NOs:24, 25, and 79, respectively,
   CDR1β, CDR2β, and CDR3β with amino acid sequences as shown in SEQ ID NOs:82, 25, and 26, respectively,
   CDR1β, CDR2β, and CDR3β with amino acid sequences as shown in SEQ ID NOs:24, 85, and 26, respectively,
   CDR1β, CDR2β, and CDR3β with amino acid sequences as shown in SEQ ID NOs:24, 88, and 26, respectively,
   CDR1β, CDR2β, and CDR3β with amino acid sequences as shown in SEQ ID NOs:24, 91, and 26, respectively,
   CDR1β, CDR2β, and CDR3β with amino acid sequences as shown in SEQ ID NOs:24, 25, and 94, respectively,
   CDR1β, CDR2β, and CDR3β with amino acid sequences as shown in SEQ ID NOs:130, 25, and 131, respectively,
   CDR1β, CDR2β, and CDR3β with amino acid sequences as shown in SEQ ID NOs:24, 91, and 35, respectively,
   CDR1β, CDR2β, and CDR3β with amino acid sequences as shown in SEQ ID NOs:130, 91, and 35, respectively,
   CDR1β, CDR2β, and CDR3β with amino acid sequences as shown in SEQ ID NOs:130, 153, and 35, respectively,
   CDR1β, CDR2β, and CDR3β with amino acid sequences as shown in SEQ ID NOs:130, 91, and 26, respectively,
   CDR1β, CDR2β, and CDR3β with amino acid sequences as shown in SEQ ID NOs:130, 91, and 162, respectively, or
   CDR1β, CDR2β, and CDR3β with amino acid sequences as shown in SEQ ID NOs:82, 91, and 33, respectively.

In some embodiments, the modified TCR comprises:
(i) an α chain variable region which comprises:
   CDR1α, CDR2α, and CDR3α with amino acid sequences as shown in SEQ ID NOs:42, 28, and 29 respectively;
   CDR1α, CDR2α, and CDR3α with amino acid sequences as shown in SEQ ID NOs:43, 28, and 29 respectively;
   CDR1α, CDR2α, and CDR3α with amino acid sequences as shown in SEQ ID NOs:44, 28, and 29 respectively;
   CDR1α, CDR2α, and CDR3α with amino acid sequences as shown in SEQ ID NOs:45, 28, and 29 respectively; or
   CDR1α, CDR2α, and CDR3α with amino acid sequences as shown in SEQ ID NOs:27, 76, and 29; and
(ii) a β chain variable region which comprises:
   CDR1β, CDR2β, and CDR3β with amino acid sequences as shown in SEQ ID NOs:24, 30, and 26 respectively;
   CDR1β, CDR2β, and CDR3β with amino acid sequences as shown in SEQ ID NOs:24, 25, and 31 respectively;
   CDR1β, CDR2β, and CDR3β with amino acid sequences as shown in SEQ ID NOs:24, 25, and 32 respectively;
   CDR1β, CDR2β, and CDR3β with amino acid sequences as shown in SEQ ID NOs:24, 25, and 33 respectively;
   CDR1β, CDR2β, and CDR3β with amino acid sequences as shown in SEQ ID NOs:24, 25, and 34 respectively;
   CDR1β, CDR2β, and CDR3β with amino acid sequences as shown in SEQ ID NOs:24, 25, and 35 respectively;
   CDR1β, CDR2β, and CDR3β with amino acid sequences as shown in SEQ ID NOs:24, 30, and 33 respectively;
   CDR1β, CDR2β, and CDR3β with amino acid sequences as shown in SEQ ID NOs:24, 30, and 34 respectively;
   CDR1β, CDR2β, and CDR3β with amino acid sequences as shown in SEQ ID NOs:24, 30, and 35 respectively;
   CDR1β, CDR2β, and CDR3β with amino acid sequences as shown in SEQ ID NOs:24, 25, and 36 respectively;
   CDR1β, CDR2β, and CDR3β with amino acid sequences as shown in SEQ ID NOs:24, 25, and 37 respectively;
   CDR1β, CDR2β, and CDR3β with amino acid sequences as shown in SEQ ID NOs:24, 30, and 36 respectively;
   CDR1β, CDR2β, and CDR3β with amino acid sequences as shown in SEQ ID NOs:24, 30, and 37 respectively;
   CDR1β, CDR2β, and CDR3β with amino acid sequences as shown in SEQ ID NOs:24, 25, and 38 respectively;
   CDR1β, CDR2β, and CDR3β with amino acid sequences as shown in SEQ ID NOs:24, 25, and 39 respectively;
   CDR1β, CDR2β, and CDR3β with amino acid sequences as shown in SEQ ID NOs:24, 25, and 40 respectively;
   CDR1β, CDR2β, and CDR3β with amino acid sequences as shown in SEQ ID NOs:24, 25, and 41 respectively.

In a preferred embodiment, the modified TCR comprises: (i) an α chain variable region which comprises CDR1α, CDR2α, and CDR3α with amino acid sequences as shown in SEQ ID NOs: 103, 76, and 29 respectively; and (ii) a β chain variable region which comprises CDR1β, CDR2β, and CDR3β with amino acid sequences as shown in SEQ ID NOs:130, 25, and 131 respectively.

In a preferred embodiment, the modified TCR comprises: (i) an α chain variable region which comprises CDR1α, CDR2α, and CDR3α with amino acid sequences as shown in SEQ ID NOs:44, 76, and 29 respectively; and (ii) a β chain variable region which comprises CDR1β, CDR2β, and CDR3β with amino acid sequences as shown in SEQ ID NOs:24, 91, and 35 respectively.

In a preferred embodiment, the modified TCR comprises: (i) an α chain variable region which comprises CDR1α, CDR2α, and CDR3α with amino acid sequences as shown in SEQ ID NOs: 140, 141, and 29 respectively; and (ii) a β chain variable region which comprises CDR1β, CDR2β, and CDR3β with amino acid sequences as shown in SEQ ID NOs:130, 91, and 35 respectively.

In a preferred embodiment, the modified TCR comprises: (i) an α chain variable region which comprises CDR1α, CDR2α, and CDR3α with amino acid sequences as shown in SEQ ID NOs: 146, 118, and 29 respectively; and (ii) a β chain variable region which comprises CDR1β, CDR2β, and CDR3β with amino acid sequences as shown in SEQ ID NOs:130, 91, and 35 respectively.

In a preferred embodiment, the modified TCR comprises: (i) an α chain variable region which comprises CDR1α, CDR2α, and CDR3α with amino acid sequences as shown in SEQ ID NOs:45, 149, and 29 respectively; and (ii) a β chain variable region which comprises CDR1β, CDR2β, and CDR3β with amino acid sequences as shown in SEQ ID NOs:130, 91, and 35 respectively.

In a preferred embodiment, the modified TCR comprises: (i) an α chain variable region which comprises CDR1α, CDR2α, and CDR3α with amino acid sequences as shown in SEQ ID NOs: 152, 149, and 29 respectively; and (ii) a β chain variable region which comprises CDR1β, CDR2β, and CDR3β with amino acid sequences as shown in SEQ ID NOs:130, 153, and 35 respectively.

In a preferred embodiment, the modified TCR comprises: (i) an α chain variable region which comprises CDR1α, CDR2α, and CDR3α with amino acid sequences as shown in SEQ ID NOs:43, 76, and 29 respectively; and (ii) a β chain variable region which comprises CDR1β, CDR2β, and CDR3β with amino acid sequences as shown in SEQ ID NOs:130, 91, and 26 respectively.

In a preferred embodiment, the modified TCR comprises: (i) an α chain variable region which comprises CDR1α, CDR2α, and CDR3α with amino acid sequences as shown in SEQ ID NOs:45, 28, and 29 respectively; and (ii) a β chain variable region which comprises CDR1β, CDR2β, and CDR3β with amino acid sequences as shown in SEQ ID NOs:130, 91, and 162 respectively.

In a preferred embodiment, the modified TCR comprises: (i) an α chain variable region which comprises CDR1α, CDR2α, and CDR3α with amino acid sequences as shown in SEQ ID NOs: 165, 118, and 29 respectively; and (ii) a β chain variable region which comprises CDR1β, CDR2β, and CDR3β with amino acid sequences as shown in SEQ ID NOs:130, 91, and 35 respectively.

In a preferred embodiment, the modified TCR comprises: (i) an α chain variable region which comprises CDR1α, CDR2α, and CDR3α with amino acid sequences as shown in SEQ ID NOs:27, 118, and 29 respectively; and (ii) a β chain variable region which comprises CDR1β, CDR2β, and CDR3β with amino acid sequences as shown in SEQ ID NOs:130, 91, and 35 respectively.

In a preferred embodiment, the modified TCR comprises: (i) an α chain variable region which comprises CDR1α, CDR2α, and CDR3α with amino acid sequences as shown in SEQ ID NOs:45, 141, and 29 respectively; and (ii) a β chain variable region which comprises CDR1β, CDR2β, and CDR3β with amino acid sequences as shown in SEQ ID NOs:130, 91, and 35 respectively.

In a preferred embodiment, the modified TCR comprises: (i) an α chain variable region which comprises CDR1α, CDR2α, and CDR3α with amino acid sequences as shown in SEQ ID NOs:170, 76, and 29 respectively; and (ii) a β chain variable region which comprises CDR1β, CDR2β, and CDR3β with amino acid sequences as shown in SEQ ID NOs:130, 91, and 35 respectively.

In a preferred embodiment, the modified TCR comprises: (i) an α chain variable region which comprises CDR1α, CDR2α, and CDR3α with amino acid sequences as shown in SEQ ID NOs:43, 118, and 29 respectively; and (ii) a β chain variable region which comprises CDR1β, CDR2β, and CDR3β with amino acid sequences as shown in SEQ ID NOs:130, 91, and 35 respectively.

In a preferred embodiment, the modified TCR comprises: (i) an α chain variable region which comprises CDR1α, CDR2α, and CDR3α with amino acid sequences as shown in SEQ ID NOs: 152, 149, and 29 respectively; and (ii) a β chain variable region which comprises CDR1β, CDR2β, and CDR3β with amino acid sequences as shown in SEQ ID NOs:82, 91, and 33 respectively.

In some embodiments, the modified TCR comprises an α chain variable region having the amino acid sequence as shown in SEQ ID NO:46 or an amino acid sequence with at least 80%, at least 85%, at least 90%, at least 91%, at least 92%, at least 93%, at least 94%, at least 95%, at least 96%, at least 97%, at least 98%, or at least 99% sequence identity to SEQ ID NO:46; and/or a β chain variable region having an amino acid sequence selected from any of SEQ ID NOs:48-64, 80, 83, 86, 89, 92, 95, 133, 137, 143, 155, 159, 163, 175 or an amino acid sequence with at least 80%, at least 85%, at least 90%, at least 91%, at least 92%, at least 93%, at least 94%, at least 95%, at least 96%, at least 97%, at least 98%, or at least 99% sequence identity to any of SEQ ID NOs:48-64, 80, 83, 86, 89, 92, 95, 133, 137, 143, 155, 159, 163, 175.

In some embodiments, the modified TCR comprises an α chain variable region having an amino acid sequence selected from any of SEQ ID NOs: 65-68, 77, 98, 101, 104, 107, 110, 113, 116, 119, 122, 125, 128, 132, 136, 142, 147, 150, 154, 158, 166, 168, 171, 173, or an amino acid sequence with at least 80%, at least 85%, at least 90%, at least 95%, or at least 99% sequence identity to any of SEQ ID NOs: 65-68, 77, 98, 101, 104, 107, 110, 113, 116, 119, 122, 125, 128, 132, 136, 142, 147, 150, 154, 158, 166, 168, 171, 173; and/or a β chain variable region having the amino acid sequence of SEQ ID NO: 47 or an amino acid sequence with at least 80%, at least 85%, at least 90%, at least 95%, or at least 99% sequence identity to SEQ ID NO: 47.

In some embodiments, the modified TCR comprises an α chain variable region having an amino acid sequence selected from any of SEQ ID NOs: 65-68, 77, 98, 101, 104, 107, 110, 113, 116, 119, 122, 125, 128, 132, 136, 142, 147, 150, 154, 158, 166, 168, 171, 173, or an amino acid sequence with at least 80%, at least 85%, at least 90%, at least 95%, or at least 99% sequence identity to any of SEQ ID NOs: 65-68, 77, 98, 101, 104, 107, 110, 113, 116, 119, 122, 125, 128, 132, 136, 142, 147, 150, 154, 158, 166, 168, 171, 173; and/or a β chain variable region having an amino acid sequence selected from any of SEQ ID NOs: 48-64, 80, 83, 86, 89, 92, 95, 133, 137, 143, 155, 159, 163, 175, or an amino acid sequence with at least 80%, at least 85%, at least 90%, at least 95%, or at least 99% sequence identity to any of SEQ ID NOs: 48-64, 80, 83, 86, 89, 92, 95, 133, 137, 143, 155, 159, 163, 175.

As used herein, the term "sequence identity" refers to the degree to which two (nucleotide or amino acid) sequences have the same residues at the same positions in an alignment and is typically expressed as a percentage. Preferably, identity is determined over the entire length of the sequences being compared. Thus, two copies with identical sequences have 100% identity, but sequences with lower levels of conservation and with deletions, additions, or substitutions may have lower levels of identity. Those skilled in the art will recognize that some algorithms can be used to determine sequence identity using standard parameters, such as Blast (Altschul et al. (1997) Nucleic Acids Res. 25:3389-3402), Blast2 (Altschul et al. (1990) J. Mol. Biol. 215:403-410), Smith-Waterman (Smith et al. (1981) J. Mol. Biol. 147:195-197), and ClustalW.

Thus, for example, the amino acid sequence of SEQ ID NO: 46 or 47 can serve as a "subject sequence" or "reference sequence," while a differing TCR α chain or β chain variable region amino acid sequence can serve as a "query sequence".

In some embodiments, the modified TCR comprises an α chain variable region having the amino acid sequence of SEQ ID NO: 46; and a β chain variable region having an amino acid sequence selected from any of SEQ ID NOs: 48-64, 80, 83, 86, 89, 92, 95, 133, 137, 143, 155, 159, 163, 175.

In some embodiments, the modified TCR comprises an α chain variable region having an amino acid sequence selected from any of SEQ ID NOs: 65-68, 77, 98, 101, 104, 107, 110, 113, 116, 119, 122, 125, 128, 132, 136, 142, 147, 150, 154, 158, 166, 168, 171, 173; and a β chain variable region having the amino acid sequence of SEQ ID NO: 47.

In some embodiments, the modified TCR comprises an α chain variable region having an amino acid sequence selected from any of SEQ ID NOs: 65-68, 77, 98, 101, 104, 107, 110, 113, 116, 119, 122, 125, 128, 132, 136, 142, 147, 150, 154, 158, 166, 168, 171, 173; and a β chain variable region having an amino acid sequence selected from any of SEQ ID NOs: 48-64, 80, 83, 86, 89, 92, 95, 133, 137, 143, 155, 159, 163, 175.

In a preferred embodiment, the modified TCR comprises an α chain variable region having the amino acid sequence as shown in SEQ ID NO:46 and a β chain variable region having the amino acid sequence as shown in SEQ ID NO:48.

In a preferred embodiment, the modified TCR comprises an α chain variable region having the amino acid sequence as shown in SEQ ID NO:46 and a β chain variable region having the amino acid sequence as shown in SEQ ID NO:49.

In a preferred embodiment, the modified TCR comprises an α chain variable region having the amino acid sequence as shown in SEQ ID NO:46 and a β chain variable region having the amino acid sequence as shown in SEQ ID NO:50.

In a preferred embodiment, the modified TCR comprises an α chain variable region having the amino acid sequence as shown in SEQ ID NO:46 and a β chain variable region having the amino acid sequence as shown in SEQ ID NO:51.

In a preferred embodiment, the modified TCR comprises an α chain variable region having the amino acid sequence as shown in SEQ ID NO:46 and a β chain variable region having the amino acid sequence as shown in SEQ ID NO:52.

In a preferred embodiment, the modified TCR comprises an α chain variable region having the amino acid sequence as shown in SEQ ID NO:46 and a β chain variable region having the amino acid sequence as shown in SEQ ID NO:53.

In a preferred embodiment, the modified TCR comprises an α chain variable region having the amino acid sequence as shown in SEQ ID NO:46 and a β chain variable region having the amino acid sequence as shown in SEQ ID NO:54.

In a preferred embodiment, the modified TCR comprises an α chain variable region having the amino acid sequence as shown in SEQ ID NO:46 and a β chain variable region having the amino acid sequence as shown in SEQ ID NO:55.

In a preferred embodiment, the modified TCR comprises an α chain variable region having the amino acid sequence as shown in SEQ ID NO:46 and a β chain variable region having the amino acid sequence as shown in SEQ ID NO:56.

In a preferred embodiment, the modified TCR comprises an α chain variable region having the amino acid sequence as shown in SEQ ID NO:46 and a β chain variable region having the amino acid sequence as shown in SEQ ID NO:57.

In a preferred embodiment, the modified TCR comprises an α chain variable region having the amino acid sequence as shown in SEQ ID NO:46 and a β chain variable region having the amino acid sequence as shown in SEQ ID NO:58.

In a preferred embodiment, the modified TCR comprises an α chain variable region having the amino acid sequence as shown in SEQ ID NO:46 and a β chain variable region having the amino acid sequence as shown in SEQ ID NO:59.

In a preferred embodiment, the modified TCR comprises an α chain variable region having the amino acid sequence as shown in SEQ ID NO:46 and a β chain variable region having the amino acid sequence as shown in SEQ ID NO:60.

In a preferred embodiment, the modified TCR comprises an α chain variable region having the amino acid sequence as shown in SEQ ID NO:46 and a β chain variable region having the amino acid sequence as shown in SEQ ID NO:61.

In a preferred embodiment, the modified TCR comprises an α chain variable region having the amino acid sequence as shown in SEQ ID NO:46 and a β chain variable region having the amino acid sequence as shown in SEQ ID NO:62.

In a preferred embodiment, the modified TCR comprises an α chain variable region having the amino acid sequence as shown in SEQ ID NO:46 and a β chain variable region having the amino acid sequence as shown in SEQ ID NO:63.

In a preferred embodiment, the modified TCR comprises an α chain variable region having the amino acid sequence as shown in SEQ ID NO:46 and a β chain variable region having the amino acid sequence as shown in SEQ ID NO:64.

In a preferred embodiment, the modified TCR comprises an α chain variable region having the amino acid sequence as shown in SEQ ID NO:65 and a β chain variable region having the amino acid sequence as shown in SEQ ID NO:47.

In a preferred embodiment, the modified TCR comprises an α chain variable region having the amino acid sequence as shown in SEQ ID NO:66 and a β chain variable region having the amino acid sequence as shown in SEQ ID NO:47.

In a preferred embodiment, the modified TCR comprises an α chain variable region having the amino acid sequence as shown in SEQ ID NO:67 and a β chain variable region having the amino acid sequence as shown in SEQ ID NO:47.

In a preferred embodiment, the modified TCR comprises an α chain variable region having the amino acid sequence as shown in SEQ ID NO:68 and a β chain variable region having the amino acid sequence as shown in SEQ ID NO:47.

In a preferred embodiment, the modified TCR comprises an α chain variable region having the amino acid sequence as shown in SEQ ID NO:77 and a β chain variable region having the amino acid sequence as shown in SEQ ID NO:47.

In a preferred embodiment, the modified TCR comprises an α chain variable region having the amino acid sequence as shown in SEQ ID NO:46 and a β chain variable region having the amino acid sequence as shown in SEQ ID NO:80.

In a preferred embodiment, the modified TCR comprises an α chain variable region having the amino acid sequence as shown in SEQ ID NO:46 and a β chain variable region having the amino acid sequence as shown in SEQ ID NO:83.

In a preferred embodiment, the modified TCR comprises an α chain variable region having the amino acid sequence as shown in SEQ ID NO:46 and a β chain variable region having the amino acid sequence as shown in SEQ ID NO:86.

In a preferred embodiment, the modified TCR comprises an α chain variable region having the amino acid sequence as shown in SEQ ID NO:46 and a β chain variable region having the amino acid sequence as shown in SEQ ID NO:89.

In a preferred embodiment, the modified TCR comprises an α chain variable region having the amino acid sequence as shown in SEQ ID NO:46 and a β chain variable region having the amino acid sequence as shown in SEQ ID NO:92.

In a preferred embodiment, the modified TCR comprises an α chain variable region having the amino acid sequence as shown in SEQ ID NO:46 and a β chain variable region having the amino acid sequence as shown in SEQ ID NO:95.

In a preferred embodiment, the modified TCR comprises an α chain variable region having the amino acid sequence as shown in SEQ ID NO:98 and a β chain variable region having the amino acid sequence as shown in SEQ ID NO:47.

In a preferred embodiment, the modified TCR comprises an α chain variable region having the amino acid sequence as shown in SEQ ID NO:101 and a β chain variable region having the amino acid sequence as shown in SEQ ID NO:47.

In a preferred embodiment, the modified TCR comprises an α chain variable region having the amino acid sequence as shown in SEQ ID NO:104 and a β chain variable region having the amino acid sequence as shown in SEQ ID NO:47.

In a preferred embodiment, the modified TCR comprises an α chain variable region having the amino acid sequence as shown in SEQ ID NO:107 and a β chain variable region having the amino acid sequence as shown in SEQ ID NO:47.

In a preferred embodiment, the modified TCR comprises an α chain variable region having the amino acid sequence as shown in SEQ ID NO:110 and a β chain variable region having the amino acid sequence as shown in SEQ ID NO:47.

In a preferred embodiment, the modified TCR comprises an α chain variable region having the amino acid sequence as shown in SEQ ID NO:113 and a β chain variable region having the amino acid sequence as shown in SEQ ID NO:47.

In a preferred embodiment, the modified TCR comprises an α chain variable region having the amino acid sequence as shown in SEQ ID NO:116 and a β chain variable region having the amino acid sequence as shown in SEQ ID NO:47.

In a preferred embodiment, the modified TCR comprises an α chain variable region having the amino acid sequence as shown in SEQ ID NO:119 and a β chain variable region having the amino acid sequence as shown in SEQ ID NO:47.

In a preferred embodiment, the modified TCR comprises an α chain variable region having the amino acid sequence as shown in SEQ ID NO:122 and a β chain variable region having the amino acid sequence as shown in SEQ ID NO:47.

In a preferred embodiment, the modified TCR comprises an α chain variable region having the amino acid sequence as shown in SEQ ID NO:125 and a β chain variable region having the amino acid sequence as shown in SEQ ID NO:47.

In a preferred embodiment, the modified TCR comprises an α chain variable region having the amino acid sequence as shown in SEQ ID NO:128 and a β chain variable region having the amino acid sequence as shown in SEQ ID NO:47.

In a preferred embodiment, the modified TCR comprises an α chain variable region having the amino acid sequence as shown in SEQ ID NO:132 and a β chain variable region having the amino acid sequence as shown in SEQ ID NO: 133.

In a preferred embodiment, the modified TCR comprises an α chain variable region having the amino acid sequence as shown in SEQ ID NO:136 and a β chain variable region having the amino acid sequence as shown in SEQ ID NO: 137.

In a preferred embodiment, the modified TCR comprises an α chain variable region having the amino acid sequence as shown in SEQ ID NO:142 and a β chain variable region having the amino acid sequence as shown in SEQ ID NO: 143.

In a preferred embodiment, the modified TCR comprises an α chain variable region having the amino acid sequence as shown in SEQ ID NO:147 and a β chain variable region having the amino acid sequence as shown in SEQ ID NO: 143.

In a preferred embodiment, the modified TCR comprises an α chain variable region having the amino acid sequence as shown in SEQ ID NO:150 and a β chain variable region having the amino acid sequence as shown in SEQ ID NO: 143.

In a preferred embodiment, the modified TCR comprises an α chain variable region having the amino acid sequence as shown in SEQ ID NO:154 and a β chain variable region having the amino acid sequence as shown in SEQ ID NO: 155.

In a preferred embodiment, the modified TCR comprises an α chain variable region having the amino acid sequence as shown in SEQ ID NO:158 and a β chain variable region having the amino acid sequence as shown in SEQ ID NO:159.

In a preferred embodiment, the modified TCR comprises an α chain variable region having the amino acid sequence as shown in SEQ ID NO:68 and a β chain variable region having the amino acid sequence as shown in SEQ ID NO: 163.

In a preferred embodiment, the modified TCR comprises an α chain variable region having the amino acid sequence as shown in SEQ ID NO:166 and a β chain variable region having the amino acid sequence as shown in SEQ ID NO: 143.

In a preferred embodiment, the modified TCR comprises an α chain variable region having the amino acid sequence as shown in SEQ ID NO:119 and a β chain variable region having the amino acid sequence as shown in SEQ ID NO: 143.

In a preferred embodiment, the modified TCR comprises an α chain variable region having the amino acid sequence as shown in SEQ ID NO:168 and a β chain variable region having the amino acid sequence as shown in SEQ ID NO: 143.

In a preferred embodiment, the modified TCR comprises an α chain variable region having the amino acid sequence as shown in SEQ ID NO:171 and a β chain variable region having the amino acid sequence as shown in SEQ ID NO: 143.

In a preferred embodiment, the modified TCR comprises an α chain variable region having the amino acid sequence as shown in SEQ ID NO:173 and a β chain variable region having the amino acid sequence as shown in SEQ ID NO: 143.

In a preferred embodiment, the modified TCR comprises an α chain variable region having the amino acid sequence as shown in SEQ ID NO:154 and a β chain variable region having the amino acid sequence as shown in SEQ ID NO: 175.

In some embodiments, the modified TCR also comprises a TCR constant region.

As used herein, the term "constant region" may be a human constant region or derived from another species, resulting in a "chimeric" TCR. For example, human α chain and/or β chain may be replaced by their murine counterparts ("murine-derived"), and the murine counterparts have been found to enhance surface expression of the human TCR and enhance binding stability of the human TCR to CD3 co-receptors by supporting preferential pairing of TCR α and β chains.

In some embodiments, the TCR constant region is a murine constant region or a human constant region.

It is reported that the addition of disulfide bonds to the constant region may promote the correct pairing of TCR α and β chains (Kuball J et al. Blood. 2007 Mar 15;109(6):2331-8). Therefore, the present invention also envisages the addition of one or more cysteine modifications to the constant region to form disulfide bonds between the TCR α and TCR β chains.

In some embodiments, the TCR constant region comprises a TCR α chain constant region and/or a TCR β chain constant region; preferably, the TCR α chain constant region and/or TCR β chain constant region comprises at least one cysteine mutation relative to a wild-type sequence to form a disulfide bond between the TCR α chain and the TCR β chain.

In some embodiments, the cysteine mutation is at one or more of the following positions: position 48 of the wild-type human TCR α chain constant region, position 48 of the wild-type murine TCR α chain constant region, position 57 of the wild-type human TCR β chain constant region, and position 57 of the wild-type murine TCR β chain constant region.

The sequence of the wild-type TCR constant region can be found in the public database of the International Immunogenetics Information System (IMGT). For example, the sequence of the TCR α chain constant domain is "TRAC*01" and the sequence of TCR β chain constant domain is "TRBC1*01 " or "TRBC2*01".

The positions of the amino acid sequences of the TCR constant region in the present invention are numbered according to the nomenclature of the International Immunogenetics Information System (IMGT). For example, if an amino acid in the TCR α chain constant region (TRAC) is designated with a position number of 48 in the IMGT, it is described herein as the amino acid at position 48 of the TCR α chain constant region (TRAC); if an amino acid in the TCR β chain constant region (TRBC) is designated with a position number of 57 in the IMGT, it is described herein as the amino acid at position 57 of the TCR β chain constant region ( TRBC). When the sequence position numbering of other amino acids is specifically described in the present invention, they are numbered as specifically described.

In some embodiments, the TCR α chain constant region further comprises an LVL mutation or an LIV mutation such that the constant region (and/or the transmembrane region) comprises the amino acid sequence LLVIVLRIL. For example, when the TCR α chain comprises a human constant region, the human constant region may comprise an LVL mutation such that the constant region (and/or the transmembrane region) comprises the amino acid sequence L**L**VI**VL**RIL. When the TCR α chain contains a murine constant region, the murine constant region may comprise an LIV mutation such that the constant region (and/or the transmembrane region) comprises the amino acid sequence L**L**V**IV**LRIL.

In some embodiments, the TCR α chain constant region comprises an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity with SEQ ID NO: 71, and/or the TCR β chain constant region comprises an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity with SEQ ID NO: 72.

In some embodiments, the TCR α chain constant region comprises the amino acid sequence as shown in SEQ ID NO: 71, and the TCR β chain constant region comprises the amino acid sequence as shown in SEQ ID NO: 72.

In some embodiments, the modified TCR may comprise a signal peptide sequence, for example, the signal peptide sequence may be comprised at the N-terminus of the α chain and/or β chain. The signal peptide sequence can be any suitable sequence known in the art. For example, the α chain signal peptide sequence of the TCR of the present invention may have the amino acid sequence as shown in SEQ ID NO: 74 (MKTFAGFSFLFLWLQLDCMS). The β chain signal peptide sequence of the TCR of the present invention may have the amino acid sequence as shown in SEQ ID NO: 75 (MLLLLLLLGPGSGL).

In some embodiments, the modified TCR comprises a TCR α chain having the amino acid sequence as shown in SEQ ID NO: 69 or an amino acid sequence with at least 80%, at least 85%, at least 90%, at least 95%, or at least 99% sequence identity with SEQ ID NO: 69; and/or a TCR β chain having an amino acid sequence selected from any of SEQ ID NOs: 3-19, 81, 84, 87, 90, 93, 96, 135, 139, 145, 157, 161, 164, 176, or an amino acid sequence with at least 80%, at least 85%, at least 90%, at least 95%, or at least 99% sequence identity with any of SEQ ID NOs: 3-19, 81, 84, 87, 90, 93, 96, 135, 139, 145, 157, 161, 164, 176. In some embodiments, the modified TCR comprises a TCR α chain having the amino acid sequence as shown in SEQ ID NO: 69 and a TCR β chain having an amino acid sequence selected from any of SEQ ID NOs: 3-19, 81, 84, 87, 90, 93, 96, 135, 139, 145, 157, 161, 164, 176.

The amino acid sequences for SEQ ID NOs: 69 and 3-19 are as follows:

MKTFAGFSFLFLWLQLDCMSRGEDVEQSLFLSVREGDSSVINCTYTDSSSTYLYWYKQE
PGAGLQLLTYIFSNMDMKQDQRLTVLLNKKDKHLSLRIADTQTGDSAIYFCAGSGGGTDKLI
FGTGTRLQVFPNIQNPEPAVYQLKDPRSQDSTLCLFTDFDSQINVPKTMESGTFITDKCVLDM
KAMDSKSNGAIAWSNQTSFTCQDIFKETNATYPSSDVPCDATLTEKSFETDMNLNFQNLLVIV
LRILLLKVAGFNLLMTLRLWSS (SEQ ID NO:69)

MLLLLLLLGPGSGLGAVVSQHPSWVICKSGTSVKIECRSLDFQATTMFWYRQFPKQSLM
LMATSDYQTCVTYEQGVEKDKFLINHASLTLSTLTVTSAHPEDSSFYICSAREPGQGPYEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:3)

MLLLLLLLGPGSGLGAVVSQHPSWVICKSGTSVKIECRSLDFQATTMFWYRQFPKQSLM
LMATSNEGSKATYEQGVEKDKFLINHASLTLSTLTVTSAHPEDSSFYICSAREPGGGPYEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:4)

MLLLLLLLGPGSGLGAVVSQHPSWVICKSGTSVKIECRSLDFQATTMFWYRQFPKQSLM
LMATSNEGSKATYEQGVEKDKFLINHASLTLSTLTVTSAHPEDSSFYICSAREPGSGPYEQYFG
PGTRLTVTEDLRNVTPPKVSLFEPSKAEIANKQKATLVCLARGFFPDHVELSWWVNGKEVHS
GVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:5)

MLLLLLLLGPGSGLGAVVSQHPSWVICKSGTSVKIECRSLDFQATTMFWYRQFPKQSLM
LMATSNEGSKATYEQGVEKDKFLINHASLTLSTLTVTSAHPEDSSFYICSAREPGQGPFEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:6)

MLLLLLLLGPGSGLGAVVSQHPSWVICKSGTSVKIECRSLDFQATTMFWYRQFPKQSLM
LMATSNEGSKATYEQGVEKDKFLINHASLTLSTLTVTSAHPEDSSFYICSAREPGQGPLEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:7)

MLLLLLLLGPGSGLGAVVSQHPSWVICKSGTSVKIECRSLDFQATTMFWYRQFPKQSLM
LMATSNEGSKATYEQGVEKDKFLINHASLTLSTLTVTSAHPEDSSFYICSAREPGQGPWEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:8)

MLLLLLLLGPGSGLGAVVSQHPSWVICKSGTSVKIECRSLDFQATTMFWYRQFPKQSLM
LMATSDYQTCVTYEQGVEKDKFLINHASLTLSTLTVTSAHPEDSSFYICSAREPGQGPFEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:9)

MLLLLLLLGPGSGLGAVVSQHPSWVICKSGTSVKIECRSLDFQATTMFWYRQFPKQSLM
LMATSDYQTCVTYEQGVEKDKFLINHASLTLSTLTVTSAHPEDSSFYICSAREPGQGPLEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:10)

MLLLLLLLGPGSGLGAVVSQHPSWVICKSGTSVKIECRSLDFQATTMFWYRQFPKQSLM
LMATSDYQTCVTYEQGVEKDKFLINHASLTLSTLTVTSAHPEDSSFYICSAREPGQGPWEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:11)

MLLLLLLLGPGSGLGAVVSQHPSWVICKSGTSVKIECRSLDFQATTMFWYRQFPKQSLM
LMATSNEGSKATYEQGVEKDKFLINHASLTLSTLTVTSAHPEDSSFYICSAREPGGGPFEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:12)

MLLLLLLLGPGSGLGAVVSQHPSWVICKSGTSVKIECRSLDFQATTMFWYRQFPKQSLM
LMATSNEGSKATYEQGVEKDKFLINHASLTLSTLTVTSAHPEDSSFYICSAREPGSGPFEQYFG
PGTRLTVTEDLRNVTPPKVSLFEPSKAEIANKQKATLVCLARGFFPDHVELSWWVNGKEVHS
GVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:13)

MLLLLLLLGPGSGLGAVVSQHPSWVICKSGTSVKIECRSLDFQATTMFWYRQFPKQSLM
LMATSDYQTCVTYEQGVEKDKFLINHASLTLSTLTVTSAHPEDSSFYICSAREPGGGPFEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:14)

MLLLLLLLGPGSGLGAVVSQHPSWVICKSGTSVKIECRSLDFQATTMFWYRQFPKQSLM
LMATSDYQTCVTYEQGVEKDKFLINHASLTLSTLTVTSAHPEDSSFYICSAREPGSGPFEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:15)

MLLLLLLLGPGSGLGAVVSQHPSWVICKSGTSVKIECRSLDFQATTMFWYRQFPKQSLM
LMATSNEGSKATYEQGVEKDKFLINHASLTLSTLTVTSAHPEDSSFYICLAREPGQGPYEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:16)

MLLLLLLLGPGSGLGAVVSQHPSWVICKSGTSVKIECRSLDFQATTMFWYRQFPKQSLM
LMATSNEGSKATYEQGVEKDKFLINHASLTLSTLTVTSAHPEDSSFYICQAREPGQGPYEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:17)

MLLLLLLLGPGSGLGAVVSQHPSWVICKSGTSVKIECRSLDFQATTMFWYRQFPKQSLM
LMATSNEGSKATYEQGVEKDKFLINHASLTLSTLTVTSAHPEDSSFYICNAREPGQGPYEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:18)

MLLLLLLLGPGSGLGAVVSQHPSWVICKSGTSVKIECRSLDFQATTMFWYRQFPKQSLM
LMATSNEGSKATYEQGVEKDKFLINHASLTLSTLTVTSAHPEDSSFYICHAREPGQGPYEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:19)

In some embodiments, the modified TCR comprises a TCR α chain having an amino acid sequence selected from any of SEQ ID NOs: 20-23, 78, 99, 102, 105, 108, 111, 114, 117, 120, 123, 126, 129, 134, 138, 144, 148, 151, 156, 160, 167, 169, 172, 174, or an amino acid sequence with at least 80%, at least 85%, at least 90%, at least 95%, or at least 99% sequence identity with any of SEQ ID NOs: 20-23, 78, 99, 102, 105, 108, 111, 114, 117, 120, 123, 126, 129, 134, 138, 144, 148, 151, 156, 160, 167, 169, 172, 174; and/or a TCR β chain having the amino acid sequence as shown in SEQ ID NO: 70 or an amino acid sequence with at least 80%, at least 85%, at least 90%, at least 95%, or at least 99% sequence identity with SEQ ID NO: 70. In some embodiments, the modified TCR comprises a TCR α chain having an amino acid sequence selected from any of SEQ ID NOs: 20-23, 78, 99, 102, 105, 108, 111, 114, 117, 120, 123, 126, 129, 134, 138, 144, 148, 151, 156, 160, 167, 169, 172, 174; and a TCR β chain having the amino acid sequence as shown in SEQ ID NO: 70.

The amino acid sequences for SEQ ID NOs: 20-23, 78, and 70 are as follows:

MKTFAGFSFLFLWLQLDCMSRGEDVEQSLFLSVREGDSSVINCTYTDSLSTYLYWYKQE
PGAGLQLLTYIFSNMDMKQDQRLTVLLNKKDKHLSLRIADTQTGDSAIYFCAGSGGGTDKLI
FGTGTRLQVFPNIQNPEPAVYQLKDPRSQDSTLCLFTDFDSQINVPKTMESGTFITDKCVLDM
KAMDSKSNGAIAWSNQTSFTCQDIFKETNATYPSSDVPCDATLTEKSFETDMNLNFQNLLVIV
LRILLLKVAGFNLLMTLRLWSS (SEQ ID NO:20)

MKTFAGFSFLFLWLQLDCMSRGEDVEQSLFLSVREGDSSVINCTYTDSSMTYLYWYKQ
EPGAGLQLLTYIFSNMDMKQDQRLTVLLNKKDKHLSLRIADTQTGDSAIYFCAGSGGGTDKL
IFGTGTRLQVFPNIQNPEPAVYQLKDPRSQDSTLCLFTDFDSQINVPKTMESGTFITDKCVLDM
KAMDSKSNGAIAWSNQTSFTCQDIFKETNATYPSSDVPCDATLTEKSFETDMNLNFQNLLVIV
LRILLLKVAGFNLLMTLRLWSS (SEQ ID NO:21)

MKTFAGFSFLFLWLQLDCMSRGEDVEQSLFLSVREGDSSVINCTYTDSSLTYLYWYKQE
PGAGLQLLTYIFSNMDMKQDQRLTVLLNKKDKHLSLRIADTQTGDSAIYFCAGSGGGTDKLI
FGTGTRLQVFPNIQNPEPAVYQLKDPRSQDSTLCLFTDFDSQINVPKTMESGTFITDKCVLDM
KAMDSKSNGAIAWSNQTSFTCQDIFKETNATYPSSDVPCDATLTEKSFETDMNLNFQNLLVIV
LRILLLKVAGFNLLMTLRLWSS (SEQ ID NO:22)

MKTFAGFSFLFLWLQLDCMSRGEDVEQSLFLSVREGDSSVINCTYTDSSQTYLYWYKQE
PGAGLQLLTYIFSNMDMKQDQRLTVLLNKKDKHLSLRIADTQTGDSAIYFCAGSGGGTDKLI
FGTGTRLQVFPNIQNPEPAVYQLKDPRSQDSTLCLFTDFDSQINVPKTMESGTFITDKCVLDM
KAMDSKSNGAIAWSNQTSFTCQDIFKETNATYPSSDVPCDATLTEKSFETDMNLNFQNLLVIV
LRILLLKVAGFNLLMTLRLWSS (SEQ ID NO:23)

MKTFAGFSFLFLWLQLDCMSRGEDVEQSLFLSVREGDSSVINCTYTDSSSTYLYWYKQE
PGAGLQLLTYIFSFMDMKQDQRLTVLLNKKDKHLSLRIADTQTGDSAIYFCAGSGGGTDKLIF
GTGTRLQVFPNIQNPEPAVYQLKDPRSQDSTLCLFTDFDSQINVPKTMESGTFITDKCVLDMK
AMDSKSNGAIAWSNQTSFTCQDIFKETNATYPSSDVPCDATLTEKSFETDMNLNFQNLLVIVL
RILLLKVAGFNLLMTLRLWSS (SEQ ID NO:78)

MLLLLLLLGPGSGLGAVVSQHPSWVICKSGTSVKIECRSLDFQATTMFWYRQFPKQSLM
LMATSNEGSKATYEQGVEKDKFLINHASLTLSTLTVTSAHPEDSSFYICSAREPGQGPYEQYF
GPGTRLTVTEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYQQGVLSATILYEILLGKATLYAVLVSTLVVMAMVKRKNSSR
(SEQ ID NO:70)

In some embodiments, the modified TCR comprises a TCR α chain having an amino acid sequence selected from any of SEQ ID NOs: 20-23, 78, 99, 102, 105, 108, 111, 114, 117, 120, 123, 126, 129, 134, 138, 144, 148, 151, 156, 160, 167, 169, 172, 174, or an amino acid sequence with at least 80%, at least 85%, at least 90%, at least 95%, or at least 99% sequence identity with any of SEQ ID NOs: 20-23, 78, 99, 102, 105, 108, 111, 114, 117, 120, 123, 126, 129, 134, 138, 144, 148, 151, 156, 160, 167, 169, 172, 174; and/or a TCR β chain having an amino acid sequence selected from any of SEQ ID NOs: 3-19, 81, 84, 87, 90, 93, 96, 135, 139, 145, 157, 161, 164, 176, or an amino acid sequence with at least 80%, at least 85%, at least 90%, at least 95%, or at least 99% sequence identity with any of SEQ ID NOs: 3-19, 81, 84, 87, 90, 93, 96, 135, 139, 145, 157, 161, 164, 176. In some embodiments, the modified TCR comprises a TCR α chain having an amino acid sequence selected from any of SEQ ID NOs: 20-23, 78, 99, 102, 105, 108, 111, 114, 117, 120, 123, 126, 129, 134, 138, 144, 148, 151, 156, 160, 167, 169, 172, 174; and a TCR β chain having an amino acid sequence selected from any of SEQ ID NOs: 3-19, 81, 84, 87, 90, 93, 96, 135, 139, 145, 157, 161, 164, 176.

In some embodiments, the modified TCR binds to an epitope comprising the amino acid sequence KVLEYVIKV (SEQ ID NO:2) or a complex of said epitope with an MHC molecule.

The term "epitope" usually refers to a site on an antigen, usually a (poly)peptide recognized by a binding domain. The term "binding domain" in its broadest sense refers to an "antigen binding site", i.e. a domain characterizing a molecule that binds to/interacts with a specific epitope on an antigenic target. The antigenic target may comprise a single epitope, but typically comprises at least two epitopes, and depending on the size, conformation and type of the antigen, the antigenic target may include any number of epitopes. The term "epitope" typically includes both linear epitopes and conformational epitopes. A linear epitope is a contiguous epitope contained in a primary sequence of amino acids and typically comprises at least 2 or more amino acids. A conformational epitope is formed by non-contiguous amino acids juxtaposed by folding of a target antigen, in particular, a target (poly)peptide.

The TCR of the invention advantageously (specifically) recognizes the MAGE-A1 antigen, particularly the epitope KVLEYVIKV (SEQ ID NO:2) on MAGE-A1 or a complex of the epitope with an MHC molecule, such as the complex of the epitope with HLA-A*02.

In some embodiments, the MHC molecule is HLA-A*02 type, for example, HLA-A*02:01 type, HLA-A*02:03 type, HLA-A*02:05 type, HLA-A*02:06 type, HLA-A*02:07 type, HLA-A*02:10 type, or HLA-A*02:11 type. In a preferred embodiment, the MHC molecule is HLA-A*02:01 type or HLA-A*02:05 type.

In some embodiments, the modified TCR is soluble or membrane-bound.

The TCR of the invention can be provided as soluble TCR (sTCR). Soluble TCR can be used as diagnostic tools and as a vector or "engager" for specifically targeting therapeutic agents or effector cells to cancer cells, for example, expressing antigenic targets recognized by the soluble TCR. The soluble TCR is typically a fragment or construct comprising a TCR α and/or β chain or a variable region or CDR thereof, and optionally is stabilized by a disulfide bond or covalently linked by a suitable linker. Typically, the soluble TCR does not include, for example, a transmembrane region.

The TCR of the invention can also be provided in a membrane-bound form. Typically, membrane-bound TCRs comprise a transmembrane region to anchor them to the cell membrane.

In some embodiments, the modified TCR is isolated or purified.

As used herein, the term "isolated or purified" means that the "isolated or purified" TCR has been identified, isolated and/or recovered from components of the environment in which it is produced, such that the "isolated or purified" TCR is free or substantially free of other contaminant components from the environment in which it is produced that may interfere with its therapeutic or diagnostic use. Contaminant components may include enzymes, hormones, and other proteins or non-protein solutes. Thus, the "isolated or purified" TCR may be prepared by at least one purification step that removes or substantially removes these contaminant components.

In another aspect, the present disclosure provides a nucleic acid encoding the modified TCR of the invention.

In another aspect, the present disclosure provides a vector comprising the nucleic acid of the invention.

As used herein, the term "vector" is a nucleic acid molecule used as a vehicle for transferring (exogenous) genetic materials into a host cell in which the nucleic acid molecule as a vector can, for example, be replicated and/or expressed. The term "vector" encompasses, but is not limited to, plasmids, viral vectors (including retroviral vectors, lentiviral vectors, adenoviral vectors, cowpox virus vectors, polyomavirus vectors and adenovirus-associated vectors (AAVs)), phages, phasmids, cosmids and artificial chromosomes (including BACs and YACs). The vector itself is usually a nucleotide sequence, usually comprises a DNA sequence comprising an insert (transgene) and a larger sequence that serves as the "backbone" of the vector. The engineered vector usually comprises an origin for self-replication in the host cell (if stable expression of polynucleotides is desired), a selection marker and a restriction enzyme cleavage site (e.g., a polyclonal site, MCS). The vector may additionally comprise a promoter, a genetic marker, a reporter gene, a targeting sequence, and/or a protein purification tag. As known to those skilled in the art, a large number of suitable vectors are known to those skilled in the art, and many are commercially available. Examples of suitable vectors are provided in J. Sambrook et al, Molecular Cloning: a Laboratory Manual (4th ed.), Cold Spring Harbor Laboratory, Cold Spring Harbor Laboratory Press, New York (2012), which is incorporated herein by reference in their entirety.

In some embodiments, the vector is preferably selected from the group consisting of lentiviral vectors, retroviral vectors, plasmids, and DNA vectors, mRNA vectors, transposon-based vectors, and artificial chromosomes.

In yet another aspect, the present disclosure provides a cell comprising the modified TCR, nucleic acid, or vector of the invention.

As used herein, the term "cell" refers to any type of cell capable of expressing the modified TCR of the present disclosure. The cell may be a eukaryotic cell, e.g., a vegetation (which does not have the potential to develop into a plant), animal, fungus, or algae cell, or may be a prokaryotic cell, e.g., a bacterium or protozoan cell. The cell may be a cultured cell or a primary cell, i.e. directly isolated from an organism, e.g. a human. The cell may be an adherent cell or suspension cell, i.e. cell grown in suspension. Suitable host cell is known in the art and includes, for example, a DH5α E. coli cell, Chinese hamster ovary cell, monkey VERO cell, COS cell, HEK293 cell, and the like. For the purpose of producing the modified TCR of the present disclosure, the cell is preferably a mammalian cell. Most preferably, the host cell is a human cell.

In some embodiments, the cell is selected from the group consisting of lymphocytes (e.g., T cells, NK cells), monocytes (e.g., PBMCs), and stem cells.

As used herein, the term "stem cell" is a stem cell used for expressing the TCR of the present disclosure. For example, a stem cell may be a lymphoid progenitor cell, an induced pluripotent stem cell (iPSC), or a hematopoietic stem cell (HSC). In some embodiments, the stem cell does not include embryonic stem cells obtained by destroying a human embryo, and/or does not include totipotent stem cells that are used to develop and form an individual animal. Transferring a gene to stem cells does not typically result in expression of a TCR on the surface of the cells, as stem cells does not express CD3 molecule on the surface. However, when stem cells differentiate into lymphoid precursors that migrate to thymus, expression of CD3 molecules will initiate expression of the introduced TCR molecules on the surface of thymocytes.

In some embodiments, the stem cell is a lymphoid progenitor cell or an induced pluripotent stem cell (iPSC).

In some embodiments, the cell is a T cell. The T cell may be any T cell, such as a cultured T cell, e.g., a primary T cell or a T cell from a cultured T cell line, e.g., Jurkat, SupTl, etc., or a T cell obtained from a mammal. If obtained from a mammal, the T cell may be obtained from many sources including, but not limited to, blood, bone marrow, lymph node, thymus or other tissues or fluids. The T cell may also be enriched or purified. Preferably, the T cell is a human T cell. More preferably, the T cell is a T cell isolated from human. The T cell may be any type of T cells and may be at any developmental stage, including but not limited to CD4+/CD8+ double positive T cells, CD4+ helper T cells, such as Th1 and Th2 cells, CD4+ T cells, CD8+ T cells (e.g., cytotoxic T cells), tumor infiltrating lymphocytes (TILs), memory T cells (e.g., central memory T cells and effector memory T cells), naive T cells, and the like. In some embodiments, the T cell does not express an endogenous TCR.

It is envisioned that the effector cells expressing the TCR of the present invention bind their antigen targets (preferably to an antigen epitope presented by antigen-presenting cells through HLA-A*02) with high affinity. The term "affinity" or "binding affinity" refers to the ability of T cells expressing the TCR of the present invention to respond to a given concentration of ligand in vitro, and is thought to be related to the in vivo efficacy of the TCR-expressing cells. By definition, TCR-expressing cells with high binding affinity respond to very low doses of antigen in vitro, while cells with lower binding affinity require higher doses of antigen to achieve an immune response comparable to that of cells with high binding affinity TCRs. Thus, binding affinity can be seen as a quantitative determinant of the activation threshold of TCR-expressing cells. This is determined by exposing such cells in vitro to varying doses of homologous antigen. Cells with high binding affinity TCRs respond to low antigen doses. For example, if TCR-expressing cells activate target cells with an EC50 of no more than 10⁻⁵ M (e.g., 10⁻⁵ M or lower, such as 10⁻⁶ M, 10⁻⁷ M, 10⁻⁸ M, or 10⁻⁹ M) when co-cultured with target antigen peptide-HLA-*02 expressing target cells, it is generally considered that these TCR-expressing cells bind their antigen targets with "high" affinity.

In another aspect, the present disclosure provides a method for preparing the cell of the invention, comprising a step of transducing or transfecting cells with the vector of the invention.

As used herein, the term "transfection" is a process of intentionally introducing nucleic acid molecules or polynucleotides (including vectors) into target cells. One example is RNA transfection, that is, a process of introducing RNA (e.g., in vitro transcribed RNA, ivtRNA) into host cells. This term is primarily used for non-viral methods in eukaryotic cells. The term "transduction" is commonly used to describe virus-mediated transfer of nucleic acid molecules or polynucleotides. Transfection of animal cells typically involves the opening of transient pores or "holes" in the cell membrane to allow uptake of the materials. Transfection can be performed by using calcium phosphate, electroporation, cell extrusion, or by mixing cationic lipids with the materials to produce liposomes that are fused with the cell membrane and deposit their cargos into the interior. Exemplary techniques for transfecting eukaryotic host cells include lipid vesicle-mediated uptake, heat shock-mediated uptake, calcium phosphate-mediated transfection (calcium phosphate/DNA co-precipitation), microinjection, and electroporation.

In some embodiments, the method further comprises a step of amplifying and/or activating the cells prior to or after the transduction or transfection

In another aspect, the present disclosure provides a conjugate comprising the modified TCR of the invention and an active agent conjugated or coupled to the TCR.

In some embodiments, the active agent is selected from the group consisting of detectable markers, immunostimulatory molecules, and therapeutic agents. Preferably, the detectable marker is selected from the group consisting of biotins, streptavidin, enzymes or catalytically active fragments thereof, radionuclides, nanoparticles, paramagnetic metal ions, nucleic acid probes, contrast agents, and fluorogenic, phosphorescent or chemiluminescent molecules. Preferably, the immunostimulatory molecule is selected from the group consisting of cytokines (e.g. IL-2 and IFN-y), chemokines (e.g. IL-8), platelet factors (e.g. platelet factor 4), and complement initiators. Preferably, the therapeutic agent is selected from the group consisting of immunomodulators, radioactive compounds, enzymes, chemotherapeutic agents and toxins. Other suitable therapeutic agents include small molecule cytotoxic agents, i.e. compounds having the ability to kill mammalian cells having a molecular weight of less than 700 daltons. Such compounds may also contain toxic metals having cytotoxic effects. In addition, it should be understood that these small molecule cytotoxic agents also include precursor drugs, i.e. compounds that decay or are transformed under physiological conditions to release cytotoxic agents. Examples of such agents include cisplatin, maytenin derivatives, rachelmycin, calicheamicin, docetaxel, etoposide, gemcitabine, isocyclic phosphorylamine, irinotecan, melphalan, mitoxantrone, sorfimer sodiumphotofrin II, temozolomide, topotecan, trimetreate glucuronate, auristatin E, vincristine, and doxorubicin; peptide cytotoxin, i.e., proteins or the fragments thereof having the ability to kill mammalian cells; e.g., ricin, diphtheria toxin, Pseudomonas bacterial exotoxin A, Dnase, and RNase; radionuclides, i.e., unstable isotopes of elements that emit one or more alpha or beta particles or gamma rays while decaying, e.g., iodine-131, rhenium-186, indium-111, yttrium-90, bismuth-210 and bismuth-213, actinium-225, and astatine-213; chelating agents which can be used to facilitate the binding of these radionuclides to molecules or multimers thereof; or heterologous protein domains, homologous protein domains, viral/bacterial protein domains, viral/bacterial peptides.

In yet another aspect, the present disclosure provides a bispecific or multispecific antigen-binding molecule comprising the modified TCR of the invention or an antigen-binding fragment thereof, and one or more additional antigen-binding regions that bind to one or more additional antigens, wherein the one or more additional antigen-binding regions are each independently selected from the group consisting of antigen-binding regions derived from antibodies and antigen-binding regions derived from TCRs.

In some embodiments, the one or more additional antigens are each independently selected from the group consisting of tumor-associated antigens (TAAs), tumor-specific antigens (TSAs), viral antigens, autoantigens, and immune cell surface molecules.

Examples of tumor-associated antigens (TAAs) comprise but not limited to, melanoma-associated antigens (e.g., gp100, MAGEA1, MAGEA3, MAGEA6, MAGEA4, MAGEA2, MAGEA12, MAGEA2B, MAGEA9B, MAGEA10, MAGEA11, MAGEB2, MAGEC1, MAGEC2), IGF2BP1, GNGT1, PI4K2B, CCR8, NPSR1, COX7B2, ONECUT3, SMC1B, FOXI3, GAGE2A, FBXO43, BRDT, PAGE2, GAGE13, POU5F1B, CTAG1A, endogenous reverse transcriptase antigens, 5T4, alpha-fetoprotein, BCMA, CA-125, carcinoembryonic antigen, CSPG4, ROR1, Tyrp-1, TACI, ALK, CLL-1, FLT3, mesothelin, and MUC-1.

Examples of tumor-specific antigens (TSAs) comprise but not limited to, KRAS, TP53, PIK3CA, CTNNB1, EGFR, BRAF, and GNAS.

Examples of viral antigens comprise but not limited to, HPV E6 or E7 antigens, CMV antigens, HBV antigens, EBV antigens, herpesvirus antigens, human immunodeficiency virus (HIV) antigens, influenza virus antigens, and coronavirus antigens.

As used herein, the term "autoantigen" refers to antigenic substances causing body's immune system inducing an immune response against altered self-tissue cell structures and components caused by biological, physical, or chemical factors. The release of hidden antigens can generate an immune response. Certain tissue components in the body, such as sperm, eye contents, and brain tissue, normally do not contact immune cells. When barriers are disrupted due to surgery, trauma, or infection, these components may come into contact with the immune system, leading to an immune response, such as autoimmune sympathetic ophthalmia. Changes in self-components can produce autoantigens. Changes in self-components due to physical factors (e.g., cold, heat, ionizing radiation), chemical factors (e.g., drugs), or biological factors (e.g., bacteria, viruses) can alter their antigenicity. The altered self-components stimulate the immune system, thereby causing autoimmune responses. For example, denatured IgG can stimulate the production of anti-denatured IgG antibodies (rheumatoid factors), leading to rheumatoid arthritis. Using certain drugs in clinical practice may alter the antigenicity of blood cell surfaces, causing autoimmune hemolytic anemia or granulocytopenia. Cross-reactivity caused by shared antigens can also induce an immune response. Some bacteria and viruses have similar antigenic determinants to those on normal human tissues, and autoantibodies and sensitized lymphocytes generated against these bacterial or viral antigenic determinants can cross-react with self-tissue cells, leading to autoimmune diseases. For example, various antigenic proteins of α-hemolytic streptococcus share common antigens with human myocardial endocardium and glomerular basement membrane. Immune responses triggered by Coxsackie virus infection can attack the beta cells of the pancreas, leading to diabetes.

In some embodiments, the autoantigens are lupus erythematosus, diabetes caused by autoimmune destruction of pancreatic islets, ankylosing spondylitis, and rheumatoid arthritis.

Examples of immune cell surface molecules comprise but not limited to, CD3 (e.g., CD3y, CD3δ, and CD3ε chains), CD4, CD8, CD10, CD11b, CD11c, CD14, CD16, CD18, CD25, CD32a, CD32b, CD41, CD41b, CD42a, CD42b, CD44, CD45RA, CD49, CD61, CD64, CD68, CD94, CD90, CD117, Nkp46, NKG2D, FcεRI, TCRα/β, TCR γ/δ, HLA-DR, CD28, 4-1BB (CD137), OX40 (CD134), ICOS (CD278), 2B4 (CD244), HVEM, LAG3, DAP10, DAP12, CD27, CD40, GITR, LFA-1, MyD88, CD2, CD7, LIGHT, and B7-H3. In some embodiments, the one or more additional antigens are CD3. In some embodiments, the one or more additional antigen-binding regions are derived from anti-CD3 antibodies or antigen-binding regions of anti-CD3 antibodies, preferably derived from antigen-binding regions of anti-CD3 antibody. In a preferred embodiment, the present disclosure provides a bispecific molecule comprising the modified TCR of the invention or antigen-binding fragment thereof and an antigen-binding region of an anti-CD3 antibody.

On the other hand, the present disclosure provides a composition comprising the modified TCR, nucleic acid, vector, cell, conjugate, or bispecific or multispecific antigen-binding molecule of the invention; preferably, the compositions further comprise a pharmaceutically acceptable carrier or excipient.

The term "composition" refers in particular to a composition suitable for the administration to humans. However, the term generally also encompasses a composition suitable for the administration to non-human animals. The composition and its components (i.e. active agents and optionally carriers or excipients) are preferably pharmaceutically acceptable, i.e. capable of eliciting the desired therapeutic effect without causing any undesired local or systemic effects in the recipient. The pharmaceutically acceptable compositions of the present invention may be, for example, sterile. Specifically, the term "pharmaceutically acceptable" may indicate approval by a regulatory agency or other recognized pharmacopoeia for use in animals, and more particularly in humans.

The term "excipient" includes fillers, adhesives, disintegrants, coating agents, adsorbents, anti-adhesives, flow aids, preservatives, antioxidants, flavoring agents, coloring agents, sweeteners, solvents, co-solvents, buffers, chelating agents, viscosity imparting agents, surfactants, diluents, wetting agents, carriers, diluents, preservatives, emulsifiers, stabilizers and tension regulators. It is known to those skilled in the art to select suitable excipients to prepare the composition of the present invention. Exemplary carriers for use in the composition of the present invention include saline, buffered saline, glucose, and water. Typically, the selection of a suitable excipient depends in particular on the active agent used, the disease to be treated and the desired dosage form of the composition.

Depending on the active agent employed (e.g. soluble TCR), the composition of the present disclosure may be formulated to various forms, such as solid, liquid, gaseous or lyophilized forms, and in particular may be in the form of ointments, creams, transdermal patches, gels, powders, tablets, solutions, aerosols, granules, pills, suspensions, emulsions, capsules, syrups, liquids, elixirs, infusions, tinctures or fluid extracts, or a form particularly suitable for the desired administration method. The processes of pharmaceutical production known to the present invention are shown in the 22nd edition of Remington's Pharmaceutical Sciences (Ed. MaackPublishing Co, Easton, Pa., 2012) and may include, for example, the conventional processes of mixing, lysing, granulating, sugar-coating, grinding, emulsifying, encapsulating, embedding, or lyophilizing. Compositions comprising, for example, the host cell or the soluble TCR as described herein are typically provided in liquid form and preferably comprise pharmaceutically acceptable buffering agents.

In some embodiments, the composition of the present disclosure further comprises a second therapeutic agent, preferably, the second therapeutic agent is selected from the group consisting of antibodies, chemotherapeutic agents, and small molecule drugs.

Preferred examples of the second therapeutic agent include known anti-cancer drugs such as cisplatin, maytenin derivatives, rachelmycin, calicheamicin, docetaxel, etoposide, gemcitabine, isocyclic phosphorylamine, irinotecan, melphalan, mitoxantrone, sorfimer sodiumphotofrin II, temozolomide, topotecan, trimetreate glucuronate, auristatin E, vincristine, and doxorubicin; and peptide cytotoxin, such as ricin, diphtheria toxin, Pseudomonas bacterial exotoxin A, Dnase, and RNase; radionuclides, e.g. iodine 131, rhenium 186, indium 111, iridium 90, bismuth 210 and 213, actinium 225 and astatine 213; prodrugs, e.g. antibody-directed enzyme prodrugs; immunostimulants, e.g. IL-2, chemokines e.g. IL-8, platelet factor 4; antibodies or fragments thereof, e.g. anti-CD3 antibodies or fragments thereof; complement initiators; viral/bacterial protein domains, and viral/bacterial peptides.

In another aspect, the present disclosure provides a method for treating MAGE-A1 positive cancer in a subject, comprising administering an effective amount of the modified TCR of the invention to the subject.

In another aspect, the present disclosure provides a method for treating MAGE-A1 positive cancer in a subject comprising administering an effective amount of the cell of the invention to the subject.

As used herein, the term "treatment", "treat" or "treating" includes therapeutic or prophylactic treatment in a subject in need thereof. The term "therapeutic or prophylactic treatment" includes prophylactic treatment intended to completely prevent clinical and/or pathological manifestations or therapeutic treatment intended to improve or alleviate clinical and/or pathological manifestations. Thus, the term "treatment" also includes improvement or prevention of diseases.

As used herein, the term "effective amount" refers to an amount of a therapeutic agent sufficient to achieve such treatment or prevention when administered to a subject for the treatment or prevention of a disease. The "effective amount" may vary depending on the compound, the disease and its severity, and the age, weight, etc. of the subject to be treated. A "therapeutically effective amount" refers to an effective amount for the therapeutic treatment. A "prophylactic effective amount" refers to an effective amount for prophylactic treatment.

Therapeutic efficacy and toxicity can be determined by standard procedures such as ED50 (a dose that is therapeutically effective in 50% of the population) and LD50 (a dose that is lethal to 50% of the population) in such as cell cultures or experimental animals. The dose ratio between therapeutic and toxic effects is a therapeutic index and can be expressed as a ratio of ED50/LD50. The pharmaceutical composition exhibiting a large therapeutic index is preferred.

The exact dose of the TCR or cell administered can be determined by those skilled in the art using known techniques. A suitable dose provides a sufficient amount of the active agent of the invention and is preferably therapeutically effective, i.e., sufficient to elicit, for example, a therapeutic or prophylactic response in a subject or animal within a reasonable time period. For example, the dose of the TCR of the present invention should be sufficient to bind to a cancer antigen or detect, treat, or prevent a cancer within a time period of about 2 hours or more, e.g., 12 hours to 24 hours or more from the time of administration (e.g., 1 month, 2 months, 3 months, 6 months, 12 months, 24 months, etc.). In some embodiments, the time period may even be longer. As known in the art, adjustments may be necessary depending on therapeutic purpose (such as alleviation of the acute onset of a disease), dosing route, time and frequency, time and frequency for administrating the formulation, age, weight, general health condition, gender, diet, severity of disease condition, drug combination, response sensitivity and tolerance/response to treatment.

A number of assays for determining the dose to be administered are known in the art. For the purposes of the present invention, the assays, including after administration of a given dose of T cells expressing the TCR of the present disclosure to a group of mammals (each being administered a different dose of T cells), comparing the extent of target cell lysis or IFN-γ secretion achieved by such T cells, may be used to determine a starting dose to be administered to a mammal. The extent of target cell lysis or IFN-γ secretion achieved after administration of a given dose can be determined by methods known in the art. The dose of the TCR or the cell of the present disclosure is also determined by the presence, nature and extent of any adverse side effects that may accompany the administration of the TCR or the cell of the present disclosure. Typically, the dose of the antigen binding protein or the cell of the present disclosure to be administered to each individual patient is determined by the attending physician, in consideration of a variety of factors such as age, weight, general health, diet, gender, active agent to be administered, route of administration, and severity of the treated medical condition. In some embodiments of the treatment method of the present disclosure, the number of cells administered per infusion may vary, for example, from about 1 x 10⁶ to about 1 x 10¹² cells or more. In some embodiments, less than 1x 10⁶ cells may be administered.

It should be recognized that treatment may require a single administration or multiple administrations of a therapeutically effective dose of the active agent of the invention. For example, depending on the formulation, half-life and clearance of a particular composition, some compositions may be administered every 3 to 4 days, weekly, or every two weeks, or administered once over a period of one month.

The composition of the present disclosure may be suitable for the administration by a variety of routes. Typically, administration is accomplished parenterally. Parenteral delivery methods include topical, intra-arterial, intramuscular, subcutaneous, intramedullary, intrathecal, intracardiac, intravenous, intraperitoneal, intrauterine, intravaginal, sublingual, or intranasal administration.

The terms "subject" or "individual" or "animal" or "patient" are used interchangeably herein and refer to any subject in need of treatment, in particular mammalian subjects. In general, mammalian subjects include humans, non-human primates, dogs, cats, guinea pigs, rabbits, rats, mice, horses, cows, dairy cattle, etc. However, it is readily understood that it is particularly contemplated that the TCRs, nucleic acids, vectors, host cells, and pharmaceutical compositions provided herein are used to treat human subjects, particularly those are HLA-A*02 positive, such as HLA-A*02:01, HLA-A*02:03, HLA-A*02:05, HLA-A*02:06, HLA-A*02:07, HLA-A*02:10 or HLA-A*02:11 positive, preferably those are HLA-A*02:01 or HLA-A*02:05.

In some embodiments of the therapeutic methods of the present disclosure, the cells are autologous or allogeneic to the subject.

In some embodiments, the method comprises the steps of: (i) isolating a sample comprising cells from the subject; (ii) transducing or transfecting the cells with the vector of the invention; and (iii) administering the cells obtained in step (ii) to the subject. In some embodiments, the method further comprises a step of knocking out the endogenous TCR in the cells after step (i) and before step (ii).

In some embodiments, the MAGE-A1 positive cancer is selected from the group consisting of melanoma, lung cancer, colorectal cancer, cervical cancer, breast cancer, liver cancer (such as hepatocellular carcinoma), gastric cancer, esophageal cancer, bladder cancer (such as urothelial carcinoma of the bladder), ovarian cancer (such as serous cystadenocarcinoma of the ovary), head and neck cancer (such as squamous cell carcinoma of the head and neck), uterine cancer, endometrial cancer, cholangiocarcinoma, prostate cancer, adrenocortical carcinoma, mesothelioma, sarcoma, pheochromocytoma, paraganglioma, and thymic carcinoma.

In some embodiments, the method further comprises administering a second therapeutic agent, preferably second therapeutic agent is selected from the group consisting of antibodies, chemotherapeutic agents, and small molecule drugs. Preferred examples of the second therapeutic agents are as described above.

In some embodiments of the therapeutic methods of the present disclosure, the subject has an HLA-A*02 allele, such as HLA-A*02:01, HLA-A*02:03, HLA-A*02:05, HLA-A*02:06, HLA-A*02:07, HLA-A*02:10 or HLA-A*02:11 allele, preferably HLA-A*02:01 or HLA-A*02:05 allele.

In another aspect, the present disclosure provides a method for detecting (e.g., diagnosing) MAGE-A1 positive cancer in a subject, which comprise (i) contacting a sample obtained from the subject with the TCR, cell, or conjugate of the invention; and (ii) detecting the presence of MAGE-A1 antigen in the sample, where the presence of the MAGE-A1 antigen indicates MAGE-A1 positive cancer. MAGE-A1 positive cancer is preferably selected from the group consisting of melanoma, lung cancer, colorectal cancer, cervical cancer, breast cancer, liver cancer (such as hepatocellular carcinoma), gastric cancer, esophageal cancer, bladder cancer (such as urothelial carcinoma of the bladder), ovarian cancer (such as serous cystadenocarcinoma of the ovary), head and neck cancer (such as squamous cell carcinoma of the head and neck), uterine cancer, endometrial cancer, cholangiocarcinoma, prostate cancer, adrenocortical carcinoma, mesothelioma, sarcoma, pheochromocytoma, paraganglioma, and thymic carcinoma.

In some embodiments, the sample obtained from the subject may be a blood sample, a urine sample, a tissue sample, or a cell sample. In some embodiments, the method is performed in vitro. In some embodiments, the method comprises (i) contacting the sample obtained from the subject with the conjugate of the present disclosure, wherein the conjugate comprises a detectable marker; and (ii) detecting the presence of a MAGE-A1 antigen in the sample by detecting the detectable marker. Examples of the detectable marker include, but are not limited to, biotins, streptavidin, enzymes or catalytically active fragments thereof, radionuclides, nanoparticles, paramagnetic metal ions, nucleic acid probes, contrast agents, and fluorogenic, phosphorescent, or chemiluminescent molecules; preferably enzymes or catalytically active fragments thereof, radionuclides, fluorogenic, phosphorescent, or chemiluminescent molecules.

In another aspect, the present disclosure provides a kit comprising the TCR or conjugate of the invention for detecting the presence of positive epitopes in a sample to be tested, where the epitope comprises KVLEYVIKV (SEQ ID NO:2).

In some embodiments, the kit is for detecting (e.g., diagnosing) MAGE-A1 positive cancer in a subject and comprises the TCR or conjugate of the invention. MAGE-A1 positive cancer is preferably selected from the group consisting of melanoma, lung cancer, colorectal cancer, cervical cancer, breast cancer, liver cancer (such as hepatocellular carcinoma), gastric cancer, esophageal cancer, bladder cancer (such as urothelial carcinoma of the bladder), ovarian cancer (such as serous cystadenocarcinoma of the ovary), head and neck cancer (such as squamous cell carcinoma of the head and neck), uterine cancer, endometrial cancer, cholangiocarcinoma, prostate cancer, adrenocortical carcinoma, mesothelioma, sarcoma, pheochromocytoma, paraganglioma, and thymic carcinoma.

In some embodiments, the conjugate comprises a detectable marker. Examples of the detectable marker include, but are not limited to, biotins, streptavidin, enzymes or catalytically active fragments thereof, radionuclides, nanoparticles, paramagnetic metal ions, nucleic acid probes, contrast agents, and fluorogenic, phosphorescent, or chemiluminescent molecules; preferably enzymes or catalytically active fragments thereof, radionuclides, fluorogenic, phosphorescent, or chemiluminescent molecules. In some embodiments, the kit may further comprise instructions on how to use the kit.

In another aspect, the present disclosure provides a usage of the modified TCR, the nucleic acid, the vector, the cell, the conjugate, the bispecific antigen-binding molecule or the multispecific antigen-binding molecule, or the composition of the invention in the manufacture of medicaments for treating MAGE-A1 positive cancer in a subject.

In another aspect, the present disclosure provides the modified TCR, the nucleic acid, the vector, the cell, the conjugate, the bispecific antigen-binding molecule or the multispecific antigen-binding molecule, or the composition of the invention, for treating MAGE-A1 positive cancer in a subject.

In another aspect, the disclosure provides a usage of the modified TCR or the conjugate of the invention in the manufacture of kits for detecting the presence of positive epitopes in a test sample, where the epitope comprises KVLEYVIKV (SEQ ID NO:2).

In some embodiments, the present disclosure provides a usage of the modified TCR or the conjugate of the invention in the manufacture of kits for detecting (e.g., diagnosing) MAGE-A1 positive cancer in a subject.

In another aspect, the present disclosure provides the modified TCR or the conjugate of the invention for detecting the presence of positive epitopes in a sample to be tested, where the epitope comprises KVLEYVIKV (SEQ ID NO:2), such as for detecting (e.g., diagnosing) MAGE-A1 positive cancer in a subject.

In some embodiments of the present disclosed usage, the MAGE-A1 positive cancer is preferably selected from the group consisting of melanoma, lung cancer, colorectal cancer, cervical cancer, breast cancer, liver cancer (such as hepatocellular carcinoma), gastric cancer, esophageal cancer, bladder cancer (such as urothelial carcinoma of the bladder), ovarian cancer (such as serous cystadenocarcinoma of the ovary), head and neck cancer (such as squamous cell carcinoma of the head and neck), uterine cancer, endometrial cancer, cholangiocarcinoma, prostate cancer, adrenocortical carcinoma, mesothelioma, sarcoma, pheochromocytoma, paraganglioma, and thymic carcinoma.

In some embodiments, the subject has an HLA-A*02 allele, such as HLA-A*02:01, HLA-A*02:03, HLA-A*02:05, HLA-A*02:06, HLA-A*02:07, HLA-A*02:10 or HLA-A*02:11 allele, preferably HLA-A*02:01 or HLA-A*02:05 allele.

The following examples further describe the invention, and the advantages and features of the invention will become clearer with the description. It should be understood that these examples are provided for illustrative purposes and are not intended to limit the scope of the invention. Experimental methods not specified with specific conditions in the following examples are conducted according to conventional conditions in the field, for example, as described in Sambrook and Russeii et al, Molecular Cloning: A Laboratory Manual (Third Edition) (2001), CSHL Press, or according to the conditions recommended by the manufacturer. Unless otherwise specified, the experimental materials and reagents used in the following examples are commercially available.

### Example 1. Screening for Affinity-Enhanced Substituted TCRs

The inventors of the present disclosure used the human-derived TCR ht27 sequence as a template and performed in vitro substitutions of the complementary determining regions (CDRs) of ht27, then substituted clones with high-affinity were obtained through in vitro positive screening of the substituted TCRs. Using this method, the selected optimal TCR sequences comprised amino acid substitutions at the following positions in the ht27 β chain: Gln29, Ala36, Thr37, Thr38 in CDR1β; Ser56, Asn57, Glu58, Gly59, Ser63, Lys64, and Ala65 in CDR2β; and Ser105, Glu108, Pro109, Gly110, Gln111, Gly112, Pro113, Tyr114, and Glu115 in CDR3β. Specific amino acid substitutions comprised Q29P, A36I, T37E, T38R, S56T, N57D, N57E, N57H, N57K, N57Q, N57R, N57S, N57T, E58A, E58F, E58I, E58L, E58M, E58P, E58V, E58Y, E58W, G59D, G59E, G59H, G59K, G59Q, G59R, G59S, G59T, G59P, G59L, S63D, S63E, S63H, S63K, S63Q, S63R, S63T, K64M, K64C, K64W, K64T, K64P, K64A, A65F, A65I, A65L, A65M, A65P, A65V, A65W, A65Y, S105L, S105N, S105H, S105Q, S105K, S105R, E108D, E108F, E108T, E108I, E108K, E108P, Q111N, Q111K, Q111S, Q111G, Q111A, Q111V, Q111E, Q111L, Q111H, G112E, P113H, EPGQGP at positions 108-113 substituted by VNDGT, Y114I, Y114F, Y114K, Y114D, Y114L, Y114W, Y114N, Y114P, Y114S, E115I, etc. The selected optimal TCR sequences comprised amino acid substitutions at the following positions in the ht27 α chain: Asp27, Ser28, Ser29, Ser36, Thr37, Tyr38 in CDR1α; and Ile56, Phe57, Ser58, Asn59, Met63, Asp64 in CDR2α. Specific amino acid substitutions comprised S28Y, S28P, S29Y, S29T, S29A, S29F, S29I, S29L, S29M, S29P, S29V, S29W, S29N, S36M, S36L, S36Q, S36I, S36F, S36P, S36V, S36D, T37I, Y38L, 156T, 156G, F57W, F57T, S58G, S58I, S58N, S58A, S58P, S58W, N59L, N59F, N59D, N59E, N59H, N59K, N59Q, N59R, N59S, N59T, M63D, M63Q, M63T, M63E, D64T, D64Y, D64S, etc. The selected optimal TCR sequences comprised amino acid substitutions at position Thr26 in the α chain framework region 1 (FR1α), such as T26A, T26V, etc.

The three CDR regions of both TCR α and β chains bind to different sites of the MHC-peptide complex and interact with the MHC-peptide complex in a relatively independent manner. For the mutations in the CDR regions described above, whether single mutations or combinations of mutations, the TCR affinity is effectively increased relative to the wild-type TCR. Some combinations of mutations even produce a synergistic effect, achieving higher affinity.

Table 1 below shows representative substituted TCRs. Table 1 shows the amino acid substitution regions and types of the representative substituted TCRs of the invention relative to the wild-type ht27 TCR.

**Table 1. Representative Substituted TCRs**

| TCR Name | Substitution Regions | | Substitution Types |
|---|---|---|---|
| ht27WT | NA | | NA |
| ht27M1001 | Vβ | CDR2 | N57D E58Y G59Q S63T K64C A65V |
| ht27M1002 | | CDR3 | Q111G |
| ht27M1003 | | CDR3 | Q111S |
| ht27M1004 | | CDR3 | Y114F |
| ht27M1005 | | CDR3 | Y114L |
| ht27M1006 | | CDR3 | Y114W |
| ht27M 1009 | | CDR2, CDR3 | N57D E58Y G59Q S63T K64C A65V Y114F |
| ht27M1010 | | CDR2, CDR3 | N57D E58Y G59Q S63T K64C A65V Y114L |
| ht27M1011 | | CDR2, CDR3 | N57D E58Y G59Q S63T K64C A65V Y114W |
| ht27M1012 | | CDR3 | Q111G Y114F |
| ht27M1013 | | CDR3 | Q111S Y114F |
| ht27M1018 | | CDR2, CDR3 | N57D E58Y G59Q S63T K64C A65V Q111G Y114F |
| ht27M1019 | | CDR2, CDR3 | N57D E58Y G59Q S63T K64C A65V Q111S Y114F |
| ht27M1128 | | CDR3 | S105L |
| ht27M1166 | | CDR3 | S105Q |
| ht27M1169 | | CDR3 | S105N |
| ht27M1170 | | CDR3 | S105H |
| ht27M1290 | | CDR3 | EPGQGP at positions 108 to 113 substituted with VNDGT |
| ht27M1387 | | CDR1 | Q29P A36I T37E T38R |
| ht27M1398 | | CDR2 | S56T E58L G59P |
| ht27M1401 | | CDR2 | S56T N57Q E58P G59L |
| ht27M1457 | | CDR2 | S56T N57D E58Y |
| ht27M1557 | | CDR3 | G112E P113H Y114L E115I |
| ht27M1226 | Vα | CDR1 | S29L |
| ht27M1240 | | CDR1 | S36M |
| ht27M1243 | | CDR1 | S36L |
| ht27M1245 | | CDR1 | S36Q |
| ht27M1285 | | CDR2 | N59F |
| ht27M1374 | | CDR1 | S29T S36D T37I Y38L |
| ht27M1378 | | FR1, CDR1 | T26A S28Y S29L |
| ht27M1381 | | FR1, CDR1 | T26V S28P S29Y |
| ht27M1360 | | CDR2 | S58G N59F M63D D64T |
| ht27M1362 | | CDR2 | S58I N59F M63Q D64Y |
| ht27M1363 | | CDR2 | S58N N59T M63Q |
| ht27M1365 | | CDR2 | N59F M63T D64Y |
| ht27M1369 | | CDR2 | S58A N59F M63E D64S |
| ht27M1414 | | CDR2 | I56T F57W S58P N59H |
| ht27M1418 | | CDR2 | I56G F57T S58W N59L |
| ht27M1436 | | CDR2 | I56T S58W N59F |

The inventors further combined mutations in different CDR regions aforementioned to screen mutated TCRs with affinity levels up to the pM range. The amino acid substitutions in the screened TCR sequences comprise the following positions in the ht27 β chain: Asp27, Phe28, Gln29, Ala36, Thr37, Thr38 in CDR1β; Ser56, Asn57, Glu58, Gly59, Ser63, Ly4, Ala65 in CDR2β; and Ser105, Ala106, Arg107, Glu108, Pro109, Gly110, Gln111, Gly112, Pro113, Tyr114, Glu115 in CDR3β; and in the ht27 α chain: Asp27, Ser28, Ser29, Ser36, Thr37, Tyr38 in CDR1α; Ile56, Phe57, Ser58, Asn59, Met63, Asp64, Met65 in CDR2α, and further comprise amino acid substitution at position Thr26 in FR1α. Representative substituted TCRs are as shown in Table 2 below. Table 2 shows the amino acid substitution regions and types of the representative substituted TCRs of the invention relative to the wild-type ht27 TCR.

**Table 2. Representative Substituted TCRs**

| TCR Name | Substitution Regions | Substitution Types |
|---|---|---|
| ht27WT | NA | NA |
| A1A2-M01-612 | CDR1β, CDR3β | Q29E G110W Q111D G112P P113W |
| | FR1α, CDR1α, CDR2α | T26V S28P S29Y N59F |
| A1A2-M01-613 | CDR2β, CDR3β | S56T N57D E58Y Y114W |
| | CDR1α, CDR2α | S36L N59F |
| A1A2-M01-614 | CDR1β, CDR2β, CDR3β | Q29E S56T N57D E58Y Y114W |
| | FR1α, CDR1α, CDR2α | T26S S28M S29L S58I N59F M63Q D64A |
| A1A2-M01-615 | CDR1β, CDR2β, CDR3β | Q29E S56T N57D E58Y Y114W |
| | FR1α, CDR1α, CDR2α | T26I D27C S28A S29F S58A N59F M63E D64S |
| A1A2-M01-616 | CDR1β, CDR2β, CDR3β | Q29E S56T N57D E58Y Y114W |
| | CDR1α, CDR2α | S36Q S58A N59F D64Q |
| A1A2-M01-617 | CDR1β, CDR2β, CDR3β | Q29E S63D K64Q Y114W |
| | FR1α, CDR1α, CDR2α | T26P S28M S29F S58A N59F D64Q |
| A1A2-M01-619 | CDR1β, CDR2β | Q29E S56T N57D E58Y |
| | CDR1α, CDR2α | S36M N59F |
| A1A2-M01-620 | CDR1β, CDR2β, CDR3β | Q29E S56T N57D E58Y G112A P113W Y114S E115D |
| | CDR1α | S36Q |
| A1A2-M01-621 | CDR1β, CDR2β, CDR3β | Q29E S56T N57D E58Y Y114W |
| | CDR1α, CDR2α | S29E S36F S58A N59F M63E D64S |
| A1A2-M01-622 | CDR1β, CDR2β, CDR3β | Q29E S56T N57D E58Y Y114W |
| | CDR2α | S58A N59F M63E D64S |
| A1A2-M01-623 | CDR1β, CDR2β, CDR3β | Q29E S56T N57D E58Y Y114W |
| | CDR1α, CDR2α | S36Q S58I N59F M63Q D64A |
| A1A2-M01-624 | CDR1β, CDR2β, CDR3β | Q29E S56T N57D E58Y Y114W |
| | FR1α, CDR1α, CDR2α | T26Q D27Y S28G S29M N59F |
| A1A2-M01-625 | CDR1β, CDR2β, CDR3β | Q29E S56T N57D E58Y Y114W |
| | CDR1α, CDR2α | S36M S58A N59F M63E D64S |
| A1A2-M01-626 | CDR1β, CDR2β, CDR3β | Q29P A36I T37E T38R S56T N57D E58Y Y114F |
| | FR1α, CDR1α, CDR2α | T26P S28M S29F S58A N59F D64Q |

The amino acid sequences of the CDRs and the variable regions of the α and β chains of the wild-type ht27 TCR are as shown in Table 3 below. The amino acid sequences of the CDRs and the variable regions of the α and β chains of the representative substituted TCRs of the present invention are as shown in Table 4 below.

**Table 3. Amino Acid Sequences of the CDRs and the Variable Regions of the α and β Chains of the ht27 TCR**

| | |
|---|---|
| CDR1α | DSSSTY (SEQ ID NO:27) |
| CDR2α | IFSNMDM (SEQ ID NO:28) |
| CDR3α | AGSGGGTDKLI (SEQ ID NO:29) |
| CDR1β | DFQATT (SEQ ID NO:24) |
| CDR2β | SNEGSKA (SEQ ID NO:25) |
| CDR3β | SAREPGQGPYEQY (SEQ ID NO:26) |
| Vα | RGEDVEQSLFLSVREGDSSVINCTYTDSSSTYLYWYKQEPGAGLQLLTYIFSNMD MKQDQRLTVLLNKKDKHLSLRIADTQTGDSAIYFCAGSGGGTDKLIFGTGTRLQV FPN (SEQ ID NO:46) |
| Vβ | GAVVSQHPSWVICKSGTSVKIECRSLDFQATTMFWYRQFPKQSLMLMATSNEGS KATYEQGVEKDKFLINHASLTLSTLTVTSAHPEDSSFYICSAREPGQGPYEQYFGP GTRLTVTE (SEQ ID NO:47) |

**Table 4. Amino Acid Sequences of the CDRs and the Variable Regions of the α and β Chains of the Representative Substituted TCRs**

| | | |
|---|---|---|
| ht27M 1001 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SDYQTCV (SEQ ID NO:30) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSDYQTCVTYEQGVEKDKFLINHASLTLSTLTVTSAHPEDSSFYICS AREPGQGPYEQYFGPGTRLTVTE (SEQ ID NO:48) |
| ht27M1002 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGGGPYEQY (SEQ ID NO:31) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGGGPYEQYFGPGTRLTVTE (SEQ ID NO:49) |
| ht27M1003 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGSGPYEQY (SEQ ID NO:32) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGSGPYEQYFGPGTRLTVTE (SEQ ID NO:50) |
| ht27M1004 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPFEQY (SEQ ID NO:33) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPFEQYFGPGTRLTVTE (SEQ ID NO:51) |
| ht27M1005 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPLEQY (SEQ ID NO:34) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPLEQYFGPGTRLTVTE (SEQ ID NO:52) |
| ht27M1006 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPWEQY (SEQ ID NO:35) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPWEQYFGPGTRLTVTE (SEQ ID NO:53) |
| ht27M1009 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SDYQTCV (SEQ ID NO:30) |
| | CDR3β | SAREPGQGPFEQY (SEQ ID NO:33) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSDYQTCVTYEQGVEKDKFLINHASLTLSTLTVTSAHPEDSSFYICS AREPGQGPFEQYFGPGTRLTVTE (SEQ ID NO:54) |
| ht27M1010 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SDYQTCV (SEQ ID NO:30) |
| | CDR3β | SAREPGQGPLEQY (SEQ ID NO:34) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSDYQTCVTYEQGVEKDKFLINHASLTLSTLTVTSAHPEDSSFYICS AREPGQGPLEQYFGPGTRLTVTE (SEQ ID NO:55) |
| ht27M1011 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SDYQTCV (SEQ ID NO:30) |
| | CDR3β | SAREPGQGPWEQY (SEQ ID NO:35) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSDYQTCVTYEQGVEKDKFLINHASLTLSTLTVTSAHPEDSSFYICS AREPGQGPWEQYFGPGTRLTVTE (SEQ ID NO:56) |
| ht27M1012 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGGGPFEQY (SEQ ID NO:36) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGGGPFEQYFGPGTRLTVTE (SEQ ID NO:57) |
| ht27M 1013 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGSGPFEQY(SEQ ID NO:37) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGSGPFEQYFGPGTRLTVTE (SEQ ID NO:58) |
| ht27M1018 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SDYQTCV (SEQ ID NO:30) |
| | CDR3β | SAREPGGGPFEQY (SEQ ID NO:36) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSDYQTCVTYEQGVEKDKFLINHASLTLSTLTVTSAHPEDSSFYICS AREPGGGPFEQYFGPGTRLTVTE (SEQ ID NO:59) |
| ht27M1019 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SDYQTCV (SEQ ID NO:30) |
| | CDR3β | SAREPGSGPFEQY(SEQ ID NO:37) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSDYQTCVTYEQGVEKDKFLINHASLTLSTLTVTSAHPEDSSFYICS AREPGSGPFEQYFGPGTRLTVTE (SEQ ID NO:60) |
| ht27M1128 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | LAREPGQGPYEQY (SEQ ID NO:38) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICLA REPGQGPYEQYFGPGTRLTVTE (SEQ ID NO:61) |
| ht27M1166 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | QAREPGQGPYEQY(SEQ ID NO:39) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICQ AREPGQGPYEQYFGPGTRLTVTE (SEQ ID NO:62) |
| ht27M1169 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | NAREPGQGPYEQY (SEQ ID NO:40) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICNA REPGQGPYEQYFGPGTRLTVTE (SEQ ID NO:63) |
| ht27M1170 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | HAREPGQGPYEQY (SEQ ID NO:41) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICH AREPGQGPYEQYFGPGTRLTVTE (SEQ ID NO:64) |
| ht27M1226 | CDR1α | DSLSTY (SEQ ID NO:42) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSLSTYLYWYKQEPGAGLQLL TYIFSNMDMKQDQRLTVLLNKKDKHLSLRIADTQTGDSAIYFCAGSG GGTDKLIFGTGTRLQVFPN (SEQ ID NO:65) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1240 | CDR1α | DSSMTY (SEQ ID NO:43) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSSMTYLYWYKQEPGAGLQLL TYIFSNMDMKQDQRLTVLLNKKDKHLSLRIADTQTGDSAIYFCAGSG GGTDKLIFGTGTRLQVFPN (SEQ ID NO:66) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1243 | CDR1α | DSSLTY (SEQ ID NO:44) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSSLTYLYWYKQEPGAGLQLL TYIFSNMDMKQDQRLTVLLNKKDKHLSLRIADTQTGDSAIYFCAGSG GGTDKLIFGTGTRLQVFPN (SEQ ID NO:67) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1245 | CDR1α | DSSQTY (SEQ ID NO:45) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSSQTYLYWYKQEPGAGLQLL TYIFSNMDMKQDQRLTVLLNKKDKHLSLRIADTQTGDSAIYFCAGSG GGTDKLIFGTGTRLQVFPN (SEQ ID NO:68) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1285 | CDR1α | DSSSTY(SEQ ID NO:27) |
| | CDR2α | IFSFMDM (SEQ ID NO:76) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YIFSFMDMKQDQRLTVLLNKKDKHLSLRIADTQTGDSAIYFCAGSGG GTDKLIFGTGTRLQVFPN (SEQ ID NO:77) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1290 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SARVNDGTYEQY (SEQ ID NO:79) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA RVNDGTYEQYFGPGTRLTVTE (SEQ ID NO:80) |
| ht27M1387 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFPIER (SEQ ID NO:82) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFPIERMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:83) |
| ht27M1398 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | TNLPSKA (SEQ ID NO:85) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATTNLPSKATYEQGVEKDKFLINHASLTLSTLTVTSAHPEDSSFYICSA REPGQGPYEQYFGPGTRLTVTE (SEQ ID NO:86) |
| ht27M1401 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | TQPLSKA (SEQ ID NO:88) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATTQPLSKATYEQGVEKDKFLINHASLTLSTLTVTSAHPEDSSFYICSA REPGQGPYEQYFGPGTRLTVTE (SEQ ID NO:89) |
| ht27M1457 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | TDYGSKA (SEQ ID NO:91) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATTDYGSKATYEQGVEKDKFLINHASLTLSTLTVTSAHPEDSSFYICS AREPGQGPYEQYFGPGTRLTVTE (SEQ ID NO:92) |
| ht27M1557 | CDR1α | DSSSTY (SEQ ID NO:27) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQEHLIQY (SEQ ID NO:94) |
| | Vα | RGEDVEQSLFLSVREGDSSVINCTYTDSSSTYLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:46) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQEHLIQYFGPGTRLTVTE (SEQ ID NO:95) |
| ht27M1374 | CDR1α | DSTDIL (SEQ ID NO:97) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSTDILLYWYKQEPGAGLQLLT YIFSNMDMKQDQRLTVLLNKKDKHLSLRIADTQTGDSAIYFCAGSGG GTDKLIFGTGTRLQVFPN (SEQ ID NO:98) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1378 | CDR1α | DYLSTY (SEQ ID NO:100) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYADYLSTYLYWYKQEPGAGLQLL TYIFSNMDMKQDQRLTVLLNKKDKHLSLRIADTQTGDSAIYFCAGSG GGTDKLIFGTGTRLQVFPN (SEQ ID NO:101) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1381 | CDR1α | DPYSTY (SEQ ID NO:103) |
| | CDR2α | IFSNMDM (SEQ ID NO:28) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYVDPYSTYLYWYKQEPGAGLQLL TYIFSNMDMKQDQRLTVLLNKKDKHLSLRIADTQTGDSAIYFCAGSG GGTDKLIFGTGTRLQVFPN (SEQ ID NO:104) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1360 | CDR1α | DSSSTY (SEQ ID NO:27) |
| | CDR2α | IFGFDTM (SEQ ID NO:106) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YIFGFDTMKQDQRLTVLLNKKDKHLSLRIADTQTGDSAIYFCAGSGG GTDKLIFGTGTRLQVFPN (SEQ ID NO:107) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1362 | CDR1α | DSSSTY (SEQ ID NO:27) |
| | CDR2α | IFIFQYM (SEQ ID NO:109) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YIFIFQYMKQDQRLTVLLNKKDKHLSLRIADTQTGDSAIYFCAGSGG GTDKLIFGTGTRLQVFPN (SEQ ID NO:110) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1363 | CDR1α | DSSSTY (SEQ ID NO:27) |
| | CDR2α | IFNTQDM (SEQ ID NO:112) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YIFNTQDMKQDQRLTVLLNKKDKHLSLRIADTQTGDSAIYFCAGSGG GTDKLIFGTGTRLQVFPN (SEQ ID NO:113) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1365 | CDR1α | DSSSTY (SEQ ID NO:27) |
| | CDR2α | IFSFTYM (SEQ ID NO:115) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YIFSFTYMKQDQRLTVLLNKKDKHLSLRIADTQTGDSAIYFCAGSGG GTDKLIFGTGTRLQVFPN (SEQ ID NO:116) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1369 | CDR1α | DSSSTY (SEQ ID NO:27) |
| | CDR2α | IFAFESM (SEQ ID NO:118) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YIFAFESMKQDQRLTVLLNKKDKHLSLRIADTQTGDSAIYFCAGSGG GTDKLIFGTGTRLQVFPN (SEQ ID NO:119) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1414 | CDR1α | DSSSTY (SEQ ID NO:27) |
| | CDR2α | TWPHMDM (SEQ ID NO:121) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YTWPHMDMKQDQRLTVLLNKKDKHLSLRIADTQTGDSAIYFCAGSG GGTDKLIFGTGTRLQVFPN (SEQ ID NO:122) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1418 | CDR1α | DSSSTY (SEQ ID NO:27) |
| | CDR2α | GTWLMDM (SEQ ID NO:124) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YGTWLMDMKQDQRLTVLLNKKDKHLSLRIADTQTGDSAIYFCAGSG GGTDKLIFGTGTRLQVFPN (SEQ ID NO:125) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| ht27M1436 | CDR1α | DSSSTY (SEQ ID NO:27) |
| | CDR2α | TFWFMDM (SEQ ID NO:127) |
| | CDR3α | AGSGGGTDKLI (SEQ ID NO:29) |
| | CDR1β | DFQATT (SEQ ID NO:24) |
| | CDR2β | SNEGSKA (SEQ ID NO:25) |
| | CDR3β | SAREPGQGPYEQY (SEQ ID NO:26) |
| | Vα | RGEDVEQSLFLSVREGDSSVINCTYTDSSSTYLYWYKQEPGAGLQLLT YTFWFMDMKQDQRLTVLLNKKDKHLSLRIADTQTGDSAIYFCAGSG GGTDKLIFGTGTRLQVFPN (SEQ ID NO:128) |
| | Vβ | GAVVSQHPSWVICKSGTSVKIECRSLDFQATTMFWYRQFPKQSLMLM ATSNEGSKATYEQGVEKDKFLINHASLTLSTLTVTSAHPEDSSFYICSA REPGQGPYEQYFGPGTRLTVTE (SEQ ID NO:47) |
| A1A2-M01-612 | CDR1α | DPYSTY (SEQ ID NO:103) |
| | CDR2α | IFSFMDM (SEQ ID NO:76) |
| | CDR3α | AGSGGGTDKLI (SEQ ID NO:29) |
| | CDR1β | DFEATT (SEQ ID NO:130) |
| | CDR2β | SNEGSKA (SEQ ID NO:25) |
| | CDR3β | SAREPWDPWYEQY (SEQ ID NO:131) |
| | Vα | RGEDVEQSLFLSVREGDSSVINCTYVDPYSTYLYWYKQEPGAGLQLL TYIFSFMDMKQDQRLTVLLNKKDKHLSLRIADTQTGDSAIYFCAGSG GGTDKLIFGTGTRLQVFPN (SEQ ID NO:132) |
| | Vβ | GAVVSQHPSWVICKSGTSVKIECRSLDFEATTMFWYRQFPKQSLMLM ATSNEGSKATYEQGVEKDKFLINHASLTLSTLTVTSAHPEDSSFYICSA REPWDPWYEQYFGPGTRLTVTE (SEQ ID NO:133) |
| A1A2-M01-613 | CDR1α | DSSLTY (SEQ ID NO:44) |
| | CDR2α | IFSFMDM (SEQ ID NO:76) |
| | CDR3α | AGSGGGTDKLI (SEQ ID NO:29) |
| | CDR1β | DFQATT (SEQ ID NO:24) |
| | CDR2β | TDYGSKA (SEQ ID NO:91) |
| | CDR3β | SAREPGQGPWEQY (SEQ ID NO:35) |
| | Vα | RGEDVEQSLFLSVREGDSSVINCTYTDSSLTYLYWYKQEPGAGLQLL TYIFSFMDMKQDQRLTVLLNKKDKHLSLRIADTQTGDSAIYFCAGSG GGTDKLIFGTGTRLQVFPN (SEQ ID NO:136) |
| | Vβ | GAVVSQHPSWVICKSGTSVKIECRSLDFQATTMFWYRQFPKQSLMLM ATTDYGSKATYEQGVEKDKFLINHASLTLSTLTVTSAHPEDSSFYICS AREPGQGPWEQYFGPGTRLTVTE (SEQ ID NO:137) |
| A1A2-M01-614 | CDR1α | DMLSTY (SEQ ID NO:140) |
| | CDR2α | IFIFQAM (SEQ ID NO:141) |
| | CDR3α | AGSGGGTDKLI (SEQ ID NO:29) |
| | CDR1β | DFEATT (SEQ ID NO:130) |
| | CDR2β | TDYGSKA (SEQ ID NO:91) |
| | CDR3β | SAREPGQGPWEQY (SEQ ID NO:35) |
| | Vα | RGEDVEQSLFLSVREGDSSVINCTYSDMLSTYLYWYKQEPGAGLQLL TYIFIFQAMKQDQRLTVLLNKKDKHLSLRIADTQTGDSAIYFCAGSGG GTDKLIFGTGTRLQVFPN (SEQ ID NO:142) |
| | Vβ | GAVVSQHPSWVICKSGTSVKIECRSLDFEATTMFWYRQFPKQSLMLM ATTDYGSKATYEQGVEKDKFLINHASLTLSTLTVTSAHPEDSSFYICS AREPGQGPWEQYFGPGTRLTVTE (SEQ ID NO:143) |
| A1A2-M01-615 | CDR1α | CAFSTY (SEQ ID NO:146) |
| | CDR2α | IFAFESM (SEQ ID NO:118) |
| | CDR3α | AGSGGGTDKLI (SEQ ID NO:29) |
| | CDR1β | DFEATT (SEQ ID NO:130) |
| | CDR2β | TDYGSKA (SEQ ID NO:91) |
| | CDR3β | SAREPGQGPWEQY (SEQ ID NO:35) |
| | Vα | RGEDVEQSLFLSVREGDSSVINCTYICAFSTYLYWYKQEPGAGLQLLT YIFAFESMKQDQRLTVLLNKKDKHLSLRIADTQTGDSAIYFCAGSGG GTDKLIFGTGTRLQVFPN (SEQ ID NO:147) |
| | Vβ | GAVVSQHPSWVICKSGTSVKIECRSLDFEATTMFWYRQFPKQSLMLM ATTDYGSKATYEQGVEKDKFLINHASLTLSTLTVTSAHPEDSSFYICS AREPGQGPWEQYFGPGTRLTVTE (SEQ ID NO:143) |
| A1A2-M01-616 | CDR1α | DSSQTY (SEQ ID NO:45) |
| | CDR2α | IFAFMQM (SEQ ID NO:149) |
| | CDR3α | AGSGGGTDKLI (SEQ ID NO:29) |
| | CDR1β | DFEATT (SEQ ID NO:130) |
| | CDR2β | TDYGSKA (SEQ ID NO:91) |
| | CDR3β | SAREPGQGPWEQY (SEQ ID NO:35) |
| | Vα | RGEDVEQSLFLSVREGDSSVINCTYTDSSQTYLYWYKQEPGAGLQLL TYIFAFMQMKQDQRLTVLLNKKDKHLSLRIADTQTGDSAIYFCAGSG GGTDKLIFGTGTRLQVFPN (SEQ ID NO:150) |
| | Vβ | GAVVSQHPSWVICKSGTSVKIECRSLDFEATTMFWYRQFPKQSLMLM ATTDYGSKATYEQGVEKDKFLINHASLTLSTLTVTSAHPEDSSFYICS AREPGQGPWEQYFGPGTRLTVTE (SEQ ID NO:143) |
| A1A2-M01-617 | CDR1α | DMFSTY (SEQ ID NO:152) |
| | CDR2α | IFAFMQM (SEQ ID NO:149) |
| | CDR3α | AGSGGGTDKLI (SEQ ID NO:29) |
| | CDR1β | DFEATT (SEQ ID NO:130) |
| | CDR2β | SNEGDQA (SEQ ID NO:153) |
| | CDR3β | SAREPGQGPWEQY (SEQ ID NO:35) |
| | Vα | RGEDVEQSLFLSVREGDSSVINCTYPDMFSTYLYWYKQEPGAGLQLL TYIFAFMQMKQDQRLTVLLNKKDKHLSLRIADTQTGDSAIYFCAGSG GGTDKLIFGTGTRLQVFPN (SEQ ID NO:154) |
| | Vβ | GAVVSQHPSWVICKSGTSVKIECRSLDFEATTMFWYRQFPKQSLMLM ATSNEGDQATYEQGVEKDKFLINHASLTLSTLTVTSAHPEDSSFYICS AREPGQGPWEQYFGPGTRLTVTE (SEQ ID NO:155) |
| A1A2-M01-619 | CDR1α | DSSMTY (SEQ ID NO:43) |
| | CDR2α | IFSFMDM (SEQ ID NO:76) |
| | CDR3α | AGSGGGTDKLI (SEQ ID NO:29) |
| | CDR1β | DFEATT (SEQ ID NO:130) |
| | CDR2β | TDYGSKA (SEQ ID NO:91) |
| | CDR3β | SAREPGQGPYEQY (SEQ ID NO:26) |
| | Vα | RGEDVEQSLFLSVREGDSSVINCTYTDSSMTYLYWYKQEPGAGLQLL TYIFSFMDMKQDQRLTVLLNKKDKHLSLRIADTQTGDSAIYFCAGSG GGTDKLIFGTGTRLQVFPN (SEQ ID NO:158) |
| | Vβ | GAVVSQHPSWVICKSGTSVKIECRSLDFEATTMFWYRQFPKQSLMLM ATTDYGSKATYEQGVEKDKFLINHASLTLSTLTVTSAHPEDSSFYICS AREPGQGPYEQYFGPGTRLTVTE (SEQ ID NO:159) |
| A1A2-M01-620 | CDR1α | DSSQTY (SEQ ID NO:45) |
| | CDR2α | IFSNMDM (SEQ ID NO:28) |
| | CDR3α | AGSGGGTDKLI (SEQ ID NO:29) |
| | CDR1β | DFEATT (SEQ ID NO:130) |
| | CDR2β | TDYGSKA (SEQ ID NO:91) |
| | CDR3β | SAREPGQAWSDQY (SEQ ID NO:162) |
| | Vα | RGEDVEQSLFLSVREGDSSVINCTYTDSSQTYLYWYKQEPGAGLQLL TYIFSNMDMKQDQRLTVLLNKKDKHLSLRIADTQTGDSAIYFCAGSG GGTDKLIFGTGTRLQVFPN (SEQ ID NO:68) |
| | Vβ | GAVVSQHPSWVICKSGTSVKIECRSLDFEATTMFWYRQFPKQSLMLM ATTDYGSKATYEQGVEKDKFLINHASLTLSTLTVTSAHPEDSSFYICS AREPGQAWSDQYFGPGTRLTVTE (SEQ ID NO:163) |
| A1A2-M01-621 | CDR1α | DSEFTY (SEQ ID NO:165) |
| | CDR2α | IFAFESM (SEQ ID NO:118) |
| | CDR3α | AGSGGGTDKLI (SEQ ID NO:29) |
| | CDR1β | DFEATT (SEQ ID NO:130) |
| | CDR2β | TDYGSKA (SEQ ID NO:91) |
| | CDR3β | SAREPGQGPWEQY (SEQ ID NO:35) |
| | Vα | RGEDVEQSLFLSVREGDSSVINCTYTDSEFTYLYWYKQEPGAGLQLL TYIFAFESMKQDQRLTVLLNKKDKHLSLRIADTQTGDSAIYFCAGSGG GTDKLIFGTGTRLQVFPN (SEQ ID NO:166) |
| | Vβ | GAVVSQHPSWVICKSGTSVKIECRSLDFEATTMFWYRQFPKQSLMLM ATTDYGSKATYEQGVEKDKFLINHASLTLSTLTVTSAHPEDSSFYICS AREPGQGPWEQYFGPGTRLTVTE (SEQ ID NO:143) |
| A1A2-M01-622 | CDR1α | DSSSTY (SEQ ID NO:27) |
| | CDR2α | IFAFESM (SEQ ID NO:118) |
| | CDR3α | AGSGGGTDKLI (SEQ ID NO:29) |
| | CDR1β | DFEATT (SEQ ID NO:130) |
| | CDR2β | TDYGSKA (SEQ ID NO:91) |
| | CDR3β | SAREPGQGPWEQY (SEQ ID NO:35) |
| | Vα | RGEDVEQSLFLSVREGDSSVINCTYTDSSSTYLYWYKQEPGAGLQLLT YIFAFESMKQDQRLTVLLNKKDKHLSLRIADTQTGDSAIYFCAGSGG GTDKLIFGTGTRLQVFPN (SEQ ID NO:119) |
| | Vβ | GAVVSQHPSWVICKSGTSVKIECRSLDFEATTMFWYRQFPKQSLMLM ATTDYGSKATYEQGVEKDKFLINHASLTLSTLTVTSAHPEDSSFYICS AREPGQGPWEQYFGPGTRLTVTE (SEQ ID NO:143) |
| A1A2-M01-623 | CDR1α | DSSQTY (SEQ ID NO:45) |
| | CDR2α | IFIFQAM (SEQ ID NO:141) |
| | CDR3α | AGSGGGTDKLI (SEQ ID NO:29) |
| | CDR1β | DFEATT (SEQ ID NO:130) |
| | CDR2β | TDYGSKA (SEQ ID NO:91) |
| | CDR3β | SAREPGQGPWEQY (SEQ ID NO:35) |
| | Vα | RGEDVEQSLFLSVREGDSSVINCTYTDSSQTYLYWYKQEPGAGLQLL TYIFIFQAMKQDQRLTVLLNKKDKHLSLRIADTQTGDSAIYFCAGSGG GTDKLIFGTGTRLQVFPN (SEQ ID NO:168) |
| | Vβ | GAVVSQHPSWVICKSGTSVKIECRSLDFEATTMFWYRQFPKQSLMLM ATTDYGSKATYEQGVEKDKFLINHASLTLSTLTVTSAHPEDSSFYICS AREPGQGPWEQYFGPGTRLTVTE (SEQ ID NO:143) |
| A1A2-M01-624 | CDR1α | YGMSTY (SEQ ID NO:170) |
| | CDR2α | IFSFMDM (SEQ ID NO:76) |
| | CDR3α | AGSGGGTDKLI (SEQ ID NO:29) |
| | CDR1β | DFEATT (SEQ ID NO:130) |
| | CDR2β | TDYGSKA (SEQ ID NO:91) |
| | CDR3β | SAREPGQGPWEQY (SEQ ID NO:35) |
| | Vα | RGEDVEQSLFLSVREGDSSVINCTYQYGMSTYLYWYKQEPGAGLQL LTYIFSFMDMKQDQRLTVLLNKKDKHLSLRIADTQTGDSAIYFCAGS GGGTDKLIFGTGTRLQVFPN (SEQ ID NO:171) |
| | Vβ | GAVVSQHPSWVICKSGTSVKIECRSLDFEATTMFWYRQFPKQSLMLM ATTDYGSKATYEQGVEKDKFLINHASLTLSTLTVTSAHPEDSSFYICS AREPGQGPWEQYFGPGTRLTVTE (SEQ ID NO:143) |
| | CDR1α | DSSMTY (SEQ ID NO:43) |
| A1A2-M01-625 | CDR2α | IFAFESM (SEQ ID NO:118) |
| | CDR3α | AGSGGGTDKLI (SEQ ID NO:29) |
| | CDR1β | DFEATT (SEQ ID NO:130) |
| | CDR2β | TDYGSKA (SEQ ID NO:91) |
| | CDR3β | SAREPGQGPWEQY (SEQ ID NO:35) |
| | Vα | RGEDVEQSLFLSVREGDSSVINCTYTDSSMTYLYWYKQEPGAGLQLL TYIFAFESMKQDQRLTVLLNKKDKHLSLRIADTQTGDSAIYFCAGSGG GTDKLIFGTGTRLQVFPN (SEQ ID NO:173) |
| | Vβ | GAVVSQHPSWVICKSGTSVKIECRSLDFEATTMFWYRQFPKQSLMLM ATTDYGSKATYEQGVEKDKFLINHASLTLSTLTVTSAHPEDSSFYICS AREPGQGPWEQYFGPGTRLTVTE (SEQ ID NO:143) |
| A1A2-M01-626 | CDR1α | DMFSTY (SEQ ID NO:152) |
| | CDR2α | IFAFMQM (SEQ ID NO:149) |
| | CDR3α | AGSGGGTDKLI (SEQ ID NO:29) |
| | CDR1β | DFPIER (SEQ ID NO:82) |
| | CDR2β | TDYGSKA (SEQ ID NO:91) |
| | CDR3β | SAREPGQGPFEQY (SEQ ID NO:33) |
| | Vα | RGEDVEQSLFLSVREGDSSVINCTYPDMFSTYLYWYKQEPGAGLQLL TYIFAFMQMKQDQRLTVLLNKKDKHLSLRIADTQTGDSAIYFCAGSG GGTDKLIFGTGTRLQVFPN (SEQ ID NO:154) |
| | Vβ | GAVVSQHPSWVICKSGTSVKIECRSLDFPIERMFWYRQFPKQSLMLM ATTDYGSKATYEQGVEKDKFLINHASLTLSTLTVTSAHPEDSSFYICS AREPGQGPFEQYFGPGTRLTVTE (SEQ ID NO:175) |

To enhance the correct pairing and expression of the exogenous TCR on the surface of human T cells, the variable regions of the TCR α and β chains were fused to murine TCR constant regions. Classic α chain C region 'LIV' substitutions were introduced into the murine TCR constant regions (i.e., amino acid substitutions were introduced into the murine TCR α chain constant region to allow which comprises amino acid sequence LLVIVLRIL, with the sequence shown in bold). Additionally, classic α chain T48C and β chain S57C substitutions were introduced (with the sequences shown in bold and underlined). Amino Acid Sequence of the α Chain Constant Region:

IQNPEPAVYQLKDPRSQDSTLCLFTDFDSQINVPKTMESGTFITDKCVLDMKAMDSKSN
GAIAWSNQTSFTCQDIFKETNATYPSSDVPCDATLTEKSFETDMNLNFQNLLVIVLRILLLKV
AGFNLLMTLRLWSS (SEQ ID NO: 71)

Amino Acid Sequence of the β Chain Constant Region:

DLRNVTPPKVSLFEPSKAEIANKQKATLVCLARGFFPDHVELSWWVNGKEVHSGVCTD
PQAYKESNYSYCLSSRLRVSATFWHNPRNHFRCQVQFHGLSEEDKWPEGSPKPVTQNISAEA
WGRADCGITSASYQQGVLSATILYEILLGKATLYAVLVSTLVVMAMVKRKNSSR (SEQ ID NO:
72)

Through the 2A self-cleaving peptide (ATNFSLLKQAGDVEENPGP (SEQ ID NO: 73)), the TCR α and β chains (the N-terminal of the α and β chains contains the signal peptide sequences MKTFAGFSFLFLWLQLDCMS (SEQ ID NO: 74) and MLLLLLLLGPGSGL (SEQ ID NO: 75) respectively) were tandemly constructed into a lentiviral expression vector in a β-T2A-α order. Taking ht27-WT TCR as an example, the amino acid sequence of the tandem TCR molecule is as follows (with the signal peptides shown in bold, the constant region sequences underlined, and the 2A self-cleaving peptide in italics):

MLLLLLLLGPGSGLGAVVSQHPSWVICKSGTSVKIECRSLDFQATTMFWYRQFPKQSL
MLMATSNEGSKATYEQGVEKDKFLINHASLTLSTLTVTSAHPEDSSFYICSAREPGQGPYEQY
FGPGTRLTVTEDLRNVTPPKVSLFEPSKAEIANKQKATLVCLARGFFPDHVELSWWVNGKEV
HSGVCTDPQAYKESNYSYCLSSRLRVSATFWHNPRNHFRCQVQFHGLSEEDKWPEGSPKPVT
QNISAEAWGRADCGITSASYQQGVLSATILYEILLGKATLYAVLVSTLVVMAMVKRKNSSRA
TNFSLLKQAGDVEENPGPMKTFAGFSFLFLWLQLDCMSRGEDVEQSLFLSVREGDSSVINCT
YTDSSSTYLYWYKQEPGAGLQLLTYIFSNMDMKQDQRLTVLLNKKDKHLSLRIADTQTGDS

AIYFCAGSGGGTDKLIFGTGTRLQVFPNIQNPEPAVYQLKDPRSQDSTLCLFTDFDSQINVPKT
MESGTFITDKCVLDMKAMDSKSNGAIAWSNQTSFTCQDIFKETNATYPSSDVPCDATLTEKSF
ETDMNLNFQNLLVIVLRILLLKVAGFNLLMTLRLWSS (SEQ ID NO: 1)

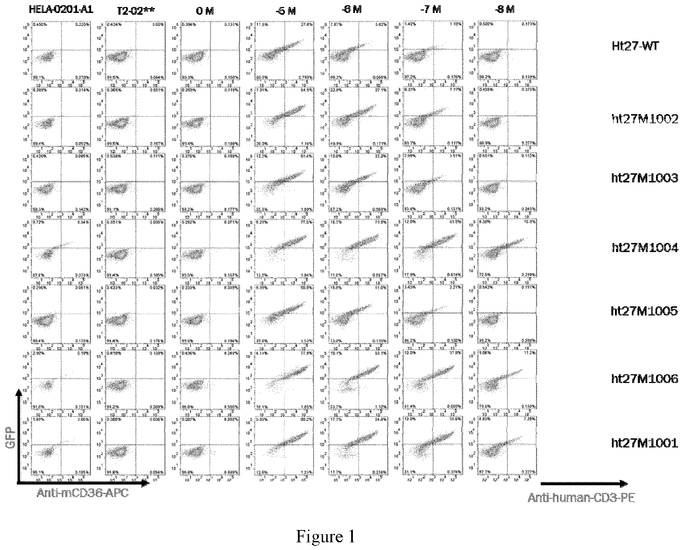

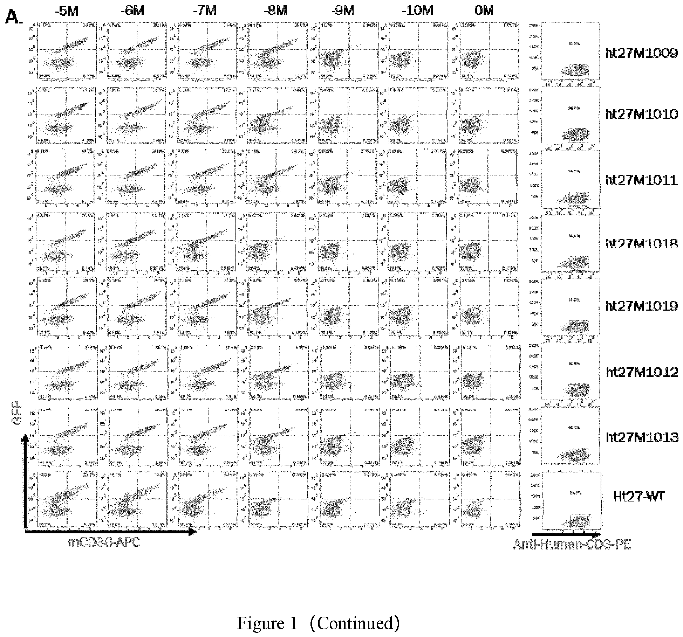

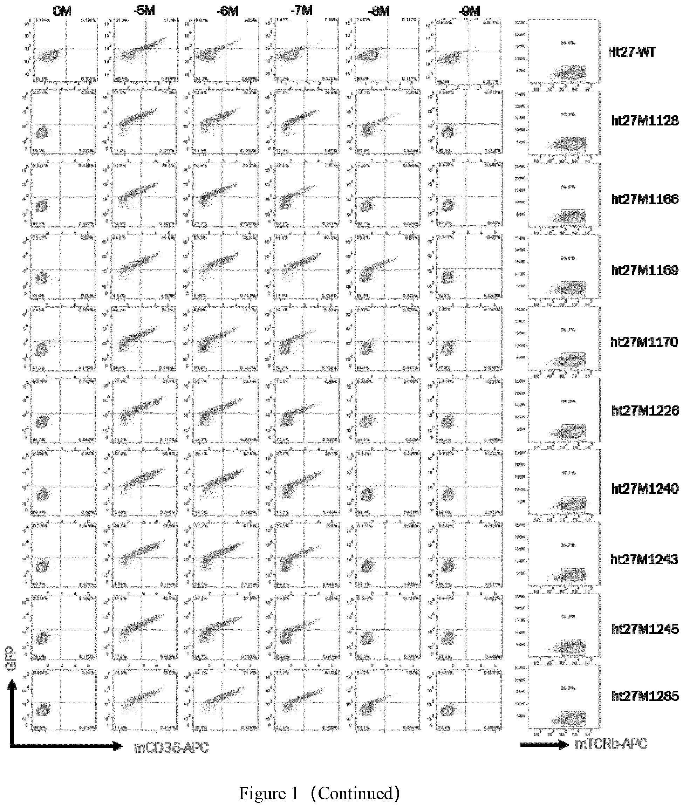

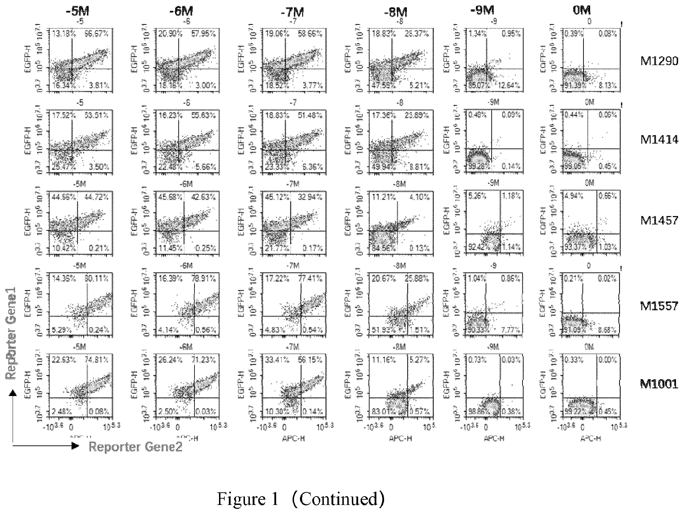

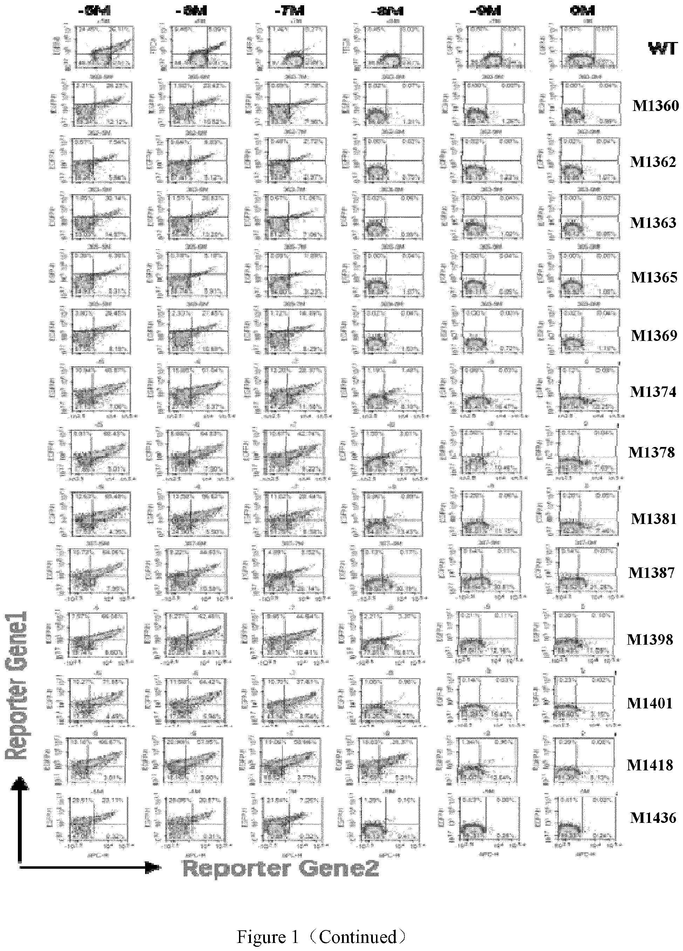

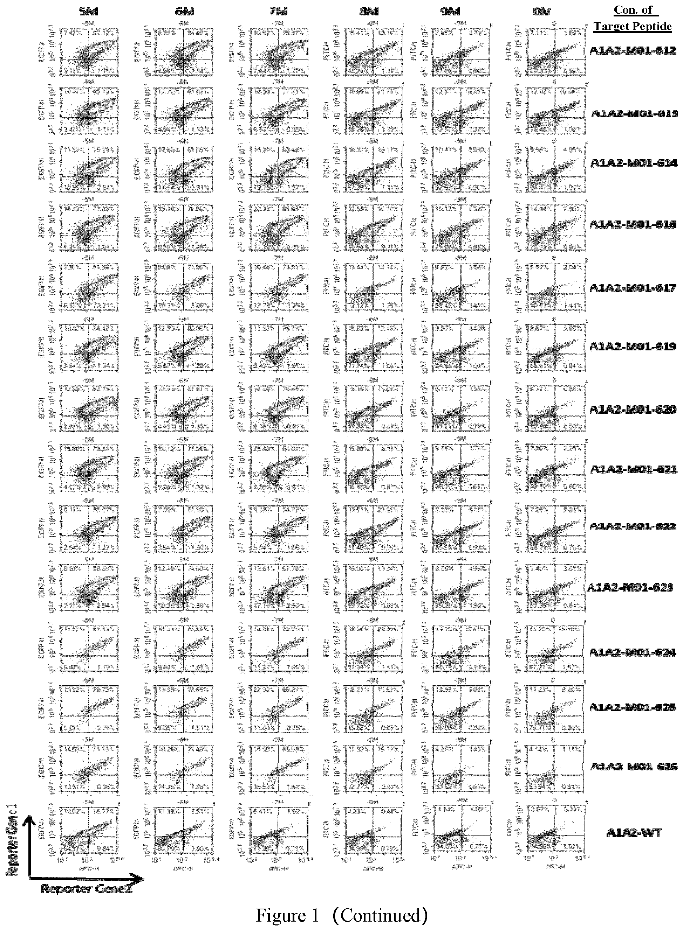

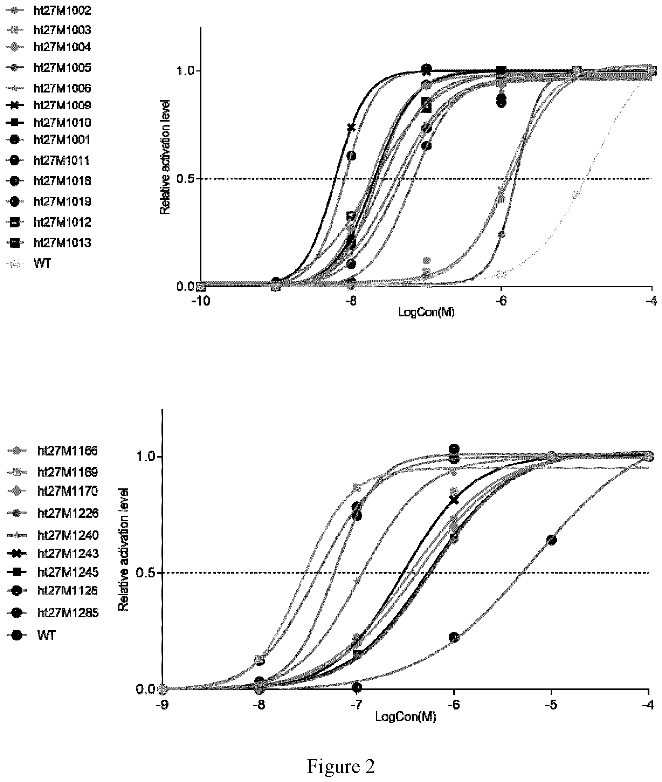

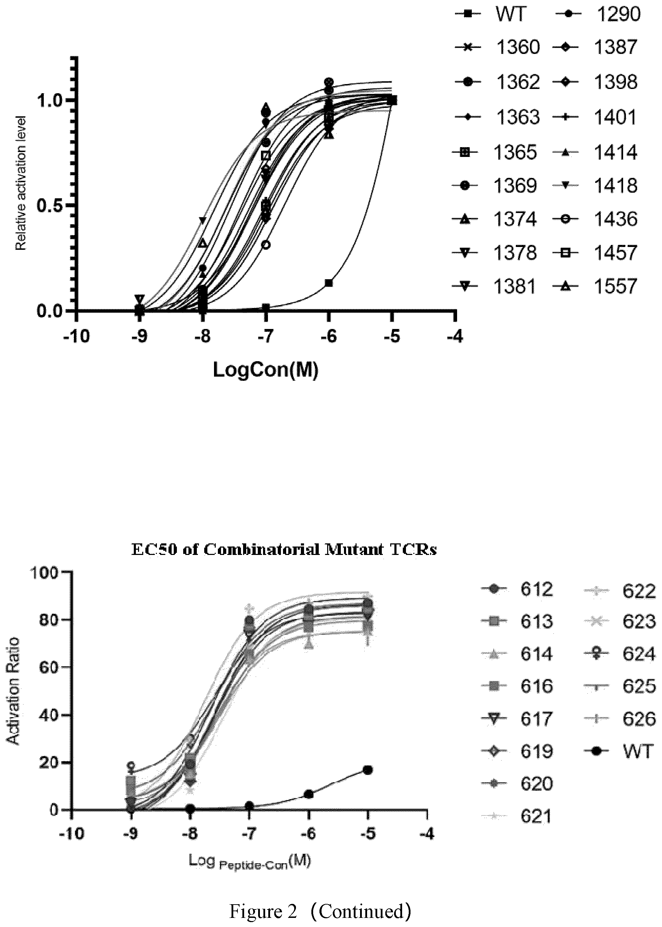

The natural human TCR ht27WT sequence and the substituted TCR sequences of the invention were cloned into lentiviral vectors tandemly expressing TCR respectively, and lentivirus was packaged and then transduced the reporter cell line JKR9, followed by sorting for TCR-expressing positive cells. HLA-A*02:01-positive T2 cells were loaded with varying concentrations of the MAGE-A1 target antigen peptide (KVLEYVIKV (SEQ ID NO: 2)), and then co-incubated with the JKR9 reporter cells transduced with the TCR ht27WT or substituted TCR of the present invention for activation. After 16 hours, the reporter gene was stained and analyzed by flow cytometry. The activation results of the high-affinity TCRs of the present invention on the reporter cells are as shown in Figures 1 and 2. The EC50 values were calculated based on the results as shown in Figure 2, with the EC50 value for ht27-WT set as 1, the EC50 enhancement factor of the substituted TCRs relative to ht27-WT is as shown in Table 5.

**Table 5. EC50 Values and Enhancement Factors of Representative Substituted TCRs Relative to Wild-type ht27 TCR**

| **TCR Name** | **EC50 Value (M)** | **EC50 Enhancement Factor** |
|---|---|---|
| ht27WT | 1.67×10⁻⁵ | 1 |
| ht27M1001 | 4.28×10⁻⁸ | 388 |
| ht27M1002 | 1.36×10⁻⁶ | 12 |
| ht27M1003 | 1.17×10⁻⁶ | 14 |
| ht27M1004 | 1.76×10⁻⁸ | 944 |
| ht27M1005 | 1.55×10⁻⁶ | 11 |
| ht27M1006 | 3.74×10⁻⁸ | 445 |
| ht27M1009 | 6.12×10⁻⁹ | 2722 |
| ht27M1010 | 2.03×10⁻⁸ | 820 |
| ht27M1011 | 8.24×10⁻⁹ | 2020 |
| ht27M1012 | 2.61×10⁻⁸ | 637 |
| ht27M1013 | 1.98×10⁻⁸ | 841 |
| ht27M1018 | 6.36×10⁻⁸ | 262 |
| ht27M1019 | 2.21×10⁻⁸ | 751 |
| ht27M1128 | 3.952 ×10⁻⁸ | 422 |
| ht27M1166 | 3.554 ×10⁻⁷ | 47 |
| ht27M1169 | 2.758 ×10⁻⁸ | 605 |
| ht27M1170 | 4.251 ×10⁻⁷ | 39 |
| ht27M1226 | 5.882 ×10⁻⁷ | 28 |
| ht27M1240 | 1.100 ×10⁻⁷ | 151 |
| ht27M1243 | 2.979 ×10⁻⁷ | 56 |
| ht27M1245 | 5.537 ×10⁻⁷ | 30 |
| ht27M1285 | 5.927 ×10⁻⁸ | 281 |
| ht27M1290 | 3.24 ×10⁻⁸ | 711.05 |
| ht27M1369 | 7.66 ×10⁻⁸ | 217.58 |
| ht27M1378 | 7.40 ×10⁻⁸ | 225.17 |
| ht27M1414 | 2.25 ×10⁻⁸ | 739.46 |
| ht27M1457 | 4.73 ×10⁻⁸ | 352.07 |
| ht27M1557 | 1.49 ×10⁻⁸ | 1115.13 |
| hT27M1387 | 1.278 ×10⁻⁷ | 130.67 |
| hT27M1398 | 5.281 ×10⁻⁸ | 316.23 |
| hT27M1401 | 9.054 ×10⁻⁸ | 184.45 |
| hT27M1374 | 1.093 ×10⁻⁷ | 152.79 |
| hT27M1381 | 1.278 ×10⁻⁷ | 130.67 |
| hT27M1360 | 6.037 ×10⁻⁸ | 276.63 |
| hT27M1362 | 3.990 ×10⁻⁸ | 418.55 |
| hT27M1363 | 6.405 ×10⁻⁸ | 260.73 |
| hT27M1365 | 1.010 ×10⁻⁷ | 165.35 |
| hT27M1418 | 1.014 ×10⁻⁸ | 1646.94 |
| hT27M1436 | 2.016 ×10⁻⁷ | 82.84 |

Wherein, the activation function of the mutated TCRs obtained by combining mutations in different CDR regions, is significantly superior to that of the wild-type TCR ht27, with an affinity in the pM level. The EC50 values are shown in Table 6.

**Table 6. EC50 Values of Combinatorial Mutant TCRs**

| TCR Name | EC50 Value (M) |
|---|---|
| ht27WT | 2.310 ×10⁻⁶ |
| A1A2-M01-612 | 2.297 ×10⁻⁸ |
| A1A2-M01-613 | 2.619 ×10⁻⁸ |
| A1A2-M01-614 | 2.937 ×10⁻⁸ |
| A1A2-M01-616 | 3.227 ×10⁻⁸ |
| A1A2-M01-617 | 2.606 ×10⁻⁸ |
| A1A2-M01-619 | 2.741 ×10⁻⁸ |
| A1A2-M01-620 | 2.504 ×10⁻⁸ |
| A1A2-M01-621 | 3.568 ×10⁻⁸ |
| A1A2-M01-622 | 1.764 ×10⁻⁸ |
| A1A2-M01-623 | 3.064 ×10⁻⁸ |
| A1A2-M01-624 | 2.587 ×10⁻⁸ |
| A1A2-M01-625 | 3.515 ×10⁻⁸ |
| A1A2-M01-626 | 2.263 ×10⁻⁸ |

The results above indicate that TCRs with one or more amino acid substitutions at positions Asn57, Glu58, Gly59, Ser63, Lys64, Ala65, Ser105, Gln111, Tyr114, Ser29, Ser36, and Asn59 show significantly improved affinity relative to the wild-type TCR ht27. These amino acid substitutions can be further combined to produce TCRs with even higher affinity.

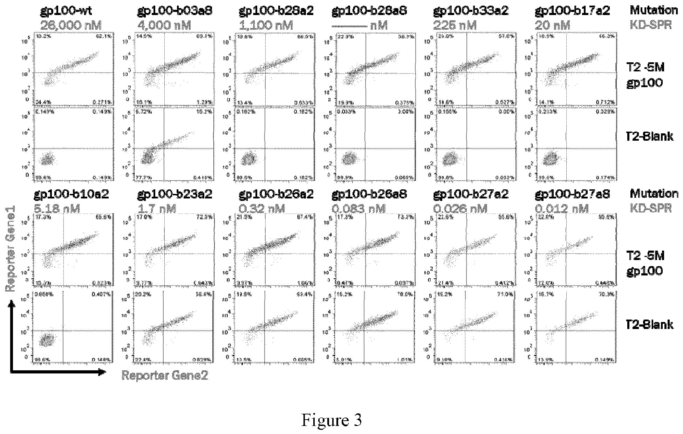

However, after transducing JKR9 reporter cells with combinatorial mutant TCRs, non-specific activation responses were observed with T2 cells not loaded with target peptides (Figure 1). This is likely due to the excessively high affinity of these TCRs. Similar non-specific activation was also observed when using ultra-high affinity gp100-targeting mutant TCRs (gp100-b03a8, gp100-b28a2, gp100-b28a8, gp100-b33a2, gp100-b17a2, gp100-b10a2, gp100-b23a2, gp100-b26a2, gp100-b26a8, gp100-b27a2, gp100-b27a8) known in the prior art in flow cytometric analysis as described above (Figure 3). Despite the presence of non-specific activation, the soluble TCR protein drug Kimmtrak (trade name: Tebentafusp-tebn) prepared with the mutant TCR gp100-b27a8 has been approved for the treatment of metastatic uveal melanoma. This indicates the therapeutic value of ultra-high affinity mutant TCRs (KD-SPR value < 1nM).

### Example 2. Assessing the Affinity of Ultra-High Affinity Mutant TCRs via Dextramer Staining

Dextramer is a multimer of pMHC molecules that can be used in in vitro experiments to identify TCR sequences specific to certain antigenic peptides from T cell libraries. The specific binding of Dextramer to TCRs can be detected and sorted through fluorescence labeling. Furthermore, the strength of Dextramer-TCR binding is positively correlated with TCR affinity. Thus, Dextramer staining can be used to screen for ultra-high affinity mutant TCRs and evaluate their affinity.

To validate this method, experiments were conducted using known affinity mutant TCRs targeting gp100 (gp100-b03a8, gp100-b28a2, gp100-b28a8, gp100-b33a2, gp100-b17a2, gp100-b10a2, gp100-b23a2, gp100-b26a2, gp100-b26a8, gp100-b27a2, gp100-b27a8). The affinities of these known gp100-TCR mutants determined by SPR are as follows:

| **Clone Name** | **WT** | **b03a8** | **b28a2** | **b28a8** | **b33a2** | **b17a2** | **b10a2** | **b23a2** | **b26a2** | **b26a8** | **b27a2** | **b27a8** |
|---|---|---|---|---|---|---|---|---|---|---|---|---|
| Affinity (SPR: nM) | 26,000 | 4,000 | 1,100 | - | 225 | 20 | 5 | 1.7 | 0.32 | 0.083 | 0.026 | 0.012 |

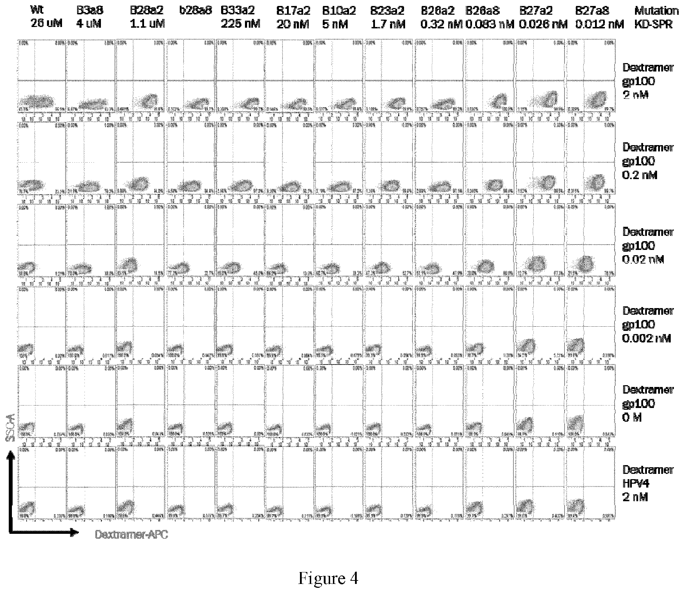

Verification Method is as following:
First, transduce JKR9 cells with gp100-mutant TCR of known affinity through lentiviral to construct a mutant TCR-expressing cell line. Then prepare Dextramer using the gp100 target antigen peptide (YLEPGPVTV, SEQ ID NO: 177), followed by diluting the Dextramer 10 times with PBS to create four concentration gradients (final staining concentrations of 2 nM to 0.002 nM). Subsequently, perform parallel staining on the JKR9 cells transduced with different affinity mutant TCRs using different concentrations of Dextramer. Record the staining positivity rates and MFI (mean fluorescence intensity) of each experimental group through flow cytometry. The results are as shown in Figure 4. Finally, analyze whether the Dextramer staining positivity rates and mean fluorescence intensity (MFI) correlate with the SPR-determined affinity by summarizing data from different experimental groups.

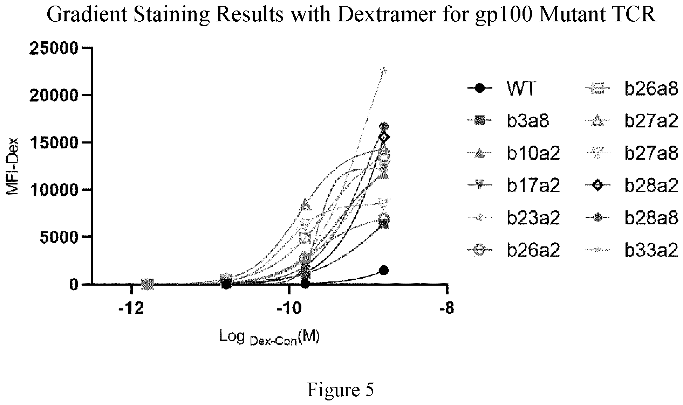

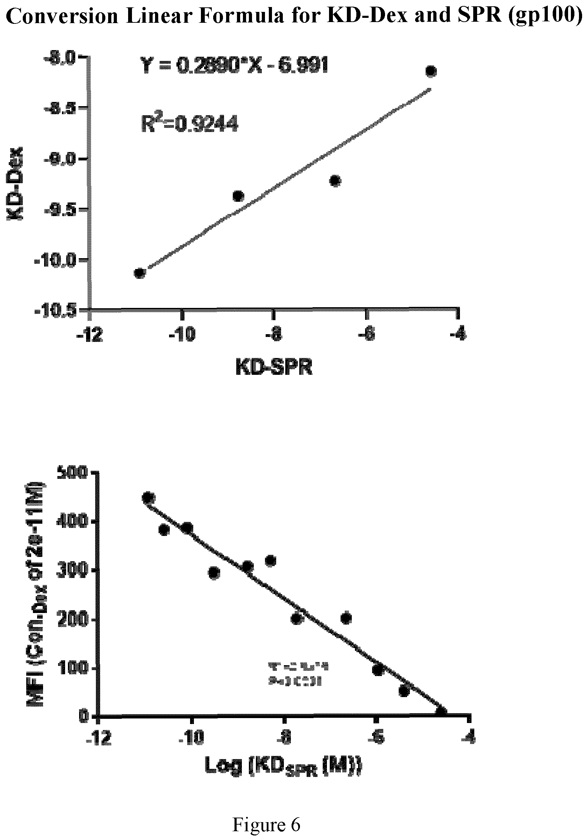

The results indicate that with decreasing Dextramer concentrations during staining, staining advantages of high-affinity TCRs are gradually shown. Both staining positivity rates and MFI exhibit staining advantages related to the affinity of mutant TCRs (Figure 4). After extracting the staining data, further analysis revealed that when the Dextramer concentration used is below 0.2 nM, the staining intensity shows a linear correspondence with the affinity of TCRs (Figure 5). Performing correlation analysis on the MFI of Dextramer staining at the extreme low concentration (0.02 nM) and the EC50 of MFI of Dextramer gradient concentration staining with the true affinity (KD-SPR) measured by SPR respectively. it was found that the MFI of Dextramer staining at the extreme low concentration and MFI of Dextramer gradient concentration staining both reflect the SPR results well and yielded a linear relationship formula between KD-SPR and MFI of Dextramer staining (Figure 6). These results suggest that Dextramer staining can be used to assess the affinity of mutant TCRs.

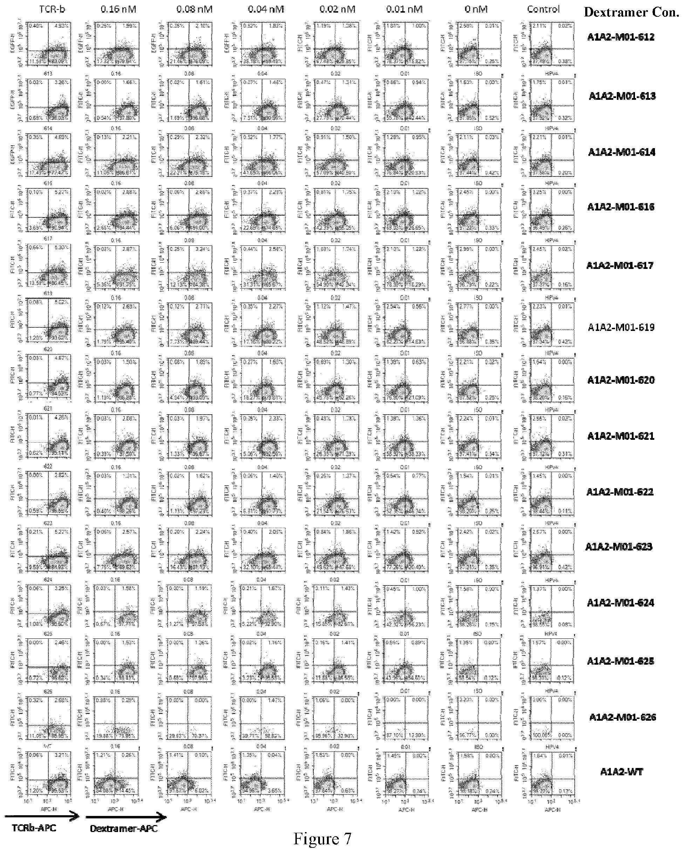

After verification using the above method, the affinity of the mutant TCRs targeting MAGE-A1 of the present invention was evaluated using the Dextramer staining method. The results are as shown in Figures 7-8.

The results show that all the mutant TCRs of the present invention displays strongly positive staining under the condition of Dextramer staining at extremely low concentration, and the mean fluorescence intensity (MFI) increases with Dextramer concentration. This indicates that the mutant TCRs of the present invention has a very high affinity for the target-pMHC complex.

Using the linear relationship formula between KD-SPR and Dextramer staining MFI as described above (Figure 6), the KD-SPR value of the mutant TCRs of the present invention was estimated by introducing the Dextramer staining MFI of the mutant TCRs into the formula. The results are as shown in the table below. The results indicate that the estimated SPR values for all mutant TCRs reached the 10 pM level, demonstrating the extremely high affinity of the mutant TCRs of the present invention.

| TCR nam e | A1A 2-M01-623 | A1A 2-M01-613 | A1A 2-M01-621 | A1A 2-M01-625 | A1A 2-M01-616 | A1A 2-M01-614 | A1A 2-M01-624 | A1A 2-M01-617 | A1A 2-M01-612 | A1A 2-M01-620 | A1A 2-M01-622 |
|---|---|---|---|---|---|---|---|---|---|---|---|
| EC5 0 | 1.87 ×10⁻¹⁰ | 9.61 ×10⁻¹¹ | 1.21 ×10⁻¹⁰ | 8.52 ×10⁻¹¹ | 8.08 ×10⁻¹¹ | 1.63 ×10⁻¹⁰ | 1.16 ×10⁻¹⁰ | 1.03 ×10⁻¹⁰ | 4.75 ×10⁻¹¹ | 2.12 ×10⁻¹⁰ | 9.11 ×10⁻¹¹ |
| KD-SPR | 3.37 ×10⁻¹⁰ | 3.38 ×10⁻¹¹ | 7.47 ×10⁻¹¹ | 2.22 ×10⁻¹¹ | 1.86 ×10⁻¹¹ | 2.08 ×10⁻¹⁰ | 6.51 ×10⁻¹¹ | 4.32 ×10⁻¹¹ | **2.94** ×10⁻¹² | 5.24 ×10⁻¹⁰ | 2.80 ×10⁻¹¹ |

### Example 3: Evaluation of Expression Stability on Cell Membrane of the Affinity-Optimized TCR

293T cells were used for packaging to produce lentiviruses introduced with ht27WT and affinity-optimized TCRs (MOI=10) respectively, followed by transducing the lentiviruses into PBMCs, which were amplified and cultured until day 11 to harvest. At harvest, the cells exhibited good proliferation, with a proliferation fold of 70-150 times and a cell viability of >90%. Staining with human CD3-FITC antibody was performed to confirm T cells and testify successful TCR transduction simultaneously. Staining with anti-mouse TCR β-APC antibody was performed to detect TCR-T expression rates, staining with CD4-APC and CD8-PE-Cy7 antibody was performed to detect CD4/CD8 ratios.

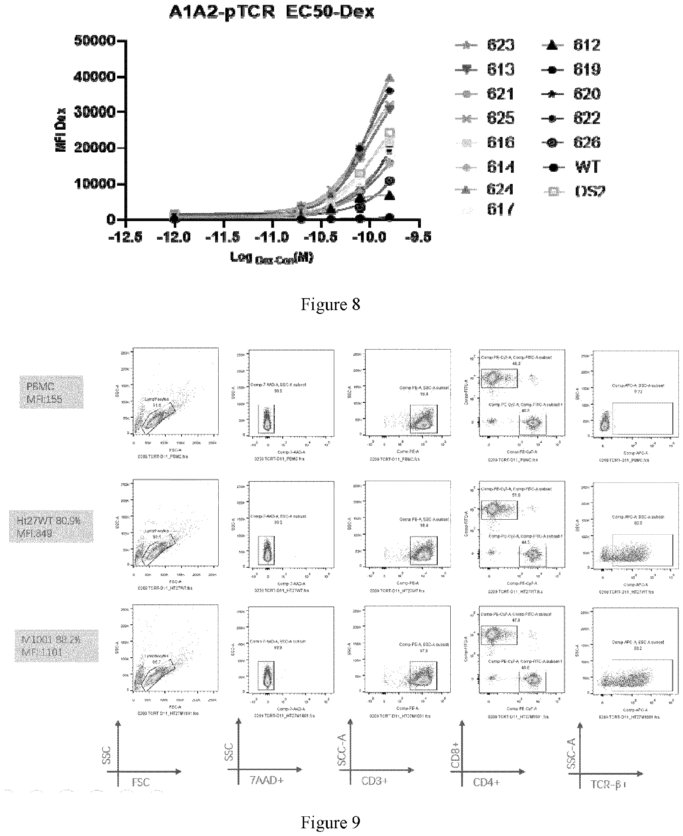

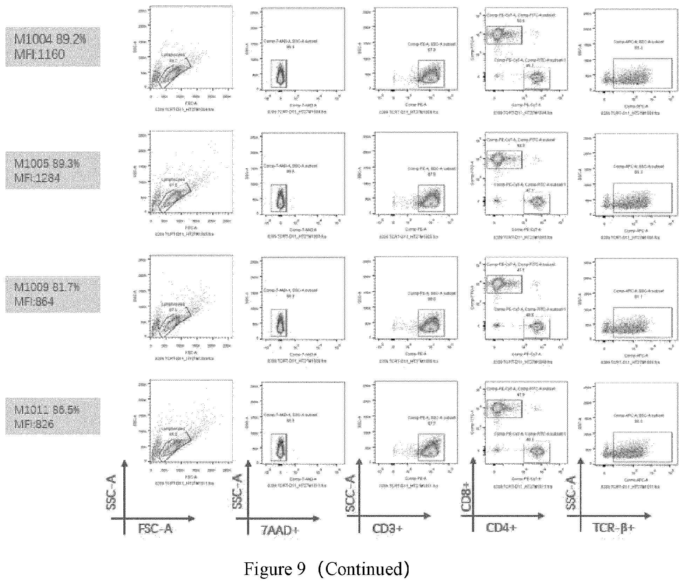

Flow cytometry analysis results showed that under the same transduction and culture conditions, the CD4/CD8 ratio of cells transduced with affinity-optimized TCRs approached 1:1, with TCR positivity rates exceeding 75%, ranging from 75-90% (Figure 9). This indicates that affinity-optimized TCR molecules have good expression stability on membrane, suggesting potential for high antigen reactivity and anti-tumor activity.

### Example 4: Highly Efficient and Specific Killing Against Antigen-Positive Tumor Cells of the Affinity-Optimized TCR-T Cells

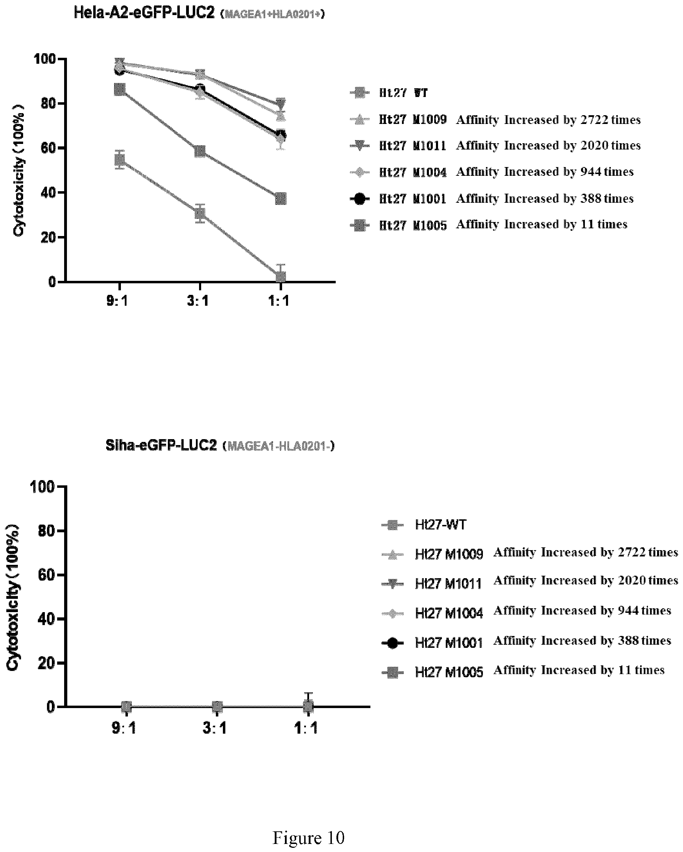

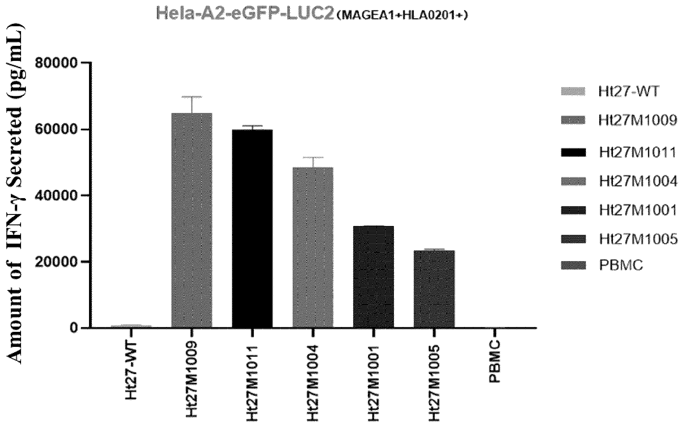

T cells expressing either affinity-optimized TCRs or ht27WT were used as effector cells, with PBMCs not transduced with TCR, expanded and cultured in parallel, as the negative control for the effector cells. Hela-A2-eGFP-LUC cells (MAGE-A1 positive, HLA-A02:01 positive) were served as positive target cells matching HLA-antigen peptide, while Siha-eGFP-LUC cells (MAGE-A1 negative, HLA-A02:01 negative) served as negative target cells non-matching HLA-antigen peptide. All target cells stably expressed the luciferase gene. Effector cells and different target cells were co-cultured at Effector-to-target ratio (E:T) of 9:1, 3:1, and 1:1 for 16-20 hours respectively, followed by addition of a luciferase substrate to detect viable target cells. The proportion of killed target cells was calculated based on the remaining target cells, as shown in Figure 10.

The results indicated that affinity-optimized TCR-T cells had significant killing activity against Hela-A2-eGFP-LUC positive target cells, and their killing activity was significantly higher than that of ht27WT TCR-T cells. Affinity-optimized TCR-T cells showed no killing effect against negative target cells. These results demonstrate that affinity-optimized TCR-T cells possess high-efficient and specific killing capability against target cells matching HLA-antigen peptide, with enhanced killing activity with increased affinity accordingly.

### Example 5: Specific INF-γ Secretion of Affinity-Optimized TCR-T Cells Against Antigen-Positive Tumor Cells

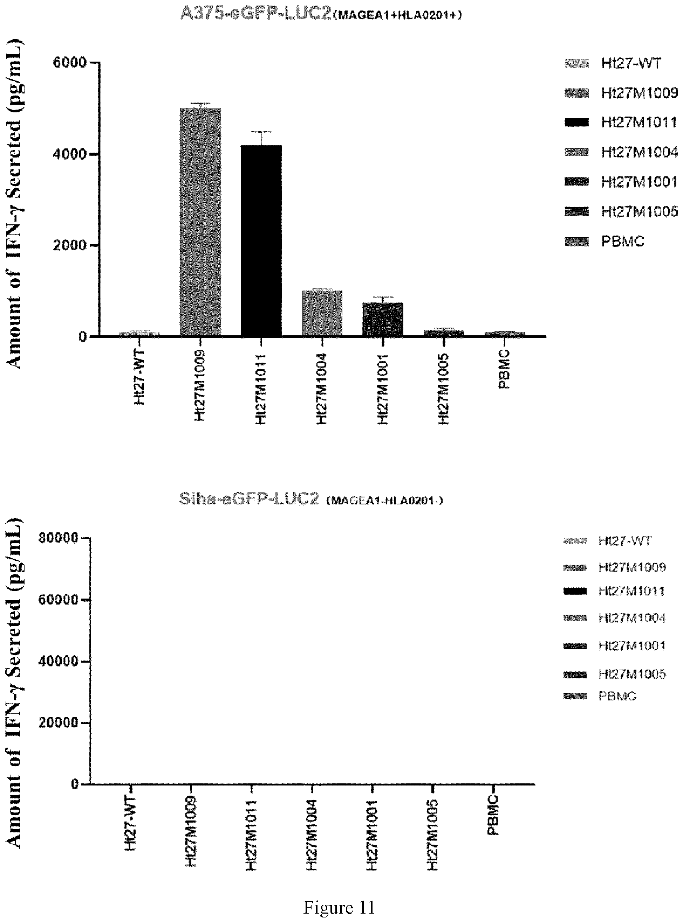

T cells expressing either affinity-optimized TCRs or ht27WT were used as effector cells, with PBMCs not transduced with TCR, expanded and cultured in parallel, as the negative control for the effector cells. Hela-A2-eGFP-LUC cells and A375-eGFP-LUC cells (MAGE-A1 positive, HLA-A02:01 positive) were served as positive target cells matching HLA-antigen peptide, while Siha-eGFP-LUC cells (MAGE-A1 negative, HLA-A02:01 negative) served as negative target cells non-matching HLA-antigen peptide. Effector cells and different target cells were co-cultured at Effector-to-target ratio (E:T) of 1:1 for 16-20 hours respectively. IFN-γ secretion in the supernatant was detected using an IFN-γ ELISA kit (ExCell, Cat# EH008-96), as shown in Figure 11.

The results showed that significant IFN-γ secretion was detected after co-culturing the affinity-optimized TCR-T cells with positive target cells A375 or Hela-A2, and the amount of IFN-γ secreted was significantly higher than that of ht27WT TCR-T cells, while no IFN-γ secretion was detected from negative target cells non-matching HLA-antigen peptide. These results indicate that affinity-optimized TCR-T cells have good specificity in killing target cells matching HLA-antigen peptide, and this specific killing effect enhances with increased affinity.

### Example 6: Non-Specific Activation of Affinity-Optimized TCR-T Cells Against T2-KO-TAP1 Cell Strains Expressing Different HLA Types

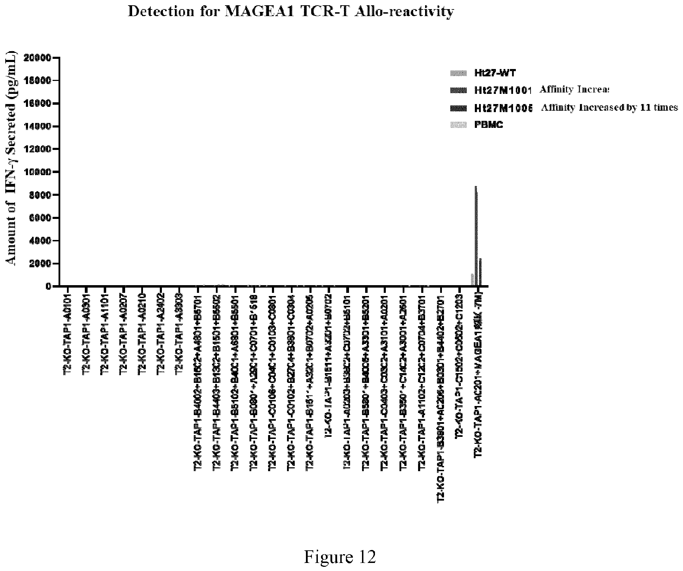

To detect whether affinity-optimized TCR-T cells produce alloreactivity, i.e., antigen-independent cross-reactivity with other mismatched HLA, T2-KO-TAP1 cell strains (HLA knockout and expressing antigen peptide transport protein TAP1) were constructed, with 66 different HLA types expressed. The selected high-frequency HLA-A, HLA-B, HLA-C can cover over 90% of the population. Transducing T2-KO-TAP1 cells with different HLA genes, followed by sorting through flow cytometry, HLA expression rates are all above 95%. 1×10⁵ cells T cells expressing affinity-optimized TCR were used as effector cells, co-cultured with 1×10⁵ target cells (T2-KO-TAP1 cells expressing different HLA types) for 16-24 hours. Supernatant was collected for IFN-γ detection, with T2 cells loaded with MAGE-A1 peptide (HLA-A*02:01 positive) as a positive control for target cells, as shown in Figure 12.

The results showed that no IFN-γ secretion was detected after co-culturing Ht27M1001 (affinity increased 388 times) and Ht27M1005 (affinity increased 11 times) with all T2-KO-TAP1-HLA cells, indicating that Ht27M1001 and Ht27M1005 have good specificity.

### Example 7: Non-Specific Activation of Affinity-Optimized TCRs Against Antigen-Negative Target Cells from Different Tissues and T2-KO-TAP1 Cell Strains Expressing Different HLA Types

To further determine the specificity of the affinity-optimized TCRs for recognizing target antigens, non-specific activation of the affinity-optimized TCRs was tested against antigen-negative target cells from different tissues and T2-KO-TAP1 cell strains expressing different HLA types. The specific experimental procedures are as follows:
Construct detection tools for off-target reaction: Based on transcriptome sequencing data TPM (Transcripts Per Million mapped reads) in the Cancer Cell Line Encyclopedia (CCLE) database, target genes with TPM=0 were defined as negative for target gene expression. Nine cell lines with negative MAGE-A1 gene expression from different tissues were selected as antigen-negative target cells, and HLA-A*02:01 gene was transduced into these cell lines to sever as tool cells for detecting off-target reactions.

Construct detection tools for allo-reactivity: Knockout the endogenous HLA-B51:01 and HLA-C01:02 genes in T2 cells to prevent the expression of endogenous class I HLA molecules (T2KO cells). Subsequently, overexpress the TAP1/2 genes in T2 cells to construct a tool cell with complete functionality to presenting endogenous antigen (T2KO-TAP). Different high-frequency HLA types were transduced into T2KO-TAP respectively to construct T2-KO-TAP1 cell lines with different HLA types as tool cells for detecting allo-reactivity. The specific construction method is as described in Example 6.

Non-specific reactivity detection: During detection, JKR9 reporter cells transduced with affinity-optimized TCRs were co-cultured with antigen-negative target cells expressing HLA-A02:01 and T2-KO-TAP1 cell strains expressing different HLA types. After 16 hours of co-cultivation, flow cytometry was used to determine whether JKR9 reporter cells transduced with high-affinity mutant TCR were activated by the tool cells described above, thereby decide whether the mutant TCR exhibits off-target reactions or allo-reactivity. HLA-antigen peptide-matching positive target cells A375-A1-A2 cells (MAGE-A1 positive, HLA-A02:01 positive) were used as positive controls. The non-specific activation results of the high-affinity TCRs are as shown in the table below.

| **Mutant TCR Clone No.** | **Off-Target Reaction** | | | | | | | | | **Allo-reactivity** | | | | | | **PC** |
|---|---|---|---|---|---|---|---|---|---|---|---|---|---|---|---|---|
| | T2 | Hela-A1KO | A549-AlKO | Siha-A0201 | Caski-A0201 | OVCAR3-A0201 | DLD1-A0201 | MG63-A0201 | SNGM-A0201 | T2-TAP-A1 (A0206/A24 02/A3001) | T2-TAP-A2 (A0207 /A0201/ A3303/A1101) | T2-TAP-B1 (B1301/B40 01/B5801) | T2-TAP-B2 (B4601/B13 02/B5101) | T2-TAP-C1 (C0303/C0302/ C0401/C0801) | T2-TAP-C2 (C0702/C0602 C0102/C0304) | A375-A1-A2 |
| ht27WT | - | - | - | - | - | - | - | - | - | - | - | - | - | - | - | + |
| ht27M1001 | - | - | - | - | - | - | - | - | - | - | - | - | - | - | - | +++ |
| ht27M1002 | - | - | - | - | - | - | - | - | - | - | - | - | - | - | - | +++ |
| ht27M1003 | - | - | - | - | - | - | - | - | - | - | - | - | - | - | - | +++ |
| ht27 M1004 | - | - | - | - | - | - | - | - | - | - | - | - | - | - | - | +++ |
| ht27M1005 | - | - | - | - | - | - | - | - | - | - | - | - | - | - | - | +++ |
| ht27M1006 | - | - | - | - | - | - | - | - | - | - | - | - | - | - | - | +++ |
| ht27M1009 | - | - | - | - | - | - | - | - | - | - | - | - | - | - | - | +++ |
| ht27M1010 | - | - | - | - | - | - | - | - | - | - | - | - | - | - | - | +++ |
| ht27M1011 | - | - | - | - | - | - | - | - | - | - | - | - | - | - | - | +++ |
| ht27M1012 | - | - | - | - | - | - | - | - | - | - | - | - | - | - | - | +++ |
| ht27M1013 | - | - | - | - | - | - | - | - | - | - | - | - | - | - | - | +++ |
| ht27M1018 | - | - | - | - | - | - | - | - | - | - | - | - | - | - | - | +++ |
| ht27M1019 | - | - | - | - | - | - | - | - | - | - | - | - | - | - | - | +++ |
| ht27M1128 | - | - | - | - | - | - | - | - | - | - | - | - | - | - | - | +++ |
| ht27M1166 | - | - | - | - | - | - | - | - | - | - | - | - | - | - | - | +++ |
| ht27M1169 | | - | - | - | - | - | - | - | - | - | - | - | - | - | - | +++ |
| ht27M1170 | - | - | - | - | - | - | - | - | - | - | - | - | - | - | - | +++ |
| ht27M1226 | - | - | - | - | - | - | - | - | - | - | - | - | - | - | - | +++ |
| ht27M1240 | - | - | - | - | - | - | - | - | - | - | - | - | - | - | - | +++ |
| ht27M1243 | - | - | - | - | - | - | - | - | - | - | - | - | - | - | - | +++ |
| ht27M1245 | - | - | - | - | - | - | - | - | - | - | - | - | - | - | - | +++ |
| ht27M1285 | - | - | - | - | - | - | - | - | - | - | - | - | - | - | - | +++ |
| ht27M1290 | - | - | - | - | - | - | - | - | - | - | - | - | - | - | - | +++ |
| ht27M1369 | - | - | - | - | - | - | - | - | - | - | - | - | - | - | - | +++ |
| ht27M1378 | - | - | - | - | - | - | - | | - | - | - | - | - | - | - | +++ |
| ht27M1414 | - | - | - | - | - | - | - | - | - | - | - | - | - | - | - | +++ |
| ht27M1457 | - | - | - | - | - | - | - | - | - | - | - | - | - | - | - | +++ |
| ht27M1557 | - | - | - | - | - | - | - | - | - | - | - | - | - | - | - | +++ |
| hT27M1387 | - | - | - | - | - | - | - | - | - | - | - | - | - | - | - | +++ |
| hT27M1398 | - | - | - | - | - | - | - | - | - | - | - | - | - | - | - | +++ |
| hT27M1401 | - | - | - | - | - | - | - | - | - | - | - | - | - | - | - | +++ |
| hT27M1374 | - | - | - | - | - | - | - | - | - | - | - | - | - | - | - | +++ |
| hT27M1381 | - | - | - | - | - | - | - | - | - | - | - | - | - | - | - | +++ |
| hT27M1360 | - | - | - | - | - | - | - | - | - | - | - | - | - | - | - | +++ |
| hT27M1362 | - | - | - | - | - | - | - | - | - | - | - | - | - | - | - | +++ |
| hT27M1363 | - | - | - | - | - | - | - | - | - | - | - | - | - | - | - | +++ |
| hT27M1365 | - | - | - | - | - | - | - | - | - | - | - | - | - | - | - | +++ |
| hT27M1418 | - | - | - | - | - | - | - | - | - | - | - | - | - | - | - | +++ |
| hT27M1436 | - | - | - | - | - | - | - | - | - | - | - | - | - | - | - | +++ |

The results show that no non-specific activation was detected for HLA-matching antigen-negative target cells or T2-KO-TAP1 cell strains expressing different HLA types for ht27M1001, ht27M1002, ht27M1003, ht27M1004, ht27M1005, ht27M1006, ht27M1009, ht27M1010, ht27M1011, ht27M1012, ht27M1013, ht27M1018, ht27M1019, ht27M1128, ht27M1166, ht27M1169, ht27M1170, ht27M1226, ht27M1240, ht27M1243, ht27M1245, ht27M1285, ht27M1290, ht27M1369, ht27M1378, ht27M1414, ht27M1457, ht27M1557, ht27M1387, ht27M1398, ht27M1401, ht27M1374, ht27M1381, ht27M1360, ht27M1362, ht27M1363, ht27M1365, ht27M1418, and ht27M1436, indicating that these affinity-optimized TCRs have good specificity.

This application references various issued patents, published patent applications, journal articles, and other publications, all of which are incorporated herein by reference. In case of any conflict between any incorporated references and this specification, this specification shall prevail. Additionally, any specific embodiments of the invention falling within the prior art scope may be explicitly excluded from any one or more claims. Such embodiments are considered known to those skilled in the art and may be excluded even if not explicitly listed in this application. Any specific embodiments of the invention may be excluded from any claims for any reason, regardless of whether related to the existence of prior art.

Although the invention has been described with reference to specific embodiments, those skilled in the art should understand that various modifications and equivalent substitutions can be made without departing from the true spirit and scope of the invention. Furthermore, many modifications can be made to adapt specific circumstances, materials, compositions, methods, or method steps to the purposes, spirit, and scope of the invention. All such modifications are intended to fall within the scope of the claims.

## Claims

1. A modified T-cell receptor (TCR), wherein the modified TCR comprises an α chain variable region comprising CDR1α, CDR2α, and CDR3α, and a β chain variable region comprising CDR1β, CDR2β, and CDR3β, the modified TCR comprises the following amino acid substitutions relative to its parental TCR:
(i) amino acid substitutions in the β chain variable region, comprising:
(a) amino acid substitutions at one or more of the following positions in CDR1β: Gln29, Ala36, Thr37, and Thr38;
(b) amino acid substitutions at one or more of the following positions in CDR2β: Ser56, Asn57, Glu58, Gly59, Ser63, Lys64, and Ala65; and/or
(c) amino acid substitutions at one or more of the following positions in CDR3β: Ser105, Glu108, Pro109, Gly110, Gln111, Gly112, Pro113, Tyr114, and Glu115;
and/or
(ii) amino acid substitutions in the α chain variable region, comprising:
(d) amino acid substitutions at one or more of the following positions in CDR1α: Asp27, Ser28, Ser29, Ser36, Thr37, and Tyr38;
(e) amino acid substitutions at the following positions in CDR2α: Ile56, Phe57, Ser58, Asn59, Met63, and Asp64; and/or
(f) amino acid substitution at position Thr26 in FR1α;
wherein the parental TCR comprises CDR1β having an amino acid sequence as set forth in SEQ ID NO: 24, CDR2β having an amino acid sequence as set forth in SEQ ID NO: 25, CDR3β having an amino acid sequence as set forth in SEQ ID NO: 26, CDR1α having an amino acid sequence as set forth in SEQ ID NO: 27, CDR2α having an amino acid sequence as set forth in SEQ ID NO: 28, and CDR3α having an amino acid sequence as set forth in SEQ ID NO: 29;
wherein amino acid positions are numbered according to the International ImMunoGeneTics Information System (IMGT) nomenclature.

2. The modified TCR of claim 1, wherein the modified TCR comprises one or more of the following amino acid substitutions (1)-(33) relative to the parental TCR:
(1) amino acid substitution at position Gln29 in CDR1β, selected from the group consisting of Q29E and Q29P;
(2) amino acid substitution at position Ala36 in CDR1β: A36I;
(3) amino acid substitution at position Thr37 in CDR1β: T37E;
(4) amino acid substitution at position Thr38 in CDR1β: T38R;
(5) amino acid substitution at position Ser56 in CDR2β: S56T;
(6) amino acid substitution at position Asn57 in CDR2β, selected from the group consisting of N57D, N57E, N57H, N57K, N57Q, N57R, N57S, and N57T;
(7) amino acid substitution at position Glu58 in CDR2β, selected from the group consisting of E58A, E58F, E58I, E58L, E58M, E58P, E58V, E58Y, and E58W;
(8) amino acid substitution at position Gly59 in CDR2β, selected from the group consisting of G59P, G59L, G59D, G59E, G59H, G59K, G59Q, G59R, G59S, and G59T;
(9) amino acid substitution at position Ser63 in CDR2β, selected from the group consisting of S63D, S63E, S63H, S63K, S63Q, S63R, and S63T;
(10) amino acid substitution at position Lys64 in CDR2β, selected from the group consisting of K64Q, K64M, K64C, K64W, K64T, K64P, and K64A;
(11) amino acid substitution at position Ala65 in CDR2β, selected from the group consisting of A65F, A65I, A65L, A65M, A65P, A65V, A65W, and A65Y;
(12) amino acid substitution at position Ser105 in CDR3β, selected from the group consisting of S105L, S105N, S105H, S105Q, S105K, and S105R;
(13) amino acid substitution at position Glu108 in CDR3β, selected from the group consisting of E108D, E108F, E108T, E108I, E108K, and E108P;
(14) amino acid substitution at position Gly110 in CDR3β: G110W;
(15) amino acid substitution at position Gln111 in CDR3β, selected from the group consisting of Q111D, Q111N, Q111K, Q111S, Q111G, Q111A, Q111V, Q111E, Q111L, and Q111H;
(16) amino acid substitution of EPGQGP with VNDGT at positions Glu108 to Pro113 in CDR3β;
(17) amino acid substitution at position Gly112 in CDR3β, selected from the group consisting of G112P, G112A, and G112E;
(18) amino acid substitution at position Pro113 in CDR3β, selected from the group consisting of P113W and P113H;
(19) amino acid substitution at position Tyr114 in CDR3β, selected from the group consisting of Y114I, Y114F, Y114K, Y114D, Y114L, Y114W, Y114N, Y114P, and Y114S;
(20) amino acid substitution at position Glu115 in CDR3β, selected from the group consisting of E115D and E115I;
(21) amino acid substitution at position Asp27 in CDR1α, selected from the group consisting of D27C and D27Y;
(22) amino acid substitution at position Ser28 in CDR1α, selected from the group consisting of S28M, S28A, S28G, S28Y, and S28P;
(23) amino acid substitution at position Ser29 in CDR1α, selected from the group consisting of S29E, S29T, S29Y, S29A, S29F, S29I, S29L, S29M, S29P, S29V, S29W, and S29N;
(24) amino acid substitution at position Ser36 in CDR1α, selected from the group consisting of S36D, S36M, S36L, S36Q, S36I, S36F, S36P, and S36V;
(25) amino acid substitution at position Thr37 in CDR1α: T37I;
(26) amino acid substitution at position Tyr38 in CDR1α: Y38L;
(27) amino acid substitution at position Ile56 in CDR2α, selected from the group consisting of I56T and I56G;
(28) amino acid substitution at position Phe57 in CDR2α, selected from the group consisting of F57W and F57T;
(29) amino acid substitution at position Ser58 in CDR2α, selected from the group consisting of S58G, S58I, S58N, S58A, S58P, and S58W;
(30) amino acid substitution at position Asn59 in CDR2α, selected from the group consisting of N59L, N59F, N59D, N59E, N59H, N59K, N59Q, N59R, N59S, and N59T;
(31) amino acid substitution at position Met63 in CDR2α, selected from the group consisting of M63D, M63Q, M63T, and M63E;
(32) amino acid substitution at position Asp64 in CDR2α, selected from the group consisting of D64A, D64Q, D64T, D64Y, and D64S;
(33) amino acid substitution at position Thr26 in FR1α, selected from the group consisting of T26S, T26I, T26P, T26Q, T26A, and T26V.

3. The modified TCR according to claim 1 or 2, wherein the modified TCR comprises one or more of the following amino acid substitutions (1)-(4) in CDR1β relative to the parental TCR:
(1) Q29E or Q29P;
(2) A36I;
(3) T37E;
(4) T38R;
preferably, the modified TCR comprises the amino acid substitutions Q29E or Q29PA36IT37ET38R in CDR1β relative to the parental TCR.

4. The modified TCR according to any one of claims 1-3, wherein the modified TCR comprises one or more of the following amino acid substitutions (1)-(7) in CDR2β relative to the parental TCR:
(1) S56T;
(2) N57Q or N57D;
(3) E58L, E58P, or E58Y;
(4) G59Q, G59P, or G59L;
(5) S63T or S63D;
(6) K64C or K64Q;
(7) A65V;
preferably, the modified TCR comprises the amino acid substitutions N57DE58YG59QS63TK64CA65V, S56TE58LG59P, S56TN57QE58PG59L, S56TN57DE58Y, or S63DK64Q in CDR2β relative to the parental TCR.

5. The modified TCR according to any one of claims 1-4, wherein the modified TCR comprises one or more of the following amino acid substitutions (1)-(8) in CDR3β relative to the parental TCR:
(1) G110W;
(2) Q111D, Q111G, or Q111S;
(3) G112E, G112P, or G112A;
(4) P113H or P113W;
(5) Y114S, Y114F, Y114L, or Y114W;
(6) E115I or E115D;
(7) S105L, S105Q, S105N, or S105H;
(8) amino acid substitution of EPGQGP with VNDGT at positions Glu108 to Pro113;
preferably, the modified TCR comprises the amino acid substitutions: G110WQ111DG112PP113W, G112AP113WY114SE115D, Y114F, Y114W, Q111GY114F, Q111SY114F, S105L, S105N, amino acid substitution of EPGQGP with VNDGT at positions Glu108 to Pro113, or G112EP113HY114LE1151 in CDR3β relative to the parental TCR.

6. The modified TCR according to any of claims 1-5, wherein the modified TCR comprises one or more of the following amino acid substitutions (1)-(3) relative to the parental TCR:
(1) amino acid substitutions in CDR1β: Q29E or Q29PA36IT37ET38R;
(2) amino acid substitutions in CDR2β: N57DE58YG59QS63TK64CA65V, S56TE58LG59P, S56TN57QE58PG59L, S56TN57DE58Y, or S63DK64Q;
(3) amino acid substitutions in CDR3β: G110WQ111DG112PP113W, G112AP113WY114SE115D, Y114F, Y114W, Q111GY114F, Q111SY114F, S105L, S105N, or the substitution of EPGQGP with VNDGT at positions Glu108 to Pro113 or G112EP113HY114LE115I.

7. The modified TCR according to any of claims 1-6, wherein the modified TCR comprises one or more of the following amino acid substitutions (1)-(7) in CDR1α and/or FR1α relative to the parental TCR:
(1) D27C or D27Y;
(2) S28Y, S28P, S28M, S28A, or S28G;
(3) S29T, S29L, S29Y, S29F, S29E, or S29M;
(4) S36F, S36D, S36M, S36L, or S36Q;
(5) T37I;
(6) Y38L;
(7) T26A, T26V, T26S, T26I, T26P, or T26Q;
preferably, the modified TCR comprises the following amino acid substitutions in CDR1α and/or FR1α relative to the parental TCR: S29TS36DT37IY38L, T26AS28YS29L, T26VS28PS29Y, S36L, T26SS28MS29L, T26ID27CS28AS29F, S36Q, T26PS28MS29F, S36M, S29ES36F, or T26QD27YS28GS29M.

8. The modified TCR according to any of claims 1-7, wherein the modified TCR comprises one or more of the following amino acid substitutions (1)-(6) in CDR2α relative to the parental TCR:
(1) I56T or 156G;
(2) F57W or F57T;
(3) S58G, S58I, S58N, S58A, S58P, or S58W;
(4) N59F, N59T, N59H, or N59L;
(5) M63D, M63Q, M63T, or M63E;
(6) D64A, D64Q, D64T, D64Y, or D64S;
preferably, the modified TCR comprises the following amino acid substitutions in CDR2α relative to the parental TCR: N59F, S58IN59FM63QD64A, S58AN59FD64Q, S58IN59FM63QD64A, S58GN59FM63DD64T, S58IN59FM63QD64Y, S58NN59TM63Q, N59FM63TD64Y, S58AN59FM63ED64S, I56TF57WS58PN59H, I56GF57TS58WN59L, or I56TS58WN59F.

9. The modified TCR according to any of claims 1-8, wherein the modified TCR comprises one or more of the following amino acid substitutions (1)-(2) relative to the parental TCR:
(1) amino acid substitutions in CDR1α and/or FR1α: S29TS36DT37IY38L, T26AS28YS29L, T26VS28PS29Y, S36L, T26SS28MS29L, T26ID27CS28AS29F, S36Q, T26PS28MS29F, S36M, S29ES36F, or T26QD27YS28GS29M;
(2) amino acid substitutions in CDR2α: N59F, S58IN59FM63QD64A, S58AN59FD64Q, S58IN59FM63QD64A, S58GN59FM63DD64T, S58IN59FM63QD64Y, S58NN59TM63Q, N59FM63TD64Y, S58AN59FM63ED64S, I56TF57WS58PN59H, I56GF57TS58WN59L, or I56TS58WN59F.

10. The modified TCR according to claim 1, wherein the modified TCR comprises:
(i) an α chain variable region, comprising CDR1α, CDR2α, and CDR3α, with amino acid sequences as shown in SEQ ID NOs: 27-29, respectively; and
(ii) a β chain variable region, comprising
CDR1β, CDR2β, and CDR3β with amino acid sequences as shown in SEQ ID NOs: 24, 30, and 26, respectively,
CDR1β, CDR2β, and CDR3β with amino acid sequences as shown in SEQ ID NOs: 24, 25, and 31, respectively,
CDR1β, CDR2β, and CDR3β with amino acid sequences as shown in SEQ ID NOs: 24, 25, and 32, respectively,
CDR1β, CDR2β, and CDR3β with amino acid sequences as shown in SEQ ID NOs: 24, 25, and 33, respectively,
CDR1β, CDR2β, and CDR3β with amino acid sequences as shown in SEQ ID NOs: 24, 25, and 34, respectively,
CDR1β, CDR2β, and CDR3β with amino acid sequences as shown in SEQ ID NOs: 24, 25, and 35, respectively,
CDR1β, CDR2β, and CDR3β with amino acid sequences as shown in SEQ ID NOs: 24, 30, and 33, respectively,
CDR1β, CDR2β, and CDR3β with amino acid sequences as shown in SEQ ID NOs: 24, 30, and 34, respectively,
CDR1β, CDR2β, and CDR3β with amino acid sequences as shown in SEQ ID NOs: 24, 30, and 35, respectively,
CDR1β, CDR2β, and CDR3β with amino acid sequences as shown in SEQ ID NOs: 24, 25, and 36, respectively,
CDR1β, CDR2β, and CDR3β with amino acid sequences as shown in SEQ ID NOs: 24, 25, and 37, respectively,
CDR1β, CDR2β, and CDR3β with amino acid sequences as shown in SEQ ID NOs: 24, 30, and 36, respectively,
CDR1β, CDR2β, and CDR3β with amino acid sequences as shown in SEQ ID NOs: 24, 30, and 37, respectively,
CDR1β, CDR2β, and CDR3β with amino acid sequences as shown in SEQ ID NOs: 24, 25, and 38, respectively,
CDR1β, CDR2β, and CDR3β with amino acid sequences as shown in SEQ ID NOs: 24, 25, and 39, respectively,
CDR1β, CDR2β, and CDR3β with amino acid sequences as shown in SEQ ID NOs: 24, 25, and 40, respectively,
CDR1β, CDR2β, and CDR3β with amino acid sequences as shown in SEQ ID NOs: 24, 25, and 41, respectively,
CDR1β, CDR2β, and CDR3β with amino acid sequences as shown in SEQ ID NOs: 24, 25, and 79, respectively,
CDR1β, CDR2β, and CDR3β with amino acid sequences as shown in SEQ ID NOs: 82, 25, and 26, respectively,
CDR1β, CDR2β, and CDR3β with amino acid sequences as shown in SEQ ID NOs: 24, 85, and 26, respectively,
CDR1β, CDR2β, and CDR3β with amino acid sequences as shown in SEQ ID NOs: 24, 88, and 26, respectively,
CDR1β, CDR2β, and CDR3β with amino acid sequences as shown in SEQ ID NOs: 24, 91, and 26, respectively,
CDR1β, CDR2β, and CDR3β with amino acid sequences as shown in SEQ ID NOs: 24, 25, and 94, respectively,
CDR1β, CDR2β, and CDR3β with amino acid sequences as shown in SEQ ID NOs: 130, 25, and 131, respectively,
CDR1β, CDR2β, and CDR3β with amino acid sequences as shown in SEQ ID NOs: 24, 91, and 35, respectively,
CDR1β, CDR2β, and CDR3β with amino acid sequences as shown in SEQ ID NOs: 130, 91, and 35, respectively,
CDR1β, CDR2β, and CDR3β with amino acid sequences as shown in SEQ ID NOs: 130, 153, and 35, respectively,
CDR1β, CDR2β, and CDR3β with amino acid sequences as shown in SEQ ID NOs: 130, 91, and 26, respectively,
CDR1β, CDR2β, and CDR3β with amino acid sequences as shown in SEQ ID NOs: 130, 91, and 162, respectively, or
CDR1β, CDR2β, and CDR3β with amino acid sequences as shown in SEQ ID NOs: 82, 91, and 33, respectively.

11. The modified TCR according to claim 1, wherein the modified TCR comprises:
(i) a β chain variable region comprising CDR1β, CDR2β, and CDR3β with amino acid sequences as shown in SEQ ID NOs: 24-26 respectively; and
(ii) an α chain variable region comprising
CDR1α, CDR2α, and CDR3α with amino acid sequences as shown in SEQ ID NOs: 42, 28, and 29 respectively,
CDR1α, CDR2α, and CDR3α with amino acid sequences as shown in SEQ ID NOs: 43, 28, and 29 respectively,
CDR1α, CDR2α, and CDR3α with amino acid sequences as shown in SEQ ID NOs: 44, 28, and 29 respectively,
CDR1α, CDR2α, and CDR3α with amino acid sequences as shown in SEQ ID NOs: 45, 28, and 29 respectively,
CDR1α, CDR2α, and CDR3α with amino acid sequences as shown in SEQ ID NOs: 27, 76, and 29 respectively,
CDR1α, CDR2α, and CDR3α with amino acid sequences as shown in SEQ ID NOs: 97, 28, and 29 respectively,
CDR1α, CDR2α, and CDR3α with amino acid sequences as shown in SEQ ID NOs: 100, 28, and 29 respectively,
CDR1α, CDR2α, and CDR3α with amino acid sequences as shown in SEQ ID NOs: 103, 28, and 29 respectively,
CDR1α, CDR2α, and CDR3α with amino acid sequences as shown in SEQ ID NOs: 27, 106, and 29 respectively,
CDR1α, CDR2α, and CDR3α with amino acid sequences as shown in SEQ ID NOs: 27, 109, and 29 respectively,
CDR1α, CDR2α, and CDR3α with amino acid sequences as shown in SEQ ID NOs: 27, 112, and 29 respectively,
CDR1α, CDR2α, and CDR3α with amino acid sequences as shown in SEQ ID NOs: 27, 115, and 29 respectively,
CDR1α, CDR2α, and CDR3α with amino acid sequences as shown in SEQ ID NOs: 27, 118, and 29 respectively,
CDR1α, CDR2α, and CDR3α with amino acid sequences as shown in SEQ ID NOs: 27, 121, and 29 respectively,
CDR1α, CDR2α, and CDR3α with amino acid sequences as shown in SEQ ID NOs: 27, 124, and 29 respectively,
CDR1α, CDR2α, and CDR3α with amino acid sequences as shown in SEQ ID NOs: 27, 127, and 29 respectively,
CDR1α, CDR2α, and CDR3α with amino acid sequences as shown in SEQ ID NOs: 103, 76, and 29 respectively,
CDR1α, CDR2α, and CDR3α with amino acid sequences as shown in SEQ ID NOs: 44, 76, and 29 respectively,
CDR1α, CDR2α, and CDR3α with amino acid sequences as shown in SEQ ID NOs: 140, 141, and 29 respectively,
CDR1α, CDR2α, and CDR3α with amino acid sequences as shown in SEQ ID NOs: 146, 118, and 29 respectively,
CDR1α, CDR2α, and CDR3α with amino acid sequences as shown in SEQ ID NOs: 45, 149, and 29 respectively,
CDR1α, CDR2α, and CDR3α with amino acid sequences as shown in SEQ ID NOs: 152, 149, and 29 respectively,
CDR1α, CDR2α, and CDR3α with amino acid sequences as shown in SEQ ID NOs: 43, 76, and 29 respectively,
CDR1α, CDR2α, and CDR3α with amino acid sequences as shown in SEQ ID NOs: 45, 28, and 29 respectively,
CDR1α, CDR2α, and CDR3α with amino acid sequences as shown in SEQ ID NOs: 165, 118, and 29 respectively,
CDR1α, CDR2α, and CDR3α with amino acid sequences as shown in SEQ ID NOs: 27, 118, and 29 respectively, or
CDR1α, CDR2α, and CDR3α with amino acid sequences as shown in SEQ ID NOs: 45, 141, and 29 respectively,
CDR1α, CDR2α, and CDR3α with amino acid sequences as shown in SEQ ID NOs: 170, 76, and 29 respectively,
CDR1α, CDR2α, and CDR3α with amino acid sequences as shown in SEQ ID NOs: 43, 118, and 29 respectively, or
CDR1α, CDR2α, and CDR3α with amino acid sequences as shown in SEQ ID NOs: 152, 149, and 29 respectively.

12. The modified TCR according to claim 1, wherein the modified TCR comprises:
(i) an α chain variable region, comprising:
CDR1α, CDR2α, and CDR3α amino acid sequences as shown in SEQ ID NOs:42, 28, and 29, respectively;
CDR1α, CDR2α, and CDR3α amino acid sequences as shown in SEQ ID NOs:43, 28, and 29, respectively;
CDR1α, CDR2α, and CDR3α amino acid sequences as shown in SEQ ID NOs:44, 28, and 29, respectively;
CDR1α, CDR2α, and CDR3α amino acid sequences as shown in SEQ ID NOs:45, 28, and 29, respectively;
CDR1α, CDR2α, and CDR3α amino acid sequences as shown in SEQ ID NOs:27, 76, and 29, respectively;
CDR1α, CDR2α, and CDR3α amino acid sequences as shown in SEQ ID NOs:97, 28, and 29, respectively;
CDR1α, CDR2α, and CDR3α amino acid sequences as shown in SEQ ID NOs:100, 28, and 29, respectively;
CDR1α, CDR2α, and CDR3α amino acid sequences as shown in SEQ ID NOs:103, 28, and 29, respectively;
CDR1α, CDR2α, and CDR3α amino acid sequences as shown in SEQ ID NOs:27, 106, and 29, respectively;
CDR1α, CDR2α, and CDR3α amino acid sequences as shown in SEQ ID NOs:27, 109, and 29, respectively;
CDR1α, CDR2α, and CDR3α amino acid sequences as shown in SEQ ID NOs:27, 112, and 29, respectively;
CDR1α, CDR2α, and CDR3α amino acid sequences as shown in SEQ ID NOs:27, 115, and 29, respectively;
CDR1α, CDR2α, and CDR3α amino acid sequences as shown in SEQ ID NOs:27, 118, and 29, respectively;
CDR1α, CDR2α, and CDR3α amino acid sequences as shown in SEQ ID NOs:27, 121, and 29, respectively;
CDR1α, CDR2α, and CDR3α amino acid sequences as shown in SEQ ID NOs:27, 124, and 29, respectively;
CDR1α, CDR2α, and CDR3α amino acid sequences as shown in SEQ ID NOs:27, 127, and 29, respectively;
CDR1α, CDR2α, and CDR3α amino acid sequences as shown in SEQ ID NOs:103, 76, and 29, respectively;
CDR1α, CDR2α, and CDR3α amino acid sequences as shown in SEQ ID NOs:44, 76, and 29, respectively;
CDR1α, CDR2α, and CDR3α amino acid sequences as shown in SEQ ID NOs:140, 141, and 29, respectively;
CDR1α, CDR2α, and CDR3α amino acid sequences as shown in SEQ ID NOs:146, 118, and 29, respectively;
CDR1α, CDR2α, and CDR3α amino acid sequences as shown in SEQ ID NOs:45, 149, and 29, respectively;
CDR1α, CDR2α, and CDR3α amino acid sequences as shown in SEQ ID NOs:152, 149, and 29, respectively;
CDR1α, CDR2α, and CDR3α amino acid sequences as shown in SEQ ID NOs:43, 76, and 29, respectively;
CDR1α, CDR2α, and CDR3α amino acid sequences as shown in SEQ ID NOs:45, 28, and 29, respectively;
CDR1α, CDR2α, and CDR3α amino acid sequences as shown in SEQ ID NOs:165, 118, and 29, respectively;
CDR1α, CDR2α, and CDR3α amino acid sequences as shown in SEQ ID NOs:27, 118, and 29, respectively; or
CDR1α, CDR2α, and CDR3α amino acid sequences as shown in SEQ ID NOs:45, 141, and 29, respectively;
CDR1α, CDR2α, and CDR3α amino acid sequences as shown in SEQ ID NOs:170, 76, and 29, respectively;
CDR1α, CDR2α, and CDR3α amino acid sequences as shown in SEQ ID NOs:43, 118, and 29, respectively; or
CDR1α, CDR2α, and CDR3α amino acid sequences as shown in SEQ ID NOs:152, 149, and 29, respectively.
(ii) a β-chain variable region, comprising:
CDR1β, CDR2β, and CDR3β amino acid sequences as shown in SEQ ID NOs:24, 30, and 26, respectively;
CDR1β, CDR2β, and CDR3β amino acid sequences as shown in SEQ ID NOs:24, 25, and 31, respectively;
CDR1β, CDR2β, and CDR3β amino acid sequences as shown in SEQ ID NOs:24, 25, and 32, respectively;
CDR1β, CDR2β, and CDR3β amino acid sequences as shown in SEQ ID NOs:24, 25, and 33, respectively;
CDR1β, CDR2β, and CDR3β amino acid sequences as shown in SEQ ID NOs:24, 25, and 34, respectively;
CDR1β, CDR2β, and CDR3β amino acid sequences as shown in SEQ ID NOs:24, 25, and 35, respectively;
CDR1β, CDR2β, and CDR3β amino acid sequences as shown in SEQ ID NOs:24, 30, and 33, respectively;
CDR1β, CDR2β, and CDR3β amino acid sequences as shown in SEQ ID NOs:24, 30, and 34, respectively;
CDR1β, CDR2β, and CDR3β amino acid sequences as shown in SEQ ID NOs:24, 30, and 35, respectively;
CDR1β, CDR2β, and CDR3β amino acid sequences as shown in SEQ ID NOs:24, 25, and 36, respectively;
CDR1β, CDR2β, and CDR3β amino acid sequences as shown in SEQ ID NOs:24, 25, and 37, respectively;
CDR1β, CDR2β, and CDR3β amino acid sequences as shown in SEQ ID NOs:24, 30, and 36, respectively;
CDR1β, CDR2β, and CDR3β amino acid sequences as shown in SEQ ID NOs:24, 30, and 37, respectively;
CDR1β, CDR2β, and CDR3β amino acid sequences as shown in SEQ ID NOs:24, 25, and 38, respectively;
CDR1β, CDR2β, and CDR3β amino acid sequences as shown in SEQ ID NOs:24, 25, and 39, respectively;
CDR1β, CDR2β, and CDR3β amino acid sequences as shown in SEQ ID NOs:24, 25, and 40, respectively;
CDR1β, CDR2β, and CDR3β amino acid sequences as shown in SEQ ID NOs:24, 25, and 41, respectively;
CDR1β, CDR2β, and CDR3β amino acid sequences as shown in SEQ ID NOs:24, 25, and 79, respectively;
CDR1β, CDR2β, and CDR3β amino acid sequences as shown in SEQ ID NOs:82, 25, and 26, respectively;
CDR1β, CDR2β, and CDR3β amino acid sequences as shown in SEQ ID NOs:24, 85, and 26, respectively;
CDR1β, CDR2β, and CDR3β amino acid sequences as shown in SEQ ID NOs:24, 88, and 26, respectively;
CDR1β, CDR2β, and CDR3β amino acid sequences as shown in SEQ ID NOs:24, 91, and 26, respectively;
CDR1β, CDR2β, and CDR3β amino acid sequences as shown in SEQ ID NOs:24, 25, and 94, respectively;
CDR1β, CDR2β, and CDR3β amino acid sequences as shown in SEQ ID NOs:130, 25, and 131, respectively;
CDR1β, CDR2β, and CDR3β amino acid sequences as shown in SEQ ID NOs:24, 91, and 35, respectively;
CDR1β, CDR2β, and CDR3β amino acid sequences as shown in SEQ ID NOs:130, 91, and 35, respectively;
CDR1β, CDR2β, and CDR3β amino acid sequences as shown in SEQ ID NOs:130, 153, and 35, respectively;
CDR1β, CDR2β, and CDR3β amino acid sequences as shown in SEQ ID NOs:130, 91, and 26, respectively;
CDR1β, CDR2β, and CDR3β amino acid sequences as shown in SEQ ID NOs:130, 91, and 162, respectively; or
CDR1β, CDR2β, and CDR3β amino acid sequences as shown in SEQ ID NOs:82, 91, and 33, respectively.

13. The modified TCR according to claim 1, wherein the modified TCR comprises an α chain variable region having an amino acid sequence as shown in SEQ ID NO: 46 or an amino acid sequence having at least 80%, at least 85%, at least 90%, at least 95%, or at least 99% sequence identity to SEQ ID NO: 46, and a β chain variable region having an amino acid sequence selected from the group consisting of any one of SEQ ID NOs: 48-64, 80, 83, 86, 89, 92, 95, 133, 137, 143, 155, 159, 163, 175, or an amino acid sequence having at least 80%, at least 85%, at least 90%, at least 95%, or at least 99% sequence identity to any one of SEQ ID NOs: 48-64, 80, 83, 86, 89, 92, 95, 133, 137, 143, 155, 159, 163, 175.

14. The modified TCR according to claim 1, wherein the modified TCR comprises an α chain variable region having an amino acid sequence selected from the group consisting of any one of SEQ ID NOs: 65-68, 77, 98, 101, 104, 107, 110, 113, 116, 119, 122, 125, 128, 132, 136, 142, 147, 150, 154, 158, 166, 168, 171, 173, or an amino acid sequence having at least 80%, at least 85%, at least 90%, at least 95%, or at least 99% sequence identity to any one of SEQ ID NOs: 65-68, 77, 98, 101, 104, 107, 110, 113, 116, 119, 122, 125, 128, 132, 136, 142, 147, 150, 154, 158, 166, 168, 171, 173, and a β chain variable region having an amino acid sequence as shown in SEQ ID NO: 47 or an amino acid sequence having at least 80%, at least 85%, at least 90%, at least 95%, or at least 99% sequence identity to SEQ ID NO: 47.

15. The modified TCR according to claim 1, wherein the modified TCR comprises an α chain variable region having an amino acid sequence selected from the group consisting of any one of SEQ ID NOs: 65-68, 77, 98, 101, 104, 107, 110, 113, 116, 119, 122, 125, 128, 132, 136, 142, 147, 150, 154, 158, 166, 168, 171, 173, or an amino acid sequence having at least 80%, at least 85%, at least 90%, at least 95%, or at least 99% sequence identity to any one of SEQ ID NOs: 65-68, 77, 98, 101, 104, 107, 110, 113, 116, 119, 122, 125, 128, 132, 136, 142, 147, 150, 154, 158, 166, 168, 171, 173, and a β chain variable region having an amino acid sequence selected from the group consisting of any one of SEQ ID NOs: 48-64, 80, 83, 86, 89, 92, 95, 133, 137, 143, 155, 159, 163, 175, or an amino acid sequence having at least 80%, at least 85%, at least 90%, at least 95%, or at least 99% sequence identity to any one of SEQ ID NOs: 48-64, 80, 83, 86, 89, 92, 95, 133, 137, 143, 155, 159, 163, 175.

16. The modified TCR according to any one of claims 1-15, wherein the modified TCR further comprises a TCR constant region.

17. The modified TCR according to claim 16, wherein the TCR constant region is a mouse constant region or a human constant region.

18. The modified TCR according to claim 16 or 17, wherein the TCR constant region comprises a TCR α chain constant region and/or a TCR β chain constant region;
preferably, the TCR α chain constant region and/or the TCR β chain constant region comprises at least one cysteine substitution relative to the wild-type sequence to form a disulfide bond between the TCR α chain and the TCR β chain;
preferably, the TCR α chain constant region comprises an LVL substitution or LIV substitution, such that the α chain constant region comprises an amino acid sequence LLVIVLRIL.

19. The modified TCR according to claim 16, wherein the modified TCR comprises a TCR α chain with an amino acid sequence as shown in SEQ ID NO: 69 or an amino acid sequence having at least 80%, at least 85%, at least 90%, at least 95%, or at least 99% sequence identity to SEQ ID NO: 69, and a TCR β chain having an amino acid sequence selected from the group consisting of any one of SEQ ID NOs: 3-19, 81, 84, 87, 90, 93, 96, 135, 139, 145, 157, 161, 164, 176, or an amino acid sequence having at least 80%, at least 85%, at least 90%, at least 95%, or at least 99% sequence identity to any one of SEQ ID NOs: 3-19, 81, 84, 87, 90, 93, 96, 135, 139, 145, 157, 161, 164, 176.

20. The modified TCR according to claim 16, wherein the modified TCR comprises a TCR α chain having an amino acid sequence selected from the group consisting of any one of SEQ ID NOs: 20-23, 78, 99, 102, 105, 108, 111, 114, 117, 120, 123, 126, 129, 134, 138, 144, 148, 151, 156, 160, 167, 169, 172, 174, or an amino acid sequence having at least 80%, at least 85%, at least 90%, at least 95%, or at least 99% sequence identity to any one of SEQ ID NOs: 20-23, 78, 99, 102, 105, 108, 111, 114, 117, 120, 123, 126, 129, 134, 138, 144, 148, 151, 156, 160, 167, 169, 172, 174, and a TCR β chain having an amino acid sequence as shown in SEQ ID NO: 70 or an amino acid sequence having at least 80%, at least 85%, at least 90%, at least 95%, or at least 99% sequence identity to SEQ ID NO: 70.

21. The modified TCR according to claim 16, wherein the modified TCR comprises a TCR α chain with an amino acid sequence selected from the group consisting of any one of SEQ ID NOs: 20-23, 78, 99, 102, 105, 108, 111, 114, 117, 120, 123, 126, 129, 134, 138, 144, 148, 151, 156, 160, 167, 169, 172, 174, or an amino acid sequence having at least 80%, 85%, 90%, 95%, or 99% sequence identity to any one of SEQ ID NOs: 20-23, 78, 99, 102, 105, 108, 111, 114, 117, 120, 123, 126, 129, 134, 138, 144, 148, 151, 156, 160, 167, 169, 172, 174, and a TCR β chain with an amino acid sequence selected from the group consisting of any one of SEQ ID NOs: 3-19, 81, 84, 87, 90, 93, 96, 135, 139, 145, 157, 161, 164, 176, or an amino acid sequence having at least 80%, 85%, 90%, 95%, or 99% sequence identity to any one of SEQ ID NOs: 3-19, 81, 84, 87, 90, 93, 96, 135, 139, 145, 157, 161, 164, 176.

22. The modified TCR according to any one of claims 1 to 21, wherein the modified TCR binds an epitope comprising an amino acid sequence of KVLEYVIKV (SEQ ID NO:2) or a complex of said epitope with an MHC molecule.

23. The modified TCR according to claim 22, wherein the MHC molecule is an HLA-A*02 type, such as HLA-A*02:01 or HLA-A*02:05.

24. The modified TCR according to any one of claims 1 to 23, wherein the modified TCR is soluble or membrane-bound.

25. The modified TCR according to any one of claims 1 to 24, wherein the modified TCR is isolated or purified.

26. A nucleic acid encoding the modified TCR according to any one of claims 1 to 25.

27. A vector comprising the nucleic acid according to claim 26.

28. The vector according to claim 27, wherein the vector is selected from the group consisting of lentiviral vectors, retroviral vectors, plasmids, DNA vectors, mRNA vectors, transposon-based vectors, and artificial chromosomes.

29. A cell comprising the modified TCR according to any one of claims 1 to 25, the nucleic acid according to claim 26, or the vector according to claim 27 or 28.

30. The cell according to claim 29, wherein the cell is selected from the group consisting of lymphocytes (such as T cells, NK cells), monocytes (such as PBMCs), and stem cells.

31. The cell according to claim 30, wherein the stem cell is a lymphoid progenitor cell or an induced pluripotent stem cell (iPSC).

32. The cell according to claim 30, wherein the cell is a T cell.

33. The cell according to claim 32, wherein the T cell does not express endogenous TCR.

34. A method for producing a cell according to any one of claims 29 to 33, comprising a step of transducing or transfecting the cell with the vector according to claim 27 or 28.

35. The method according to claim 34, further comprising a step of expanding and/or activating the cell before or after said transduction or transfection.

36. A conjugate comprising the modified TCR according to any one of claims 1 to 25 and an active agent conjugated or coupled to the TCR.

37. The conjugate according to claim 36, wherein the active agent is selected from the group consisting of a detectable label, an immunostimulatory molecule and a therapeutic agent;
preferably, the detectable marker is selected from the group consisting of biotins, streptavidin, enzymes or catalytically active fragments thereof, radionuclides, nanoparticles, paramagnetic metal ions, nucleic acid probes, contrast agents, and fluorescent, phosphorescent or chemiluminescent molecules;
preferably, the immunostimulatory molecule is selected from the group consisting of cytokines, chemokines, platelet factors and complement initiators;
preferably, the therapeutic agent is selected from the group consisting of immunomodulators, radioactive compounds, enzymes, chemotherapeutic agents and toxins.

38. A bispecific or multispecific antigen-binding molecule comprising the modified TCR according to any one of claims 1 to 25 or an antigen-binding fragment thereof, and one or more additional antigen-binding regions that bind one or more additional antigens;
optionally, wherein the one or more additional antigen-binding regions are each independently selected from the group consisting of antigen-binding regions derived from antibodies and antigen-binding regions derived from TCRs.

39. The bispecific or multispecific antigen-binding molecule according to claim 38, wherein the one or more additional antigens are each independently selected from the group consisting of tumor-associated antigens (TAAs), tumor-specific antigens (TSAs), viral antigens, autoantigens, and immune cell surface molecules.

40. The bispecific or multispecific antigen-binding molecule according to claim 39, wherein the one or more of the additional antigens are CD3,
preferably, wherein the one or more additional antigen-binding regions are derived from antigen-binding regions of an anti-CD3 antibody.

41. A composition comprising the modified TCR according to any one of claims 1 to 25, the nucleic acid according to claim 26, the vector according to claim 27 or 28, the cell according to any one of claims 29 to 33, the conjugate according to claim 36 or 37, or the bispecific or multispecific antigen-binding molecule according to any one of claims 38 to 40;
preferably, the composition further comprises a pharmaceutically acceptable carrier or excipient.

42. The composition according to claim 41, wherein the composition further comprises a second therapeutic agent;
preferably, wherein the second therapeutic agent is selected from the group consisting of antibodies, chemotherapeutic agents, and small molecule drugs.

43. A method for treating MAGE-A1 positive cancer in a subject, comprising administering to the subject an effective amount of the modified TCR according to any one of claims 1 to 25.

44. A method for treating MAGE-A1 positive cancer in a subject, comprising administering to the subject an effective amount of the cell according to any one of claims 29 to 33.

45. The method according to claim 44, wherein the cell is autologous or allogeneic to the subject.

46. The method according to claim 44, comprising the steps of:
(i) isolating a sample containing cells from the subject;
(ii) transducing or transfecting the cells with the vector according to claim 27 or 28; and
(iii) administering the cells obtained in step (ii) to the subject.

47. The method according to claim 46, further comprising a step of knocking out endogenous TCRs in the cells after step (i) and before step (ii).

48. The method according to any one of claims 43 to 47, wherein the MAGE-A1 positive cancer is selected from the group consisting of melanoma, lung cancer, colorectal cancer, cervical cancer, breast cancer, liver cancer (such as hepatocellular carcinoma), gastric cancer, esophageal cancer, bladder cancer (such as urothelial carcinoma of the bladder), ovarian cancer (such as serous cystadenocarcinoma of the ovary), head and neck cancer (such as squamous cell carcinoma of the head and neck), uterine cancer, endometrial cancer, cholangiocarcinoma, prostate cancer, adrenocortical carcinoma, mesothelioma, sarcoma, pheochromocytoma, paraganglioma, and thymic carcinoma.

49. The method according to any one of claims 43 to 48, wherein the method further comprises administering a second therapeutic agent, preferably, wherein the second therapeutic agent is selected from the group consisting antibodies, chemotherapeutic agents, and small molecule drugs.

50. The method according to any one of claims 43 to 49, wherein the subject has an HLA-A*02 allele, such as an HLA-A*02:01 or HLA-A*02:05 allele.
